# EUROPÄISCHE PATENTANMELDUNG

(11) **EP 0 264 020 A2**
(43) Veröffentlichungstag der Anmeldung: **20.04.1988**
(21) Anmeldenummer: 87114213.9
(22) Anmeldetag: 29.09.1987
(51) Int. Cl.: C07D 239/42, A01N 47/36, C07C 143/72

(54) **Verfahren zur Herstellung von 0-Aryl-N'-pyrimidin-2-yl-isoharnstoffen**

(30) Priorität: 14.10.1986 DE 3634926
(71) Anmelder: BAYER AG, 51368 Leverkusen (DE)
(72) Erfinder: Fest, Christa, Dr., D-5600 Wuppertal1 (DE)

(57) **Zusammenfassung**

Man erhält die herbizid wirksmen 0-Aryl-N'-pyrimidin-2-yl-harnstoffe der allgemeinen Formel (I) 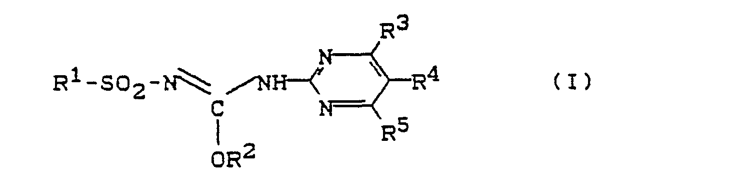 (worin die Reste R¹, R², R³, R⁴ und R⁵ die in der Beschreibung angegebenen Bedeutungen haben) in guten Ausbeuten, indem man Iminokohlensäureester der Formel (II) 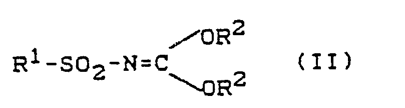 mit Aminen der Formel (III) 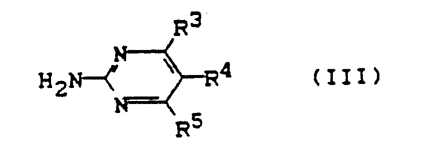 in Gegenwart von Säureakzeptoren und gegebenenfalls in Gegenwart von Verdünnungsmitteln bei Temperaturen zwischen 0 °C und 150 °C umsetzt.

## Beschreibung

Die vorliegende Erfindung betrifft ein neues Verfahren zur Herstellung von O-Aryl-N'-pyrimidin-2-yl- isoharnstoffen, sowie neue Zwischenprodukte hierfür. Die Verfahrensprodukte sind bekannt und können als Herbizide verwendet werden.

Es ist bekannt, daß man 0-Aryl-N'-pyrimidin-2-yl-isoharnstoffe herstellen kann, indem man Sulfonylguanidine mit Phenolen bzw. Phenolaten umsetzt. Nachteilig bei diesem Verfahren sind die teilweise unbefriedigenden Ausbeuten bei der Herstellung der einzusetzenden Sulfonylguanidine und/oder bei der Herstellung der 0-Aryl-N'-pyrimidin-2-yl-isoharnstoffe (vergl. EP-OS 173 957).

Es wurde nun gefunden, daß man 0-Aryl-N'-pyrimidin-2-yl-isoharnstoffe der allgemeinen Formel (I) 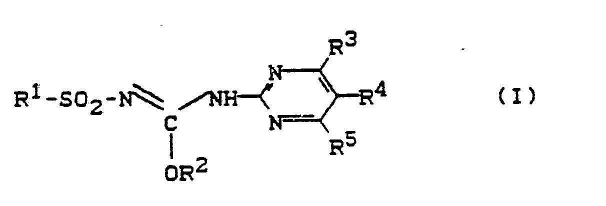 in welcher
R¹ für einen gegebenenfalls substituierten Rest aus der Reihe Aralkyl, Aryl und Heteroaryl steht,
R² für gegebenenfalls substituiertes Aryl steht,
R³ für Wasserstoff, Halogen, Hydroxy, C₁-C₆-Alkylamino, Di-(Ci-C6-alkyl)-amino oder für gegebenenfalls substituiertes C₁-C₆-Alkyl, C₁-C₆-Alkoxy und C₁-C₆-Alkylthio steht,
R⁴ für Wasserstoff, Halogen, gegebenenfalls substituiertes C₁-C₆-Alkyl, Cyano, Formyl, C₁-C₆-Alkylcarbonyl oder C₁-C₆-Alkoxycarbonyl steht und
R⁵ für Wasserstoff, Halogen, Hydroxy, für gegebenenfalls substituiertes C₁-C₆-Alkyl, C₁-C₆-Alkoxy und C₁-C₆--Alkylthio, für C₁-C₆-Alkylamino oder Di-(C₁-C₆-alkyl)-amino steht, oder
R⁴ und R⁵ gemeinsam für C₃-C₆-Alkandiyl stehen, auf einfache Weise in hohen Ausbeuten erhält, wenn man Iminokohlensäureester der Formel (II) 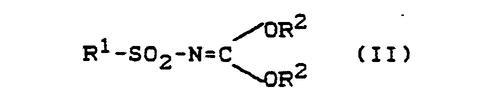 in welcher
R¹ und R² die oben angegebenen Bedeutungen haben, mit Aminen der Formel (III) 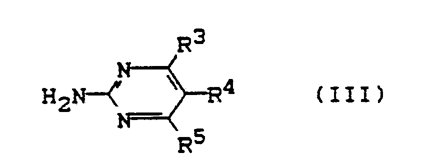 in welcher
R³, R⁴ und R⁵ die oben angegebenen Bedeutungen haben,
in Gegenwart von Säureakzeptoren und gegebenenfalls in Gegenwart von Verdünnungsmitteln bei Temperaturen zwischen 0 °C und 150 °C umsetzt.

Überraschenderweise gelingt es mit Hilfe des erfindungsgemäßen Verfahrens die Verbindungen der Formel (I) auf einfache Weise in guter Ausbeute herzustellen.

Bevorzugt werden mit Hilfe des erfindungsgemäßen Verfahrens diejenigen Verbindungen der Formel (I) hergestellt, in welcher R' für den Rest 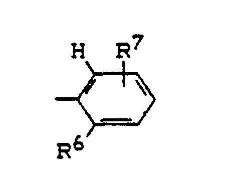 steht, worin
R⁶ und R⁷ gleich oder verschieden sind und für Wasserstoff, Halogen [wie insbesondere Fluor, Chlor, Brom und/oder lod], Cyano, C₁-C₆-Alkyl [welches gegebenenfalls durch Fluor, Chlor, Brom, Cyano, C₁-C₄-Alkoxy, C₃-C₆-Cycloalkyl oder Phenyl substituiert ist], für C₁-C₄-Alkoxy [welches gegebenenfalls durch Fluor, Chlor, Brom, Cyano oder C₁-C₄-Alkoxy substituiert ist], für Di-(C₁-C₄-alkyl)-aminosulfonyl, für Phenyl oder Phenoxy, für C₁-C₄-Alkylcarbonylamino, C₁-C₄-Alkylamino-carbonyl-amino, Di-(C₁-C₄-alkyl)-amino-carbonylamino, Di-(C₁-C₄-alkyl)-aminocarbonyl oder für Di-(C₁-C₄-alkyl)-aminosulfonylamino stehen, worin weiter
R' für den Rest 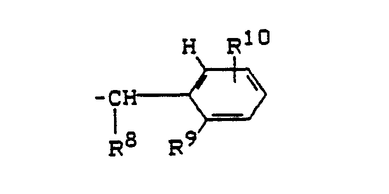 steht, worin
R⁸ für Wasserstoff oder C₁-C₄-Alkyl steht,
R⁹ und R¹⁰ gleich oder verschieden sind und für Wasserstoff, Fluor, Chlor, Brom, Nitro, Cyano, Ci-C_{d}-Alkyl [welches gegebenenfalls durch Fluor und/oder Chlor substituiert ist], C₁-C₄-Alkoxy [welches gegebenenfalls durch Fluor undioder Chlor substituiert ist] oder Di-(C₁-C₄-alkyl)-aminosulfonyl stehen; worin weiter
R¹ für den Rest 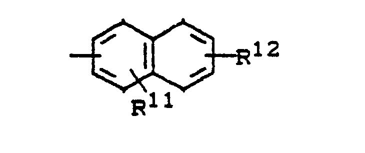 steht, wonn
R¹¹ und R¹² gleich oder verschieden sind und für Wasserstoff. Fluor, Chlor, Brom, Nitro, Cyano, C₁-C₄-Alkyl [welches gegebenenfalls durch Fluor und/oder Chlor substituiert ist] oder C₁-C₄-Alkoxy [welches gegebenenfalls durch Fluor und/oder Chlor substituiert ist], stehen; worin weiter
R² für einen Phenylrest steht, welcher gegebenenfalls substituiert ist durch einen oder mehrere Reste aus der Reihe
   Halogen [wie insbesondere Fluor, Chlor, Brom und lod], Cyano, Nitro, Hydroxy, Carboxy, C₁-C₆-Alkyl [welches gegebenenfalls durch Fluor, Chlor, Brom, Nitro, Cyano, Hydroxy, Carboxy, C₁-C₄-Alkoxy-carbonyl, C₁-C₄-Alkoxy, C₁-C₄-Alkylthio oder Phenyl substituiert ist], C₂-C₆-Cycloalkyl, Ci-C_{d}-Alkoxy [welches gegebenenfalls durch Fluor, Chlor, Brom, Cyano, Carboxy, C₁-C₄-Alkoxy, C₁-C₄-Alkylthio oder C₁-C₄-Alkoxy-carbonyl substituiert ist], C₁-C₄-Alkylthio [welches gegebenenfalls durch Fluor, Chlor, Brom, Cyano, Carboxy, C₁-C₄-Alkoxy-carbonyl substituiert ist], Amino, C₁-C₄-Alkyl-amino bzw. Di-(C₁-C₄-alkyl)-amino [welche gegebenenfalls durch Fluor, Chlor, Brom, Cyano, Carboxy, C₁-C₄-Alkoxy oder C₁-C₄-Alkoxy-carbonyl substituiert sind], Ci-C₄-Alkyl-carbonyl-amino, C₁-C₄-Alkoxy-carbonyl-amino, (Di)-Ci-C₄-Alkylamino-carbonyl-amino, Formyl, C₁-C₄-Alkyl-carbonyl, Benzoyl, C₁-C₄-Alkoxy-carbonyl, Phenoxy-carbonyl, Benzyloxycarbonyl, Phenyl [welches gegebenenfalls durch Fluor, Chlor, Brom, Cyano, Nitro, Hydroxy oder Methyl substituiert ist], Phenoxy, Phenylthio, Phenylsulfonyl, Phenylamino oder Phenylazo [welche gegebenenfalls durch Fluor, Chlor, Brom, Cyano, Nitro, Methyl und/oder Trifluormethyl substituiert sind], Pyridoxy oder Pyrimidoxy [welche gegebenenfalls durch Fluor, Chlor, Brom, Cyano, Nitro, Methyl und/oder Trifluormethyl substituiert sind], C₁-C₄-Alkyl-carbonyloxy, C₁-C₄-Alkoxy-carbonyloxy, C₁-C₄-Alkyl-amino-carbonyloxy und Di-(C₁-C₄-alkyl)-amino-car- bonyloxy, oder welcher gegebenenfalls durch eine Alkylenkette [welche gegebenenfalls verzweigt und/oder durch ein oder mehrere Sauerstoffatome unterbrochen ist] oder einen Benzorest [welcher gegebenenfalls durch Fluor, Chlor, Brom, Cyano, Nitro, Methyl und/oder Trifluormethyl substituiert ist] anelliert ist; worin weiter
R³ für Wasserstoff, Fluor, Chlor, Brom, Hydroxy, C₁-C₄-Alkylamino, Di-(C₁-C₄-alkyl)-amino, C₁-C₄-Alkyl [weiches gegebenenfalls durch Fluor und/oder Chlor substituiert ist], C₁-C₄-Alkoxy [welches gegebenenfalls durch Fluor und/oder Chlor substituiert ist], C₁-C₄-Alkylthio [welches gegebenenfalls durch Fluor und/oder Chlor substituiert ist] steht,
R⁴ für Wasserstoff, Fluor, Chlor, Brom, C₁-C₄-Alkyl [welches gegebenenfalls durch Fluor und/oder Chlor substituiert ist], Cyano, Formyl, C₁-C₄-Alkyl-carbonyl oder C₁-C₄-Alkoxy-carbonyl steht und
R⁵ für Wasserstoff, Fluor, Chlor, Brom, Hydroxy, C₁-C₄-Alkyl [welches gegebenenfalls durch Fluor und/oder Chlor substituiert ist], C₁-C₄-Alkoxy [welches gegebenenfalls durch Fluor und/oder Chlor substituiert ist], Cₗ-C₄-Alkylthio [welches gegebenenfalls durch Fluor und/oder Chlor substituiert ist], C₁-C₄-Alkyl-amino oder Di-(C₁-C₄-alkyl)-amino steht, oder
R⁴ und R⁵ gemeinsam für C₃-C₄-Alkandiyl stehen.

Verwendet man beispielsweise N-(2-Chlor-benzolsulfonyl)-iminokohlensäurediphenylester und 2-Amino-4-methoxy-6-methyl-pyrimidin als Ausgangsstoffe, so kann der Reak tionsablauf nach dem erfindungsgemäßen Verfahren durch folgendes Formelschema wiedergegeben werden:

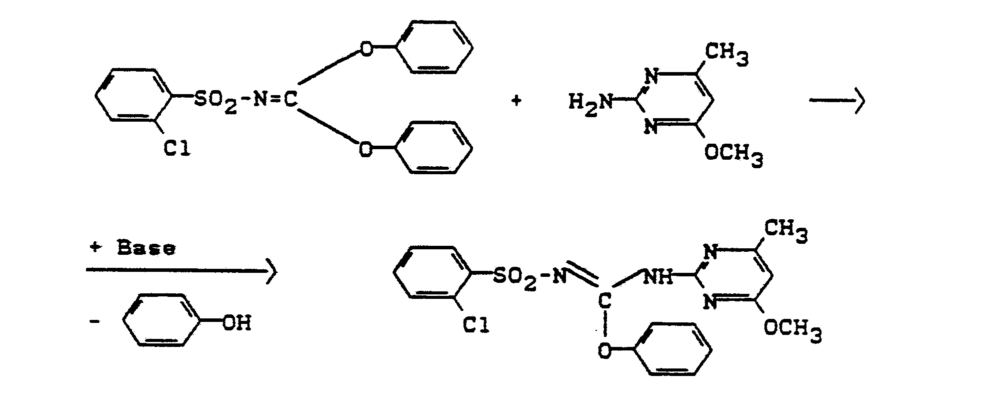

Die beim erfindungsgemäßen Verfahren als Ausgangsstoffe zu verwendenden Iminokohlensäureester sind neu und durch die Formel (II) allgemein definiert. Bevorzugt sind die neuen Iminokohlensäureester der Formel (II) in welcher
R¹ für den Rest 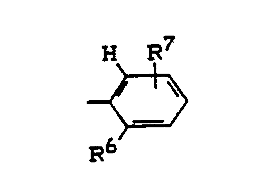 steht, worin
R⁶ und R⁷ gleich oder verschieden sind und für Wasserstoff, Halogen [wie insbesondere Fluor, Chlor, Brom und/oder lod], Cyano, C₁-C₆-Alkyl [welches gegebenenfalls durch Fluor, Chlor, Brom, Cyano, C₁-C₄-Alkoxy, C₃-C₆-Cycloalkyl oder Phenyl substituiert ist], für C₁-C₄-Alkoxy [welches gegebenenfalls durch Fluor, Chlor, Brom, Cyano oder Cₗ-C₄-Alkoxy substituiert ist], für Di-(C₁-C₄-alkyl)-aminosulfonyl, für Phenyl oder Phenoxy, für C₁-C₄-Alkylcarbonylamino, C₁-C₄-Alkylamino-carbonyl-amino, Di-(C₁-C₄-alkyl)-amino-carbonylamino, Di-(C₁-C₄-alkyl)-aminocarbonyl oder für Di-(C₁-C₄-alkyl)-aminosulfonylamino stehen, worin weiter
R¹ für den Rest 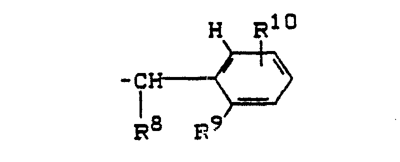 steht, worin
R^{e} für Wasserstoff oder C₁-C₄-Alkyl steht,
R⁹ und R¹⁰ gleich oder verschieden sind und für Wasserstoff, Fluor, Chlor, Brom, Nitro, Cyano, C₁-C₄-Alkyl [welches gegebenenfalls durch Fluor und/oder Chlor substituiert ist], C₁-C₄-Alkoxy [welches gegebenenfalls durch Fluor und/oder Chlor substituiert ist] oder Di-(C₁-C₄-alkyl)-aminosulfonyl stehen; worin weiter
R' für den Rest 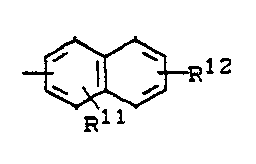 steht, worin
R¹¹ und R¹² gleich oder verschieden sind und für Wasserstoff, Fluor, Chlor, Brom, Nitro, Cyano, C₁-C₄-Alkyl [welches gegebenenfalls durch Fluor und/oder Chlor substituiert ist] oder C₁-C₄-Alkoxy [welches gegebenenfalls durch Fluor und/oder Chlor substituiert ist], stehen; und
R² für einen Phenylrest steht, welcher gegebenenfalls substituiert ist durch einen oder mehrere Reste aus der Reihe
   Halogen [wie insbesondere Fluor, Chlor, Brom und lod], Cyano, Nitro, Hydroxy, Carboxy, C₁-C₆-Alkyl [welches gegebenenfalls durch Fluor, Chlor, Brom, Nitro, Cyano, Hydroxy, Carboxy, C₁-C₄-Alkoxy-carbonyl, C₁-C₄-Alkoxy, C₁-C₄-Alkylthio oder Phenyl substituiert ist], C₃-C₆-Cycloalkyl, C₁-C₄-Alkoxy [welches gegebenenfalls durch Fluor, Chlor, Brom, Cyano, Carboxy, C₁-C₄-Alkoxy, C₁-C₄-Alkylthio oder C₁-C₄-Alkoxy-carbonyl substituiert ist], C₁-C₄-Alkylthio [welches gegebenenfalls durch Fluor, Chlor, Brom, Cyano, Carboxy, C₁-C₄-Alkoxy-carbonyl substituiert ist], Amino, C₁-C₄-Alkyl-amino bzw. Di-(C₁-C₄-alkyl)-amino [welche gegebenenfalls durch Fluor, Chlor, Brom, Cyano, Carboxy, C₁-C₄-Alkoxy oder C₁-C₄-Alkoxy-carbonyl substituiert sind], C₁-C₄-Alkyl-carbonyl-amino, Ci-C_{d}-Alkoxy-carbonyl-amino, (Di)-C₁-C₄-Alkylamino-carbonyl-amino, Formyl, Ci-Ce-Alkyl-carbonyl, Benzoyl, C₁-C₄-Alkoxy-carbonyl, Phenoxy-carbonyl, Benzyloxycarbonyl, Phenyl [welches gegebenenfalls durch Fluor, Chlor, Brom, Cyano, Nitro, Hydroxy oder Methyl substituiert ist], Phenoxy, Phenylthio, Phenylsulfonyl, Phenylamino oder Phenylazo [welche gegebenenfalls durch Fluor, Chlor, Brom, Cyano, Nitro, Methyl und/oder Trifluormethyl substituiert sind], Pyridoxy oder Pyrimidoxy [welche gegebenenfalls durch Fluor, Chlor, Brom, Cyano, Nitro, Methyl und/oder Trifluormethyl substituiert sind], C₁-C₄-Alkyl-carbonyloxy, C₁-C₄-Alkoxy-carbonyloxy, C₁-C₄-Alkyl-amino-carbonyloxy und Di-(C₁-C₄-alkyl)-amino-car- bonyloxy, oder welcher gegebenenfalls durch eine Alkylenkette [welche gegebenenfalls verzweigt und/oder durch ein oder mehrere Sauerstoffatome unterbrochen ist] oder einen Benzorest [welcher gegebenenfalls durch Fluor, Chlor, Brom, Cyano, Nitro, Methyl und/oder Trifluormethyl substituiert ist] anelliert ist.

Als Beispiele für die Iminokohlensäureester der Formel (II) seien genannt:

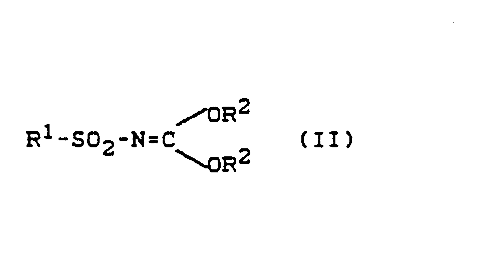

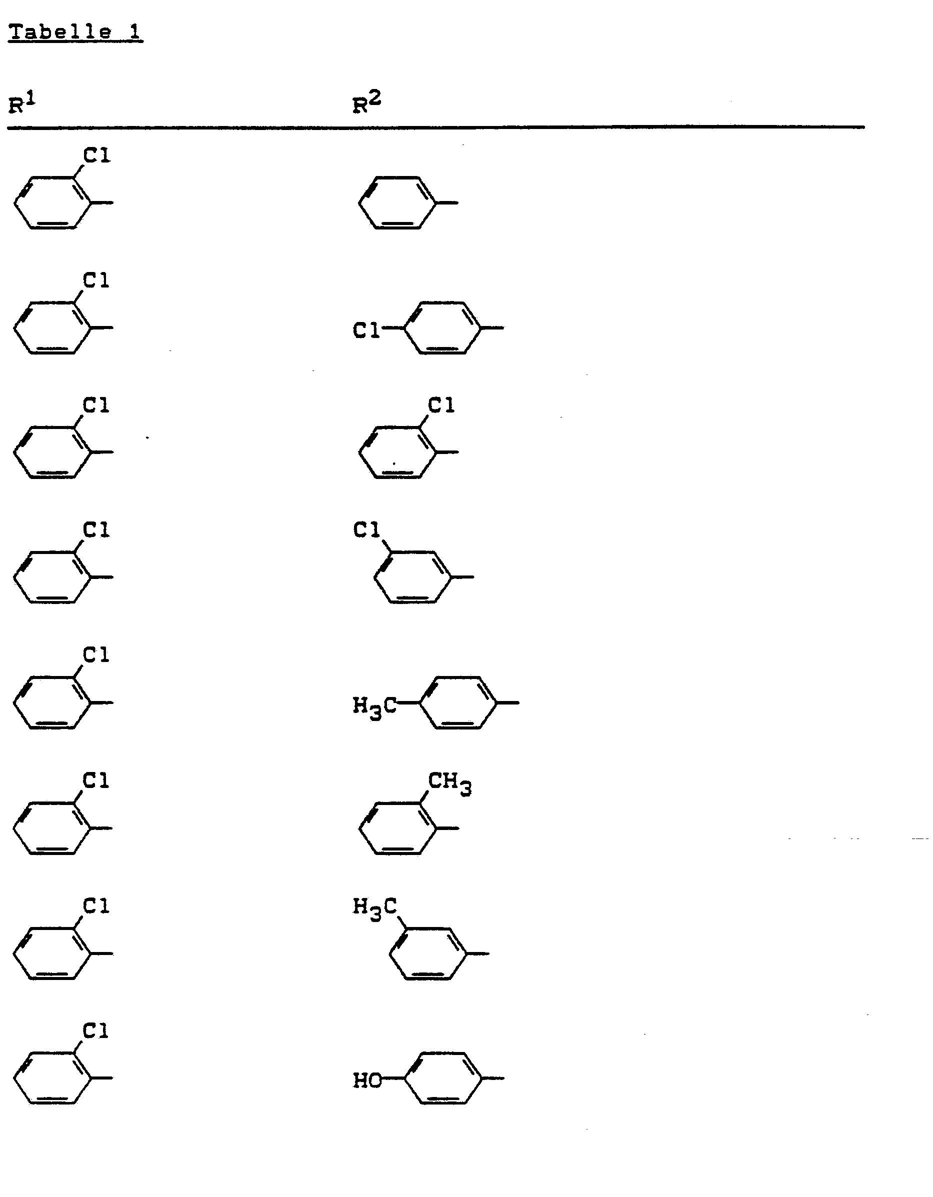

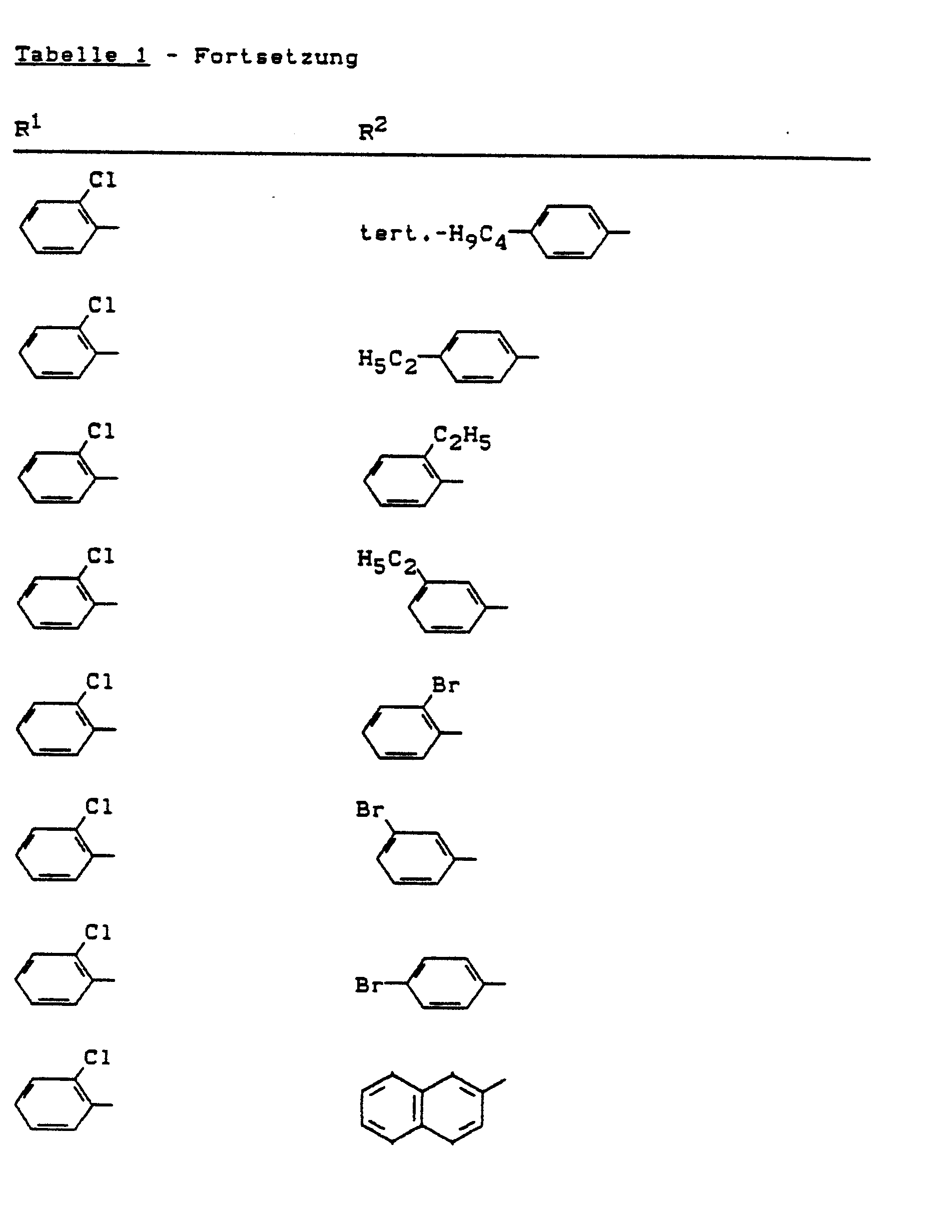

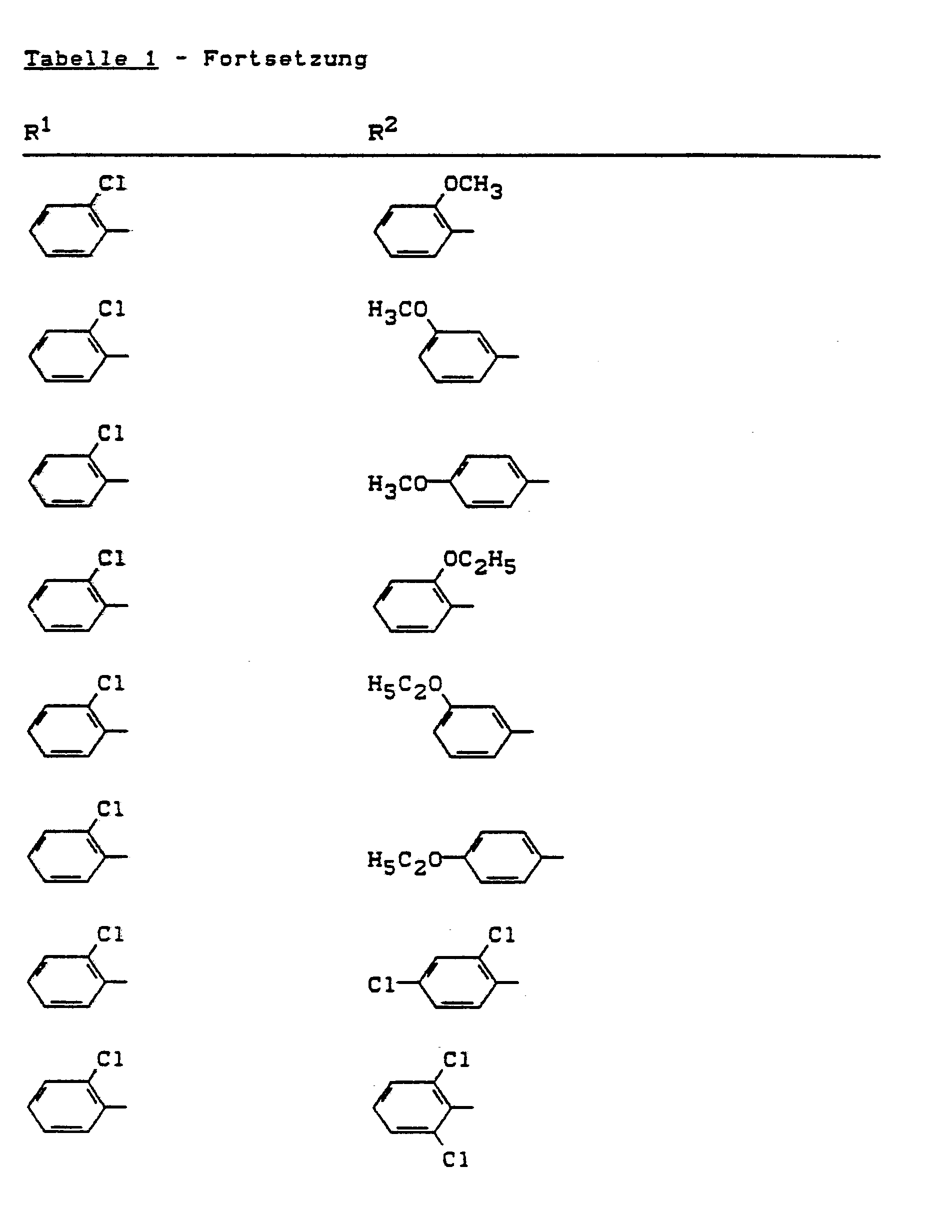

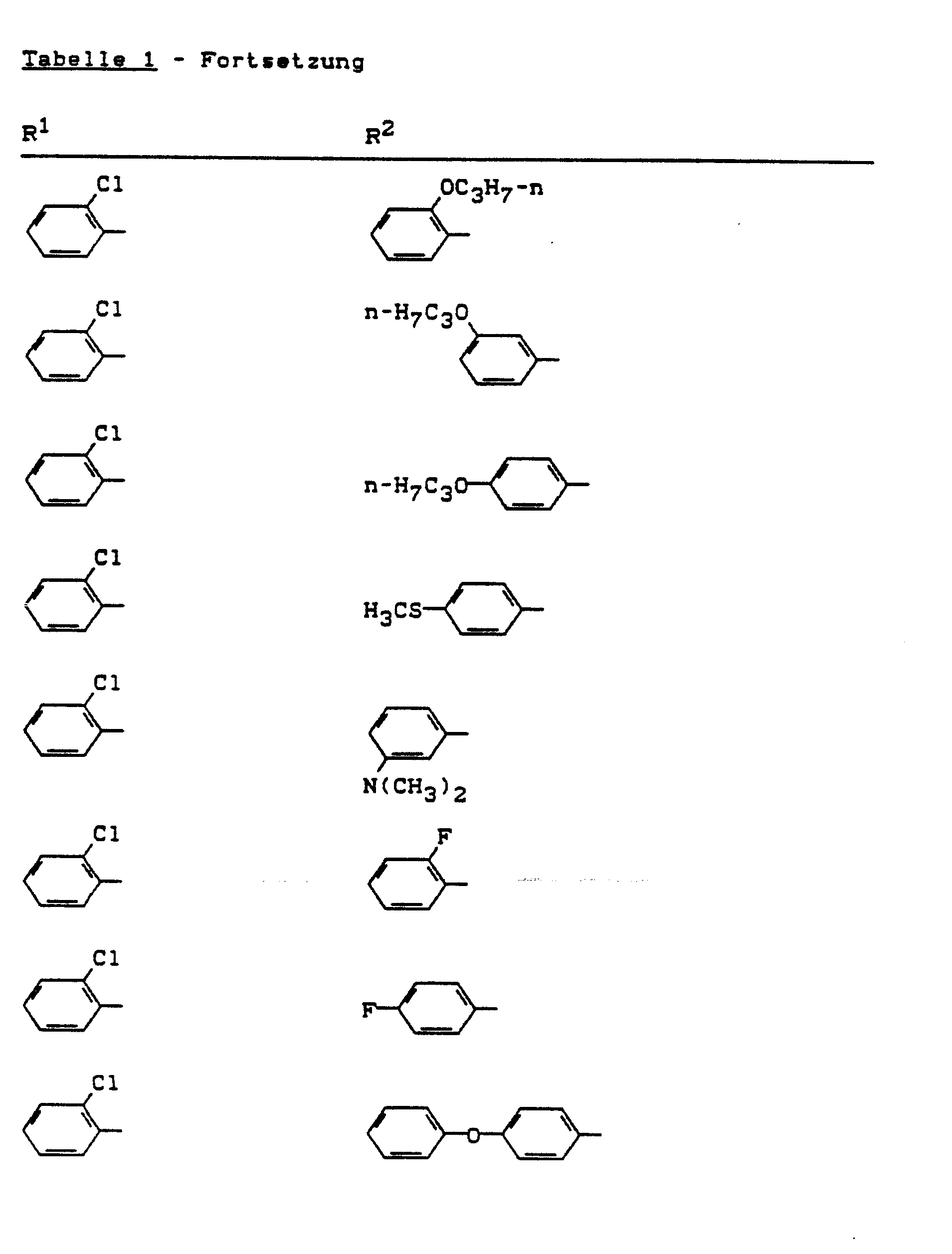

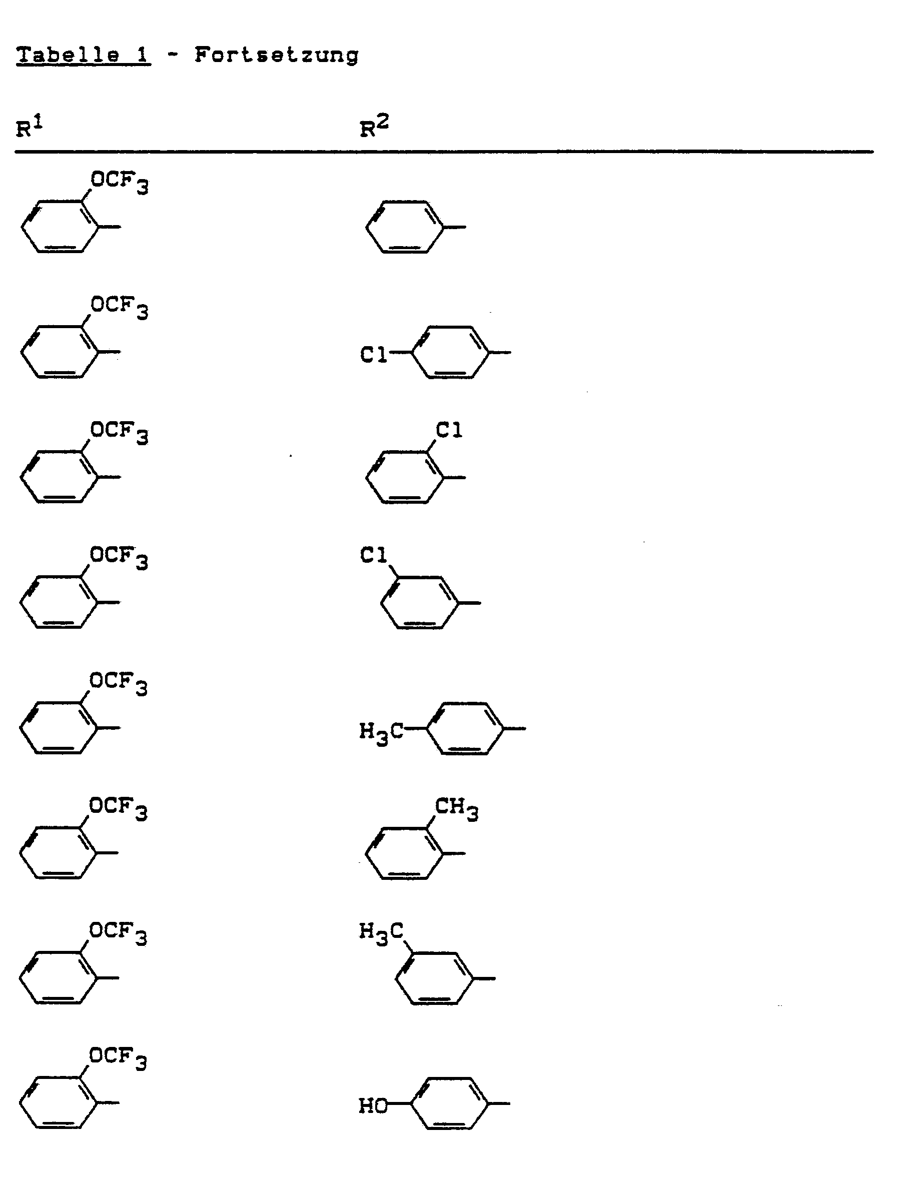

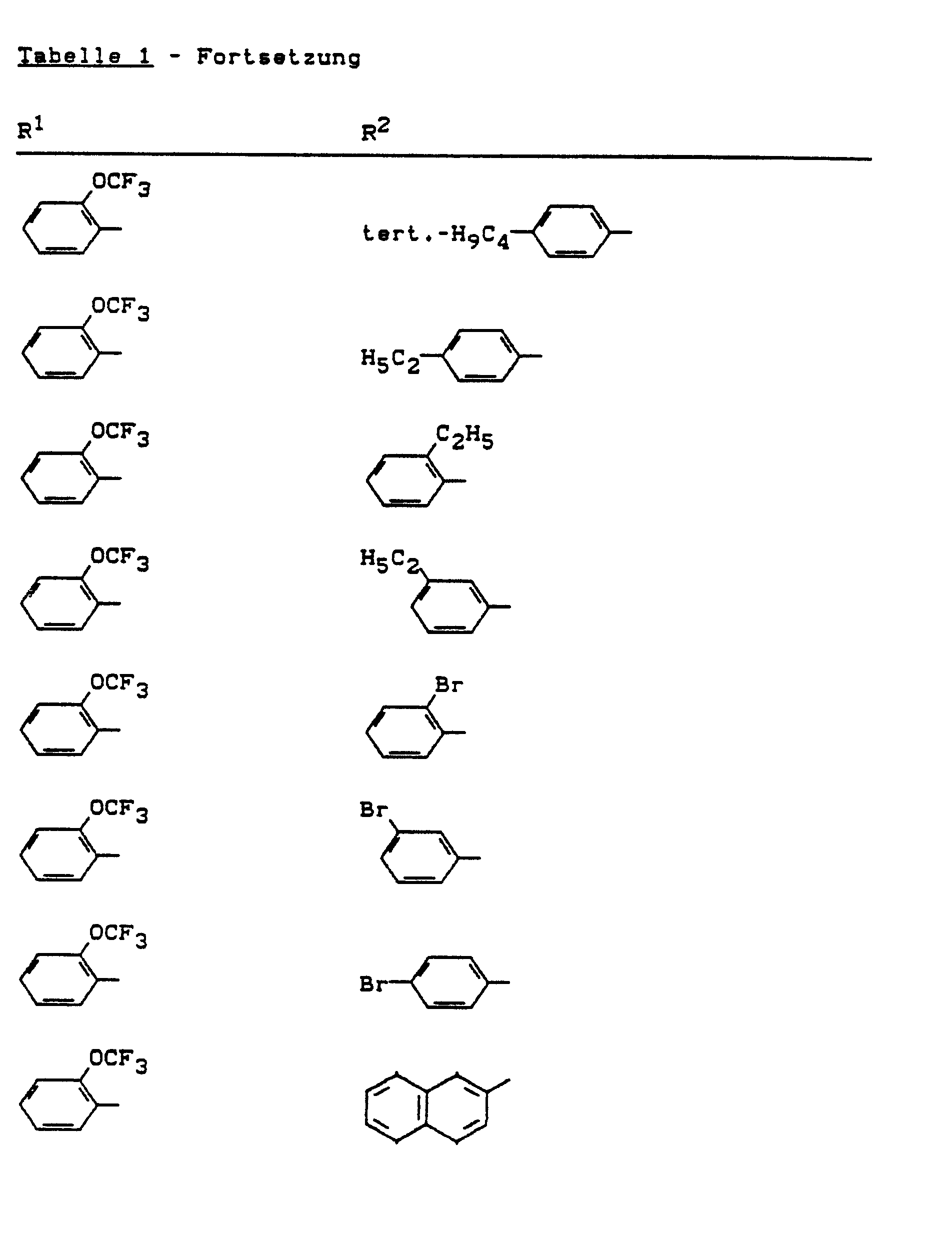

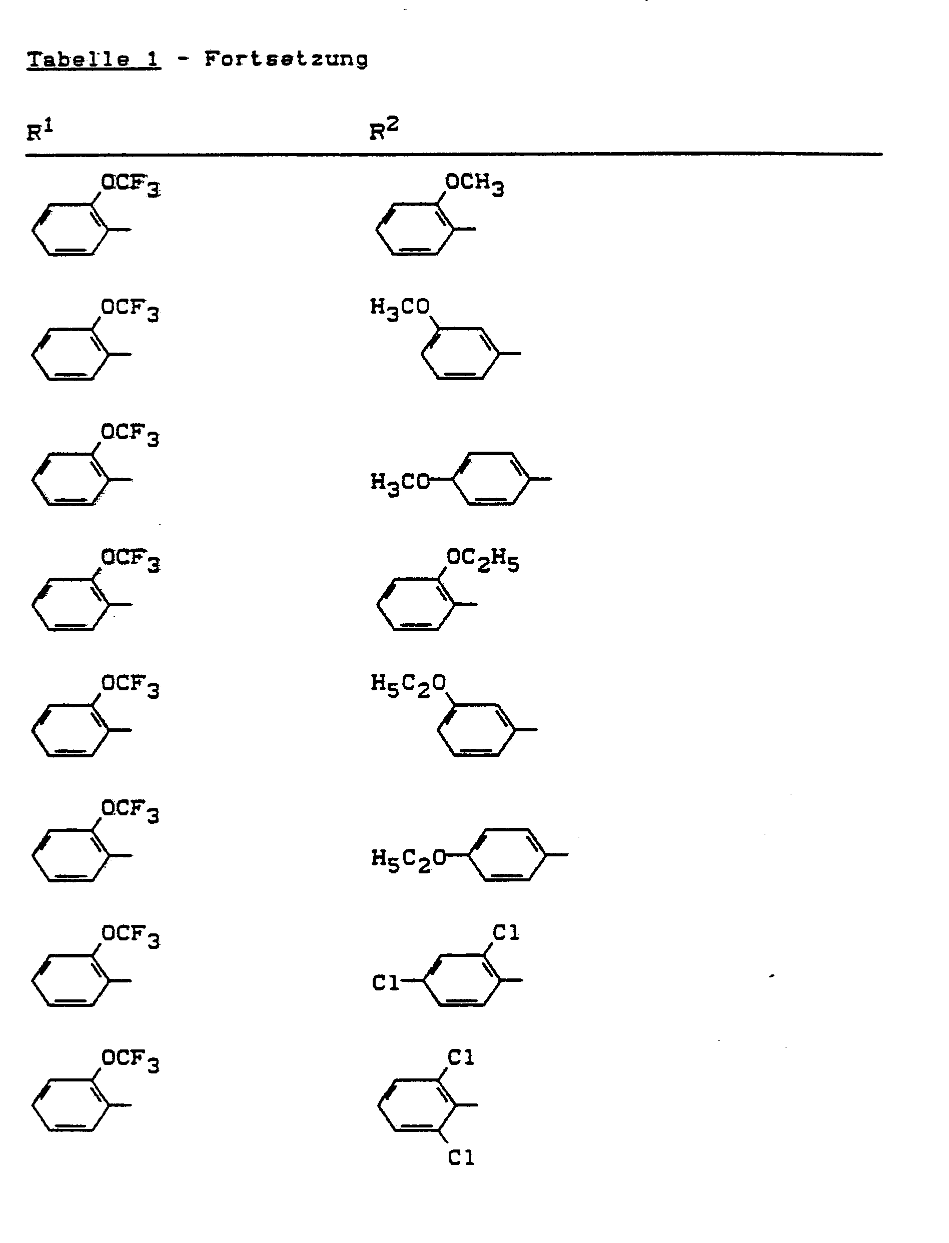

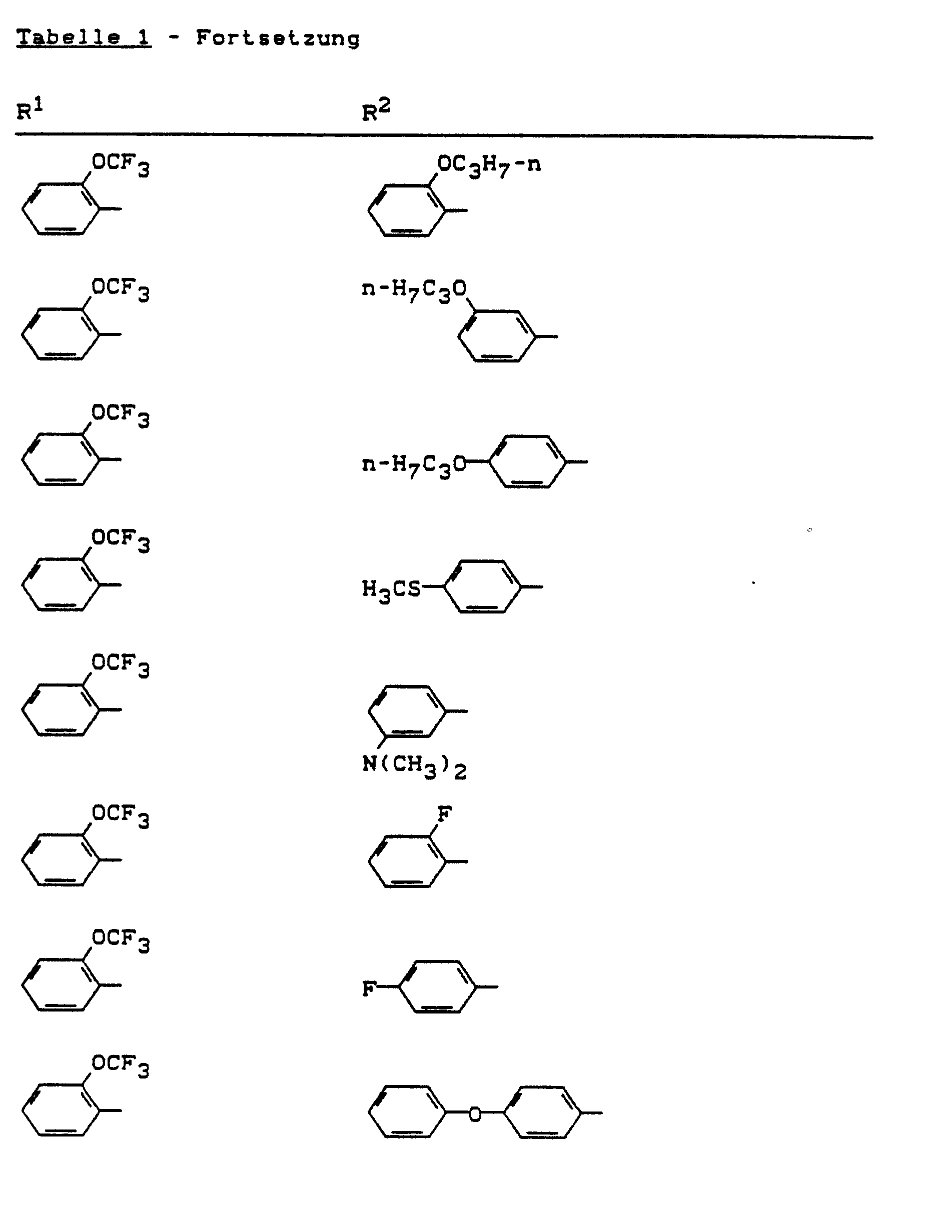

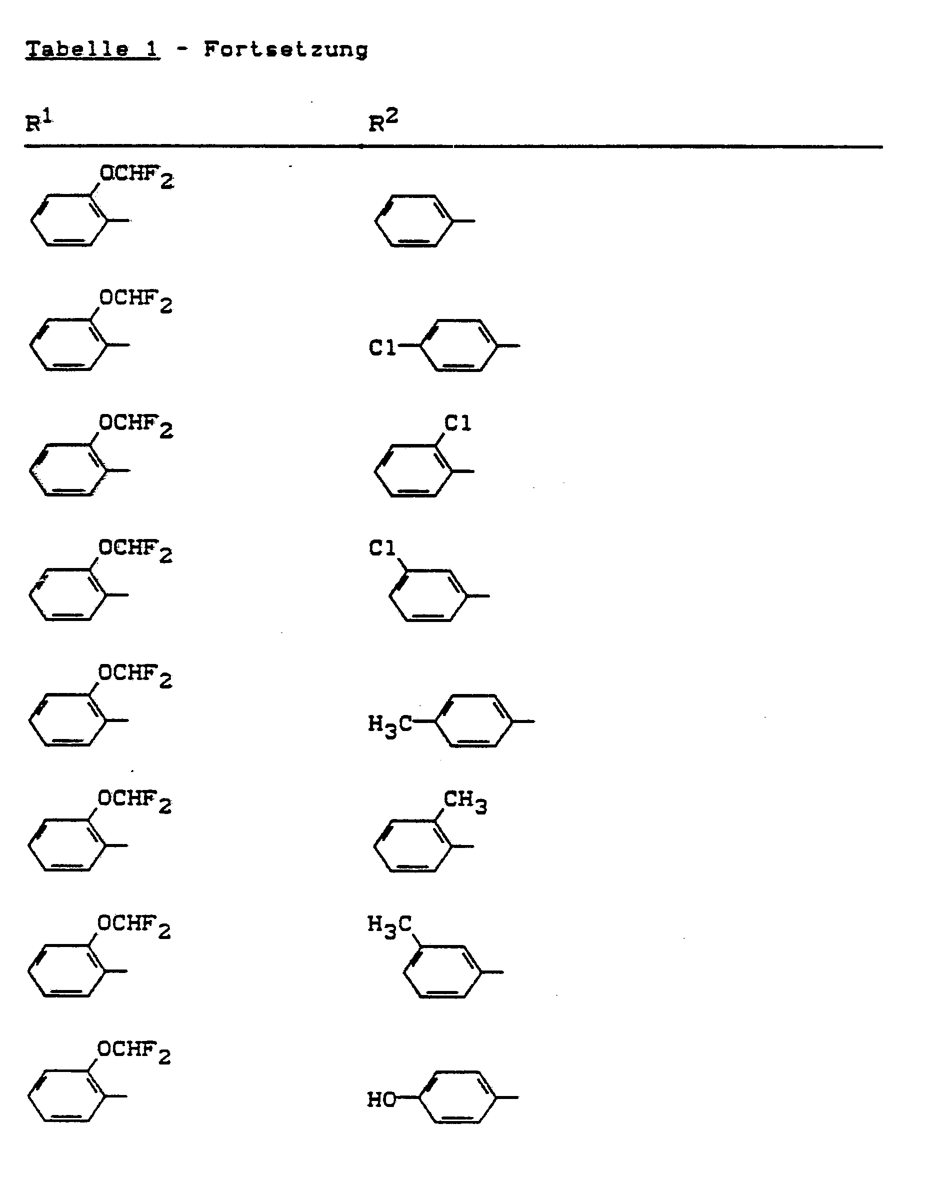

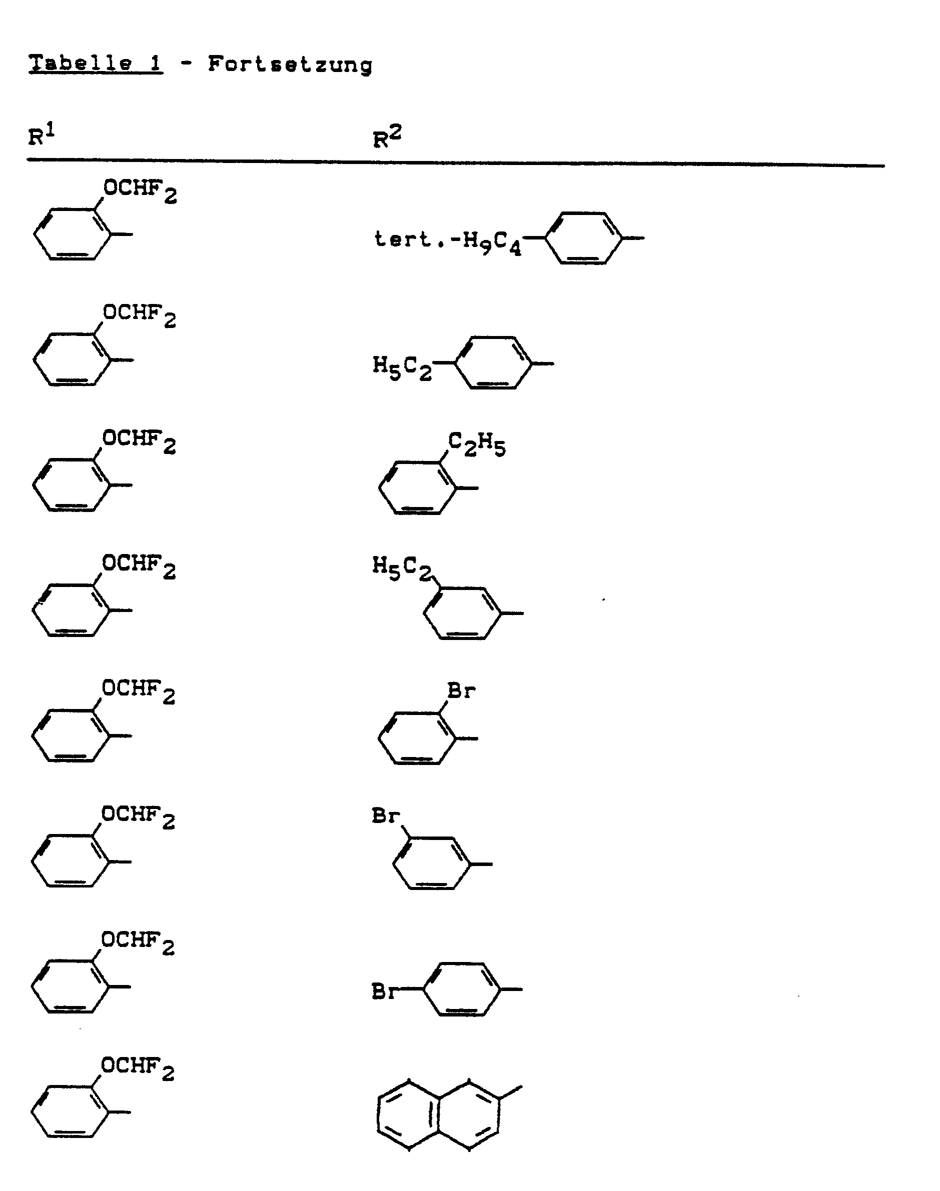

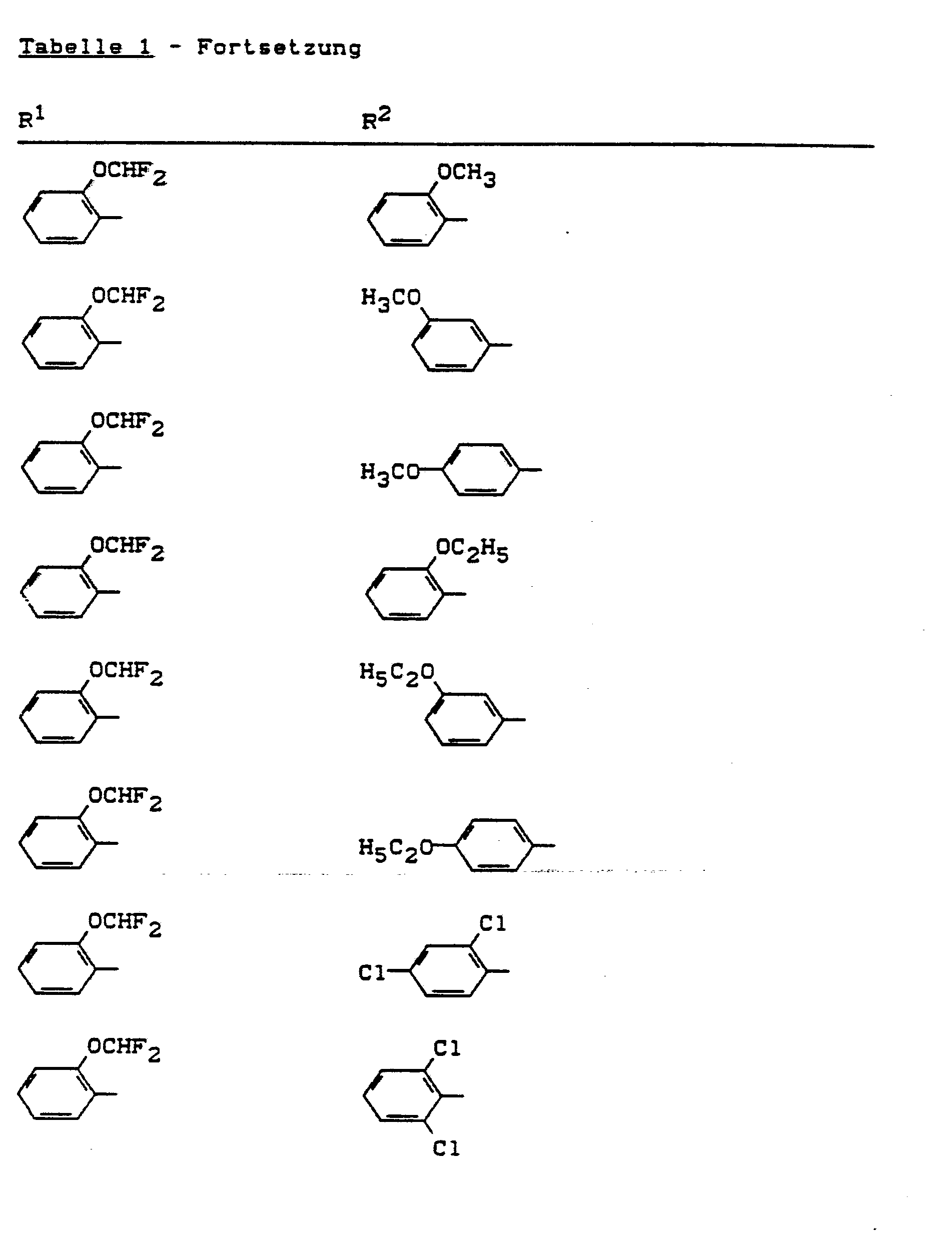

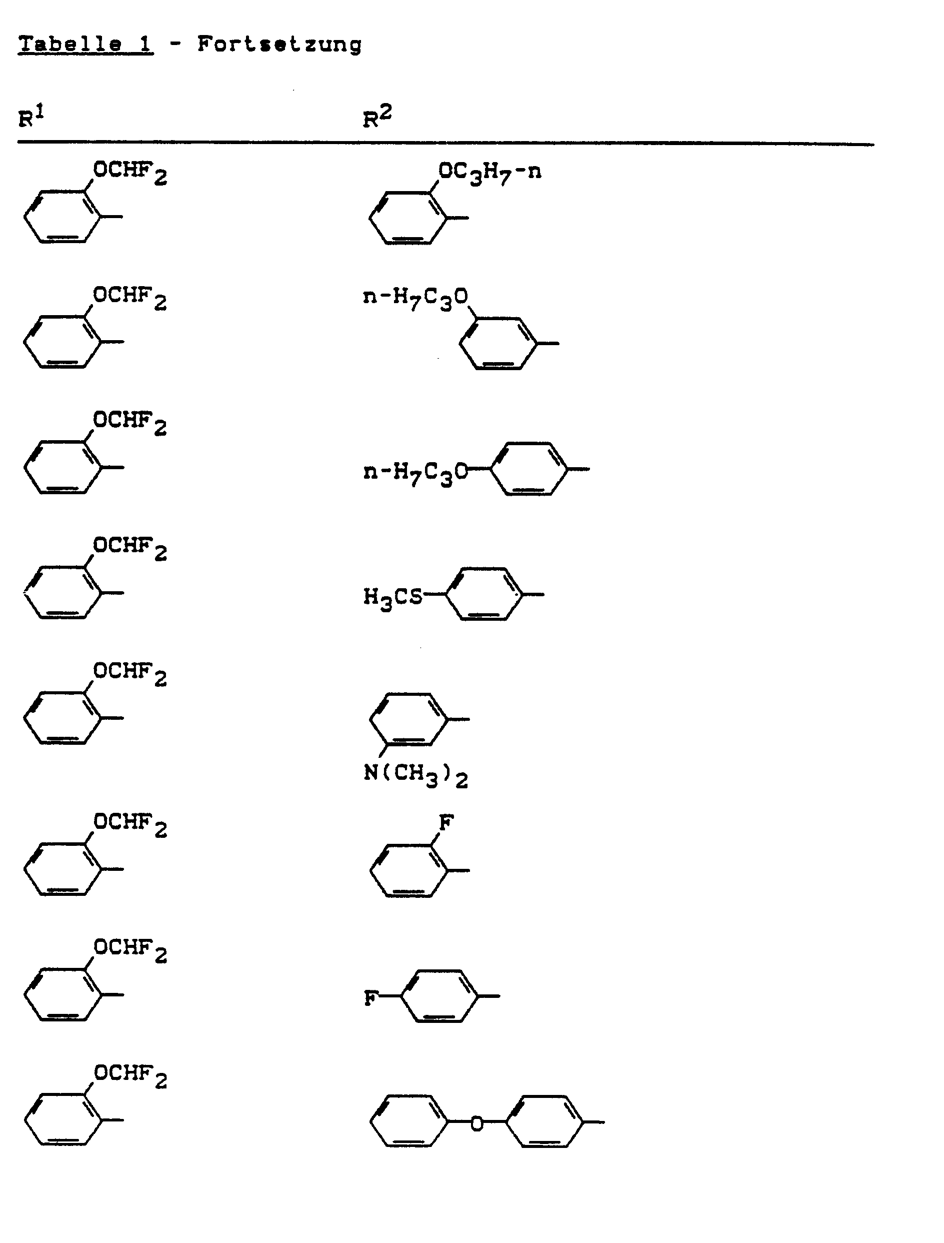

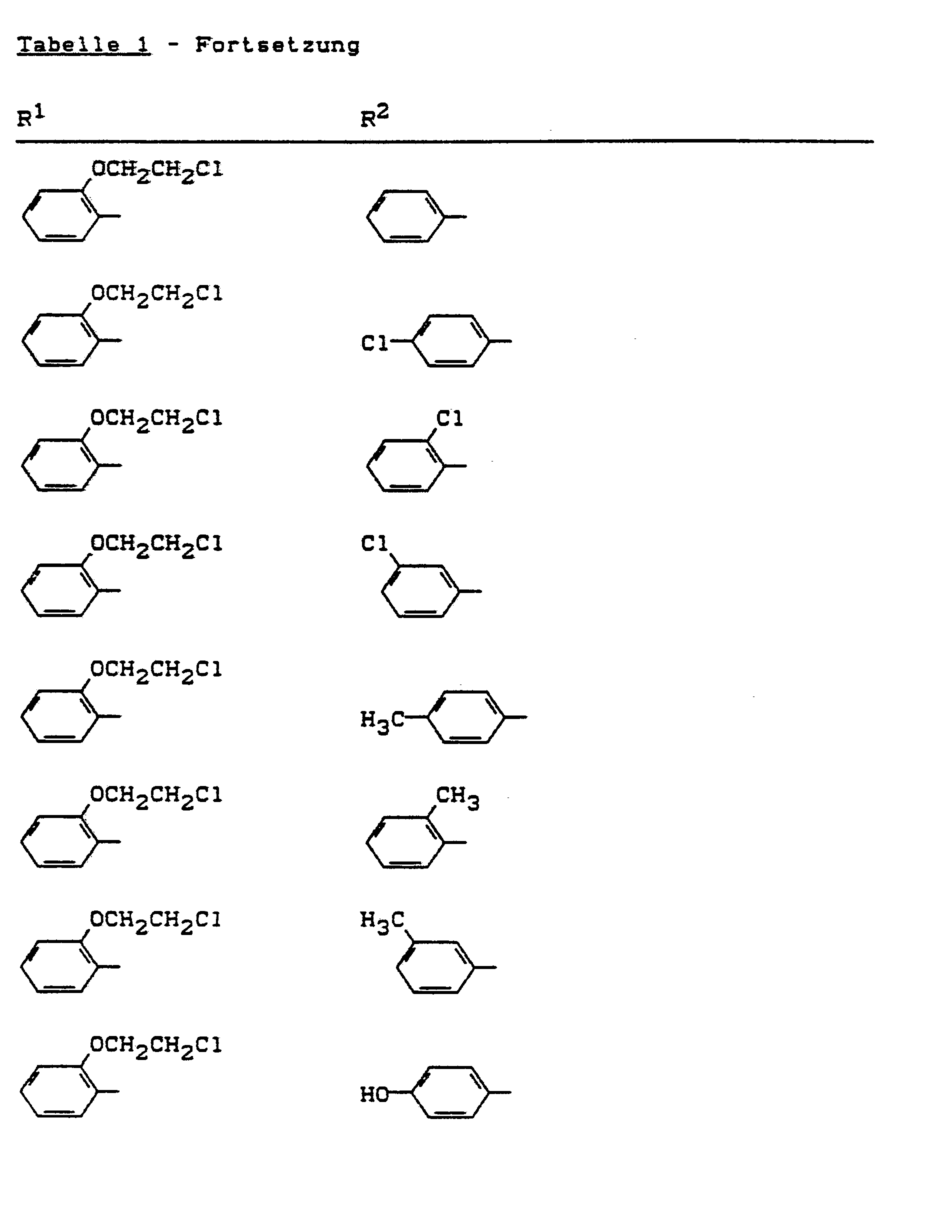

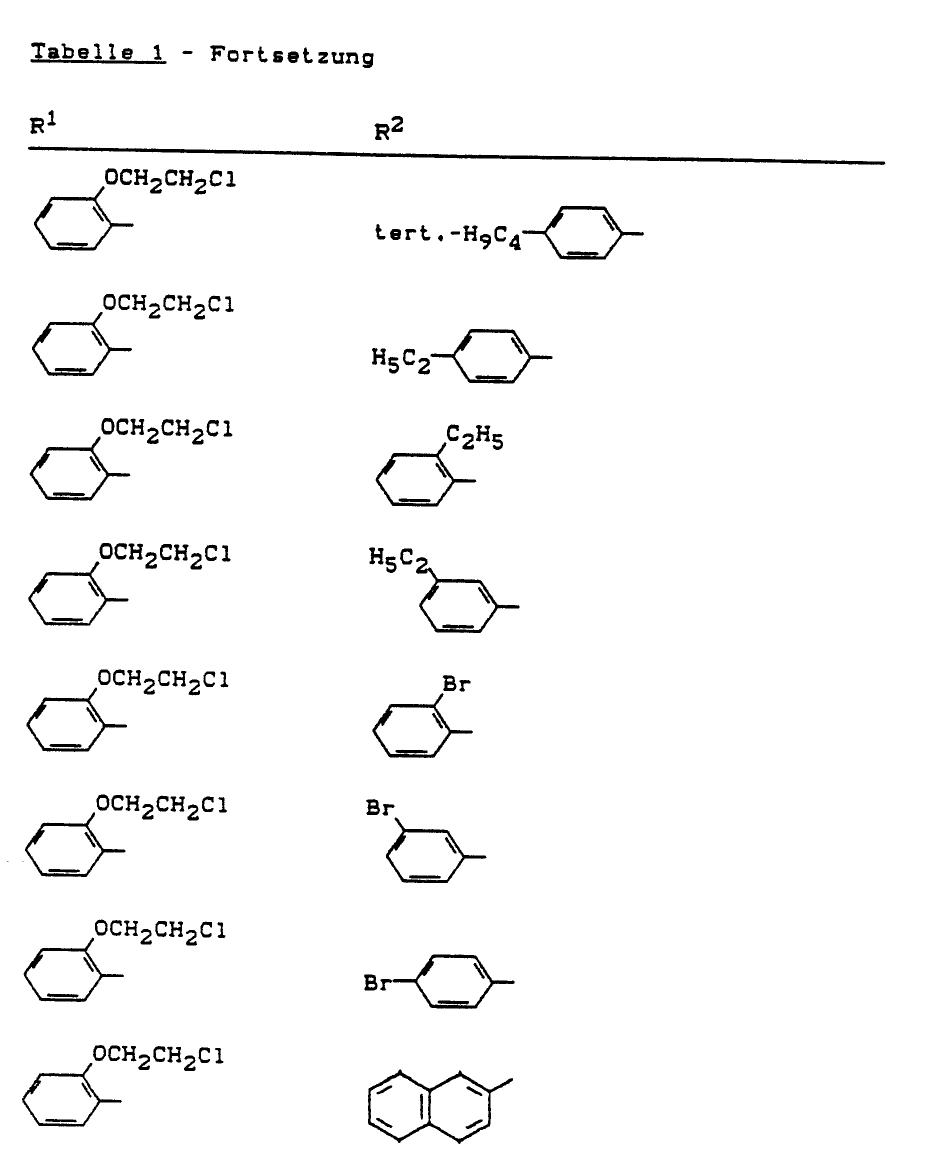

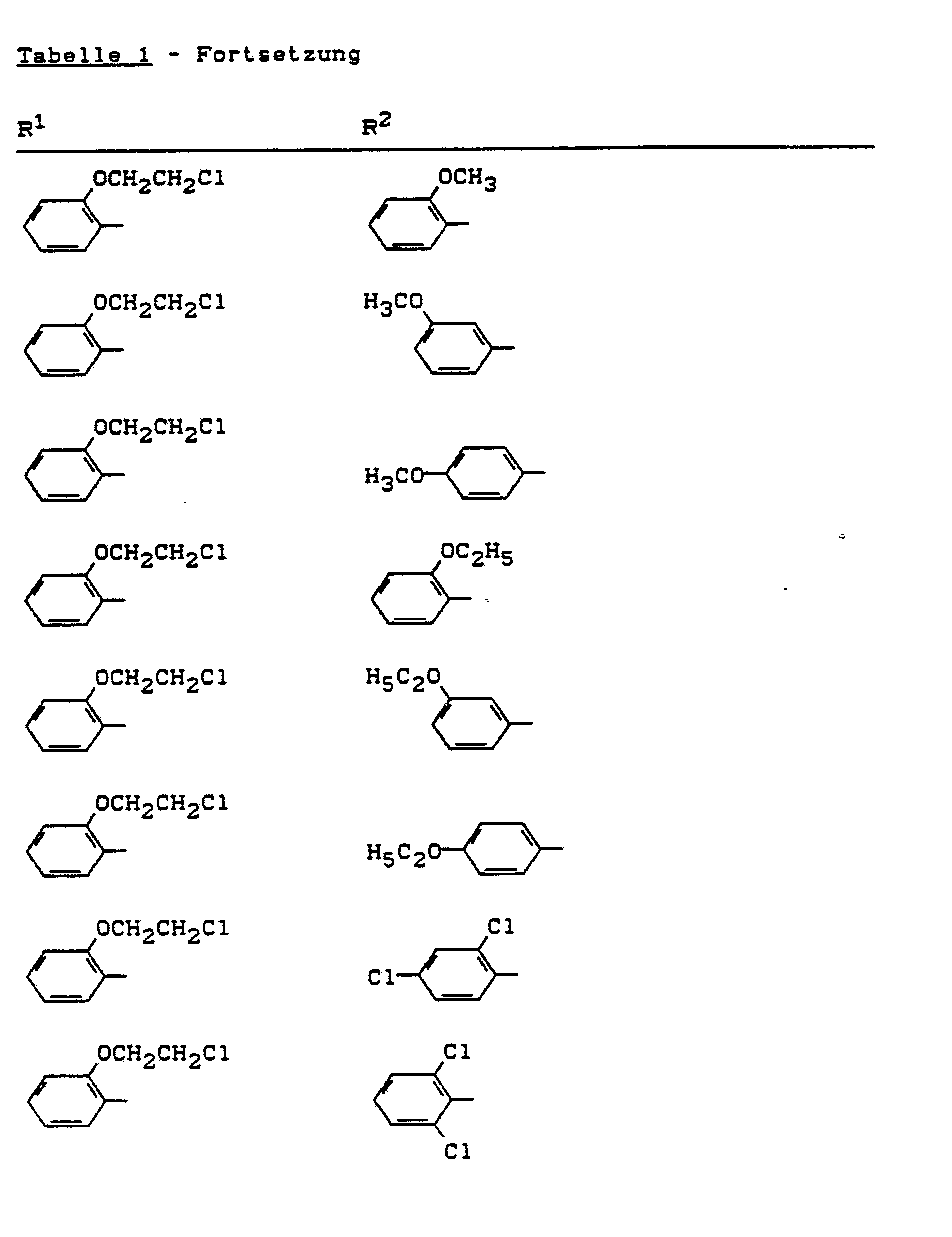

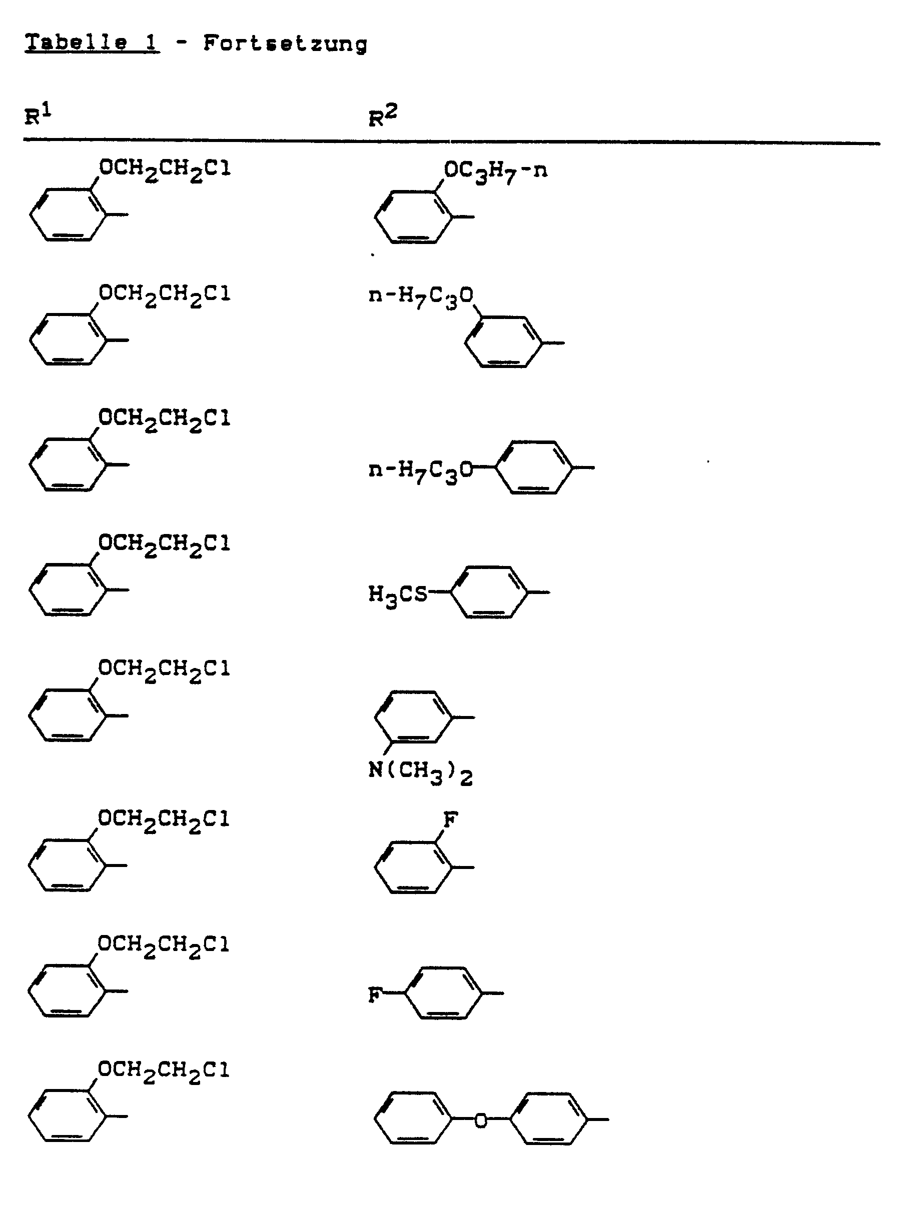

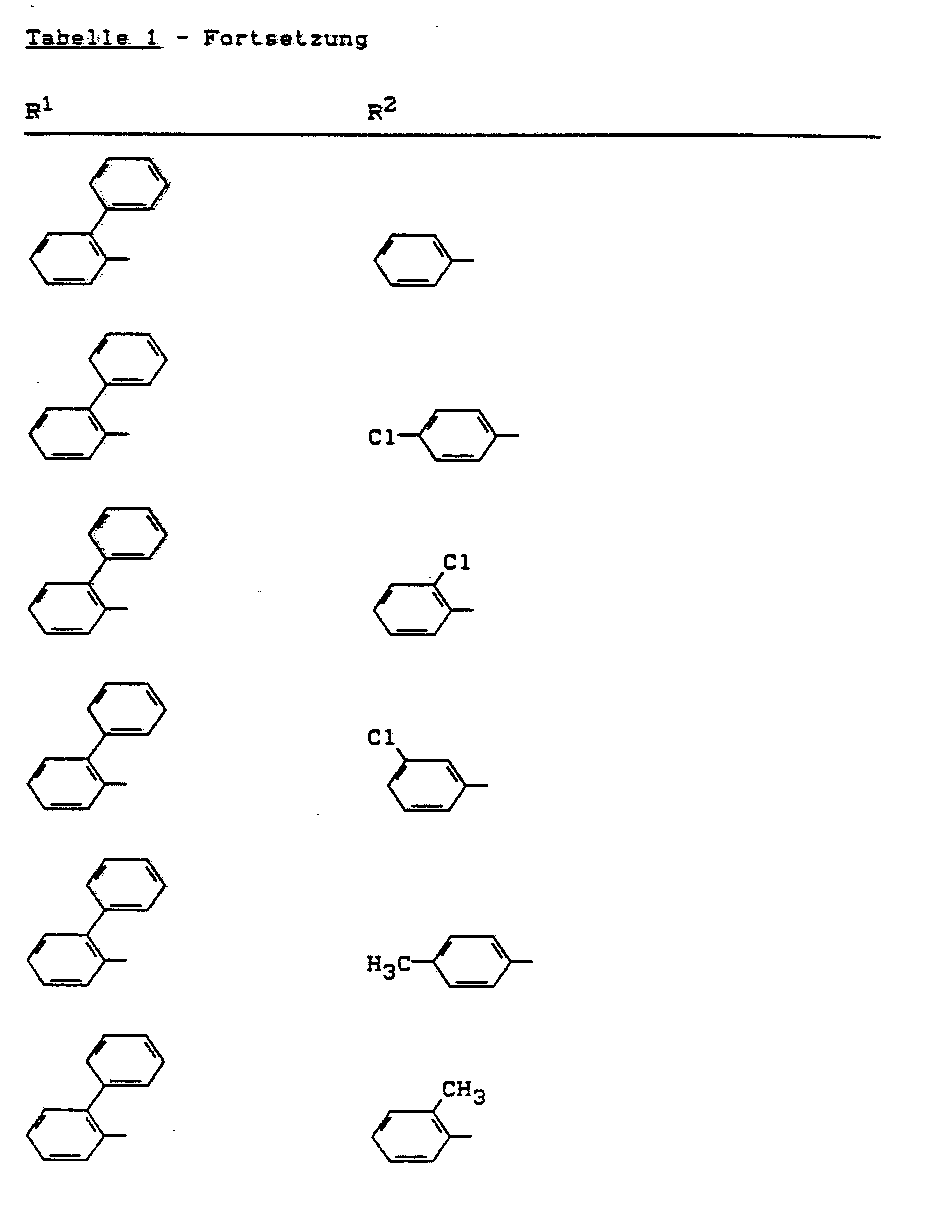

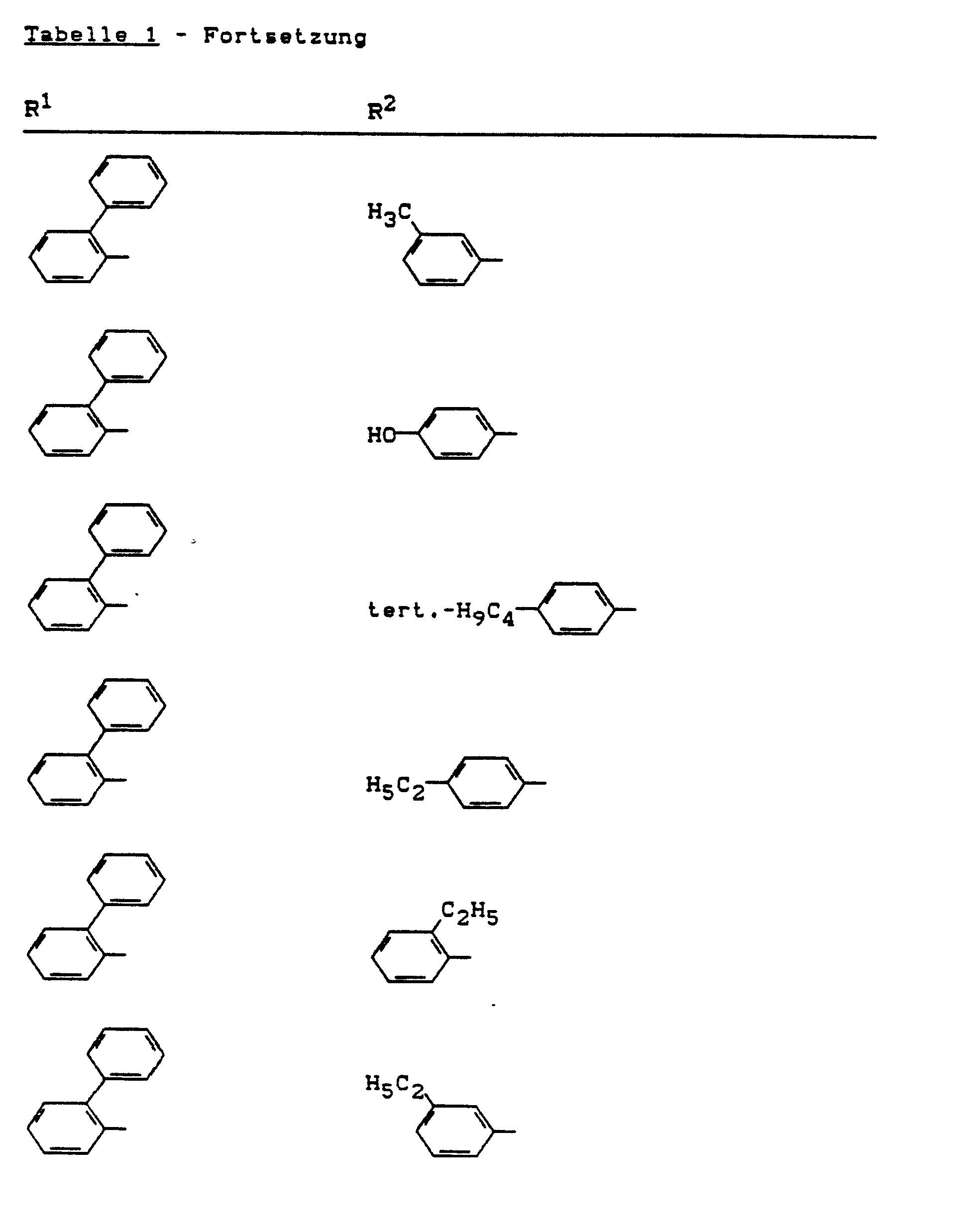

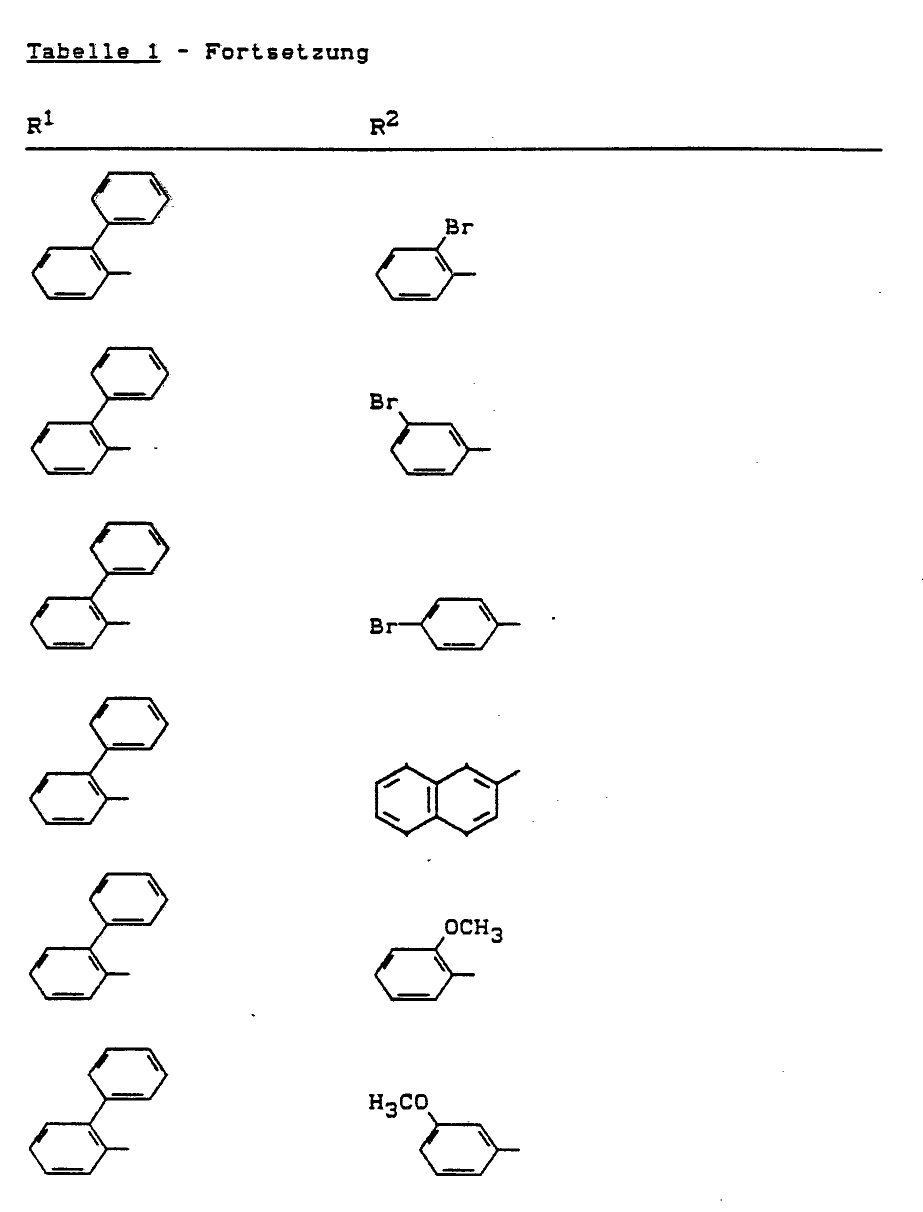

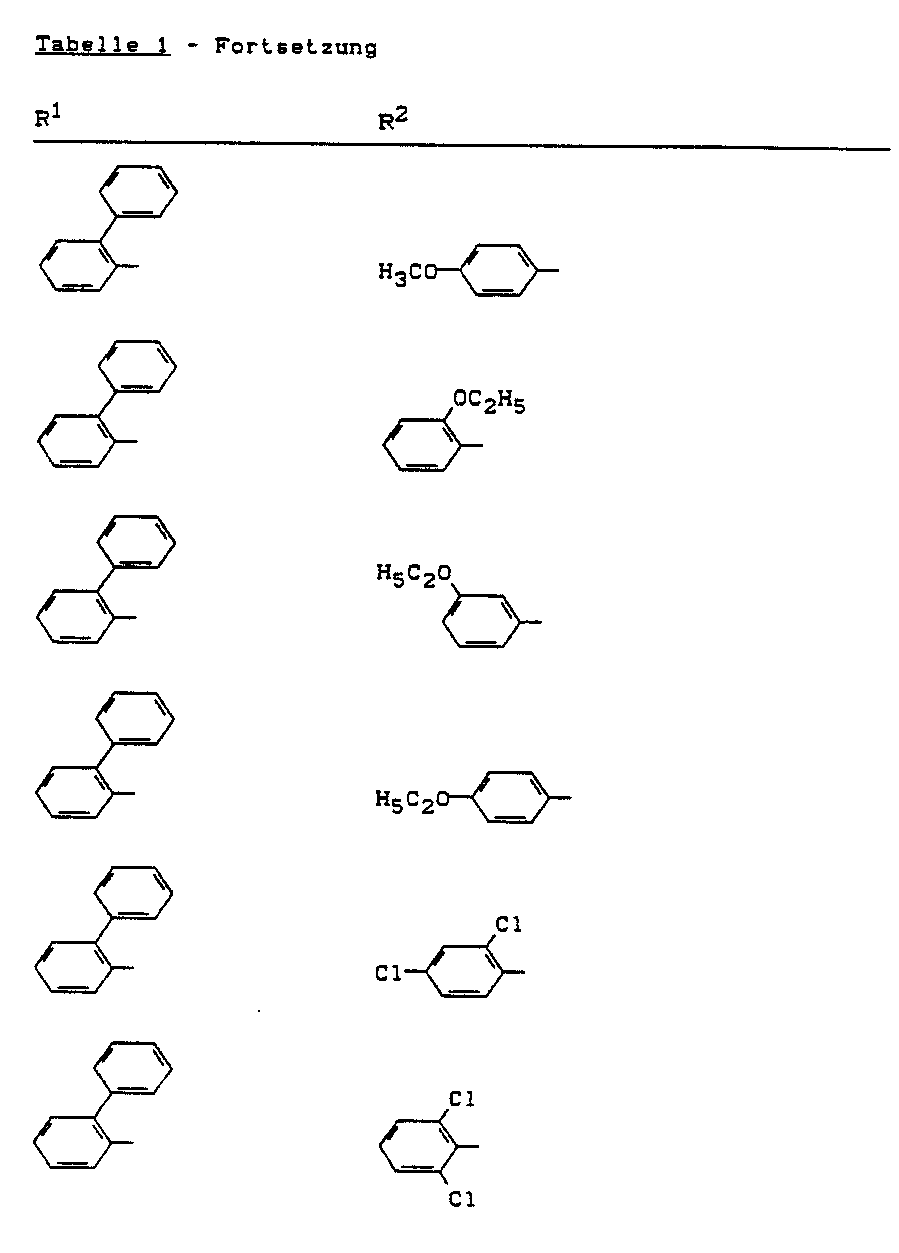

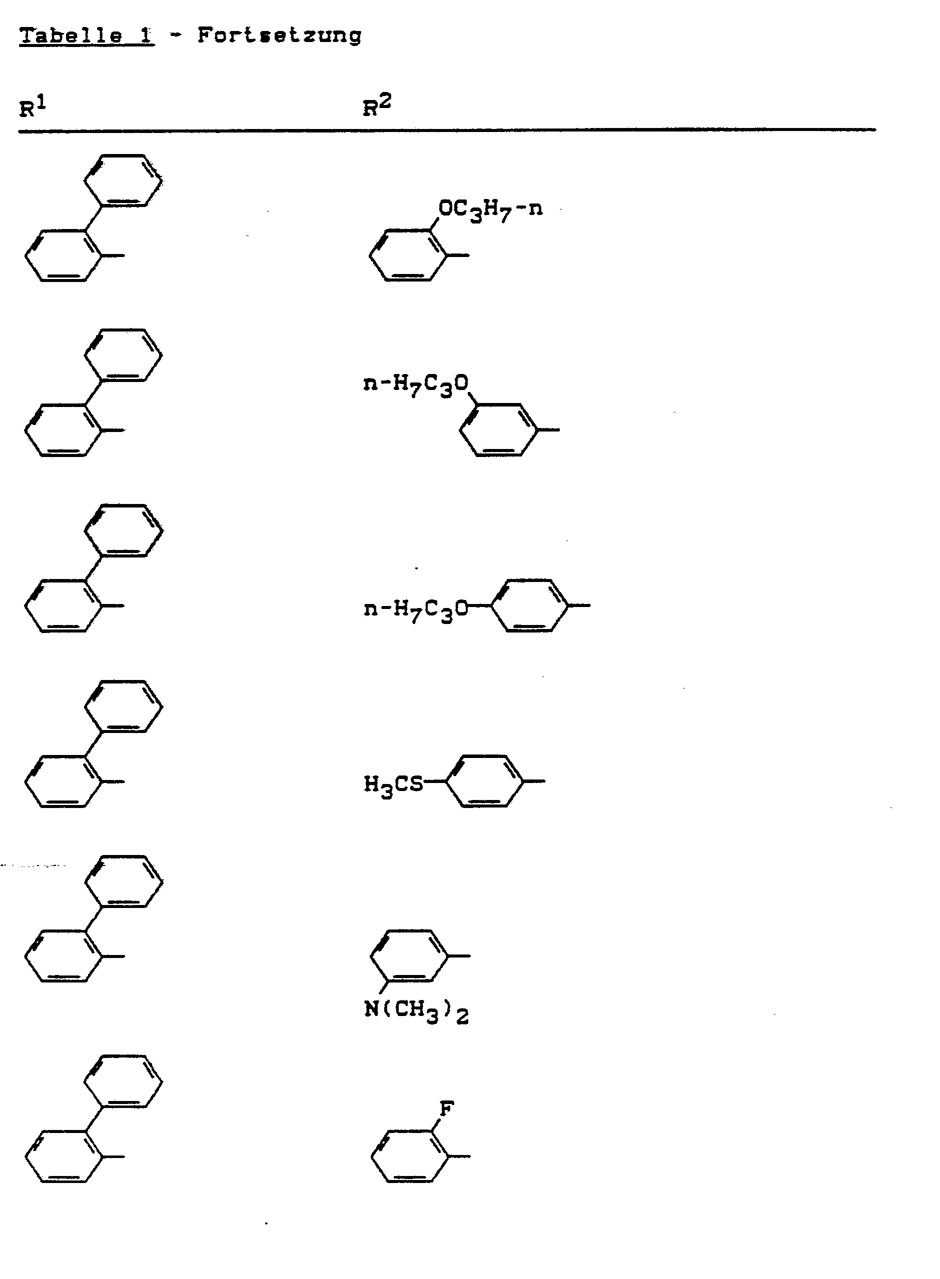

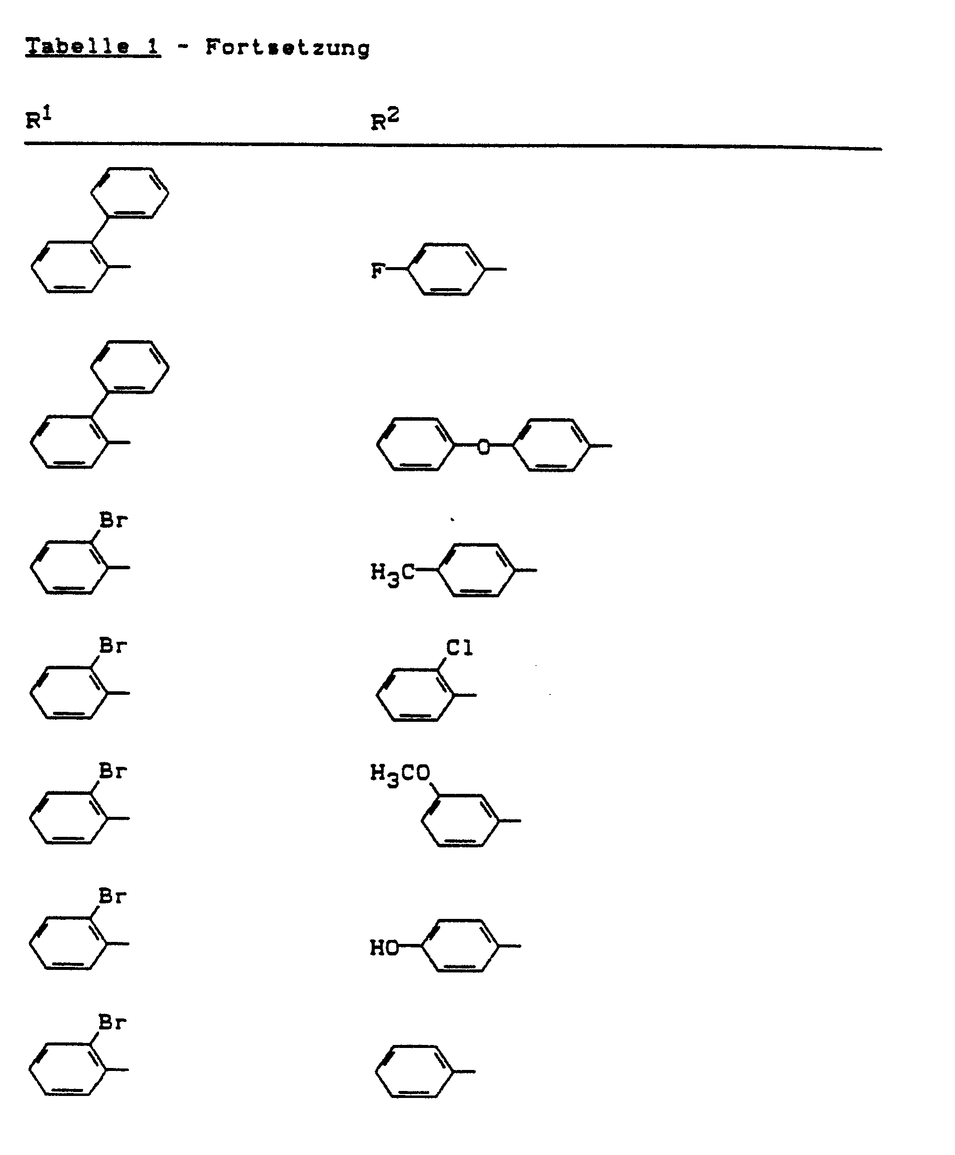

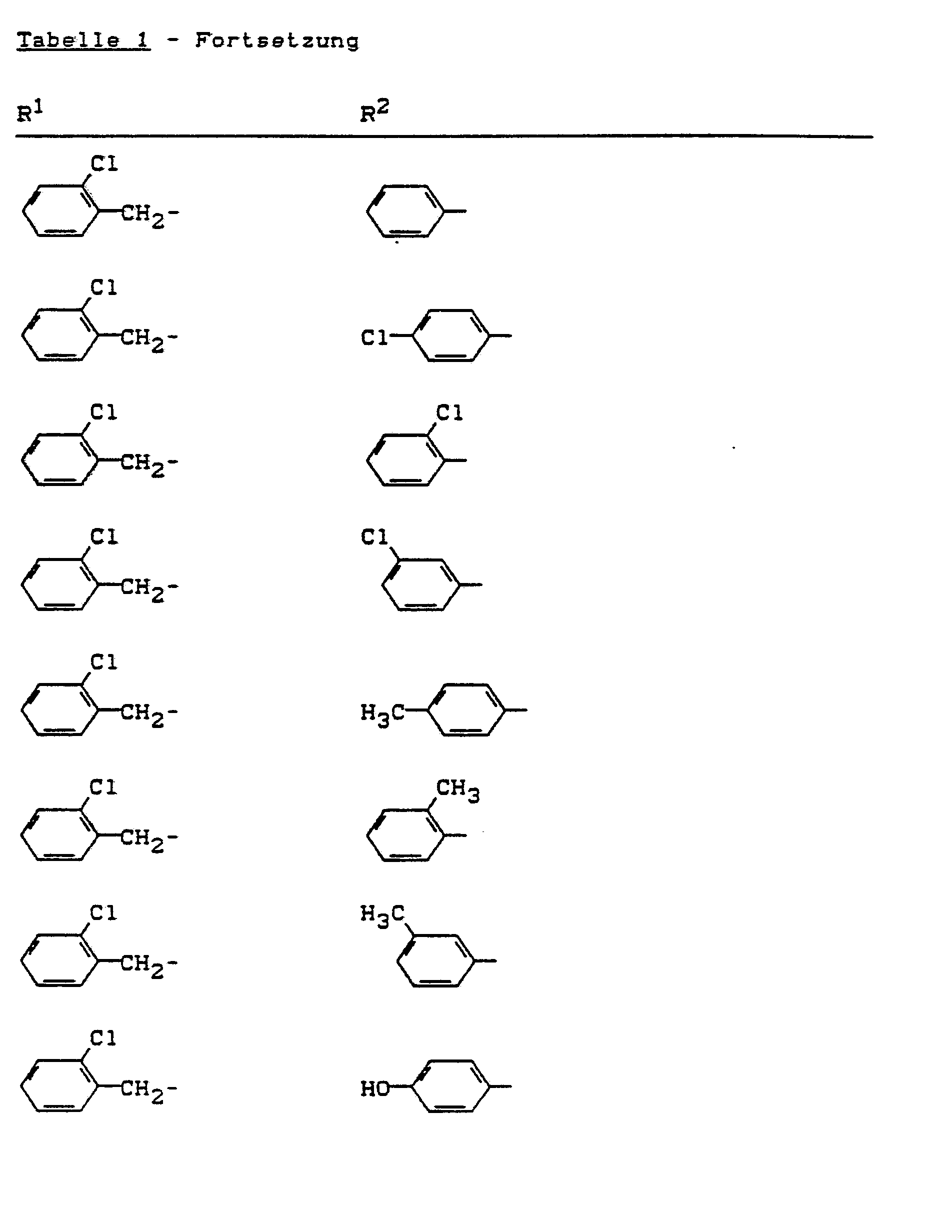

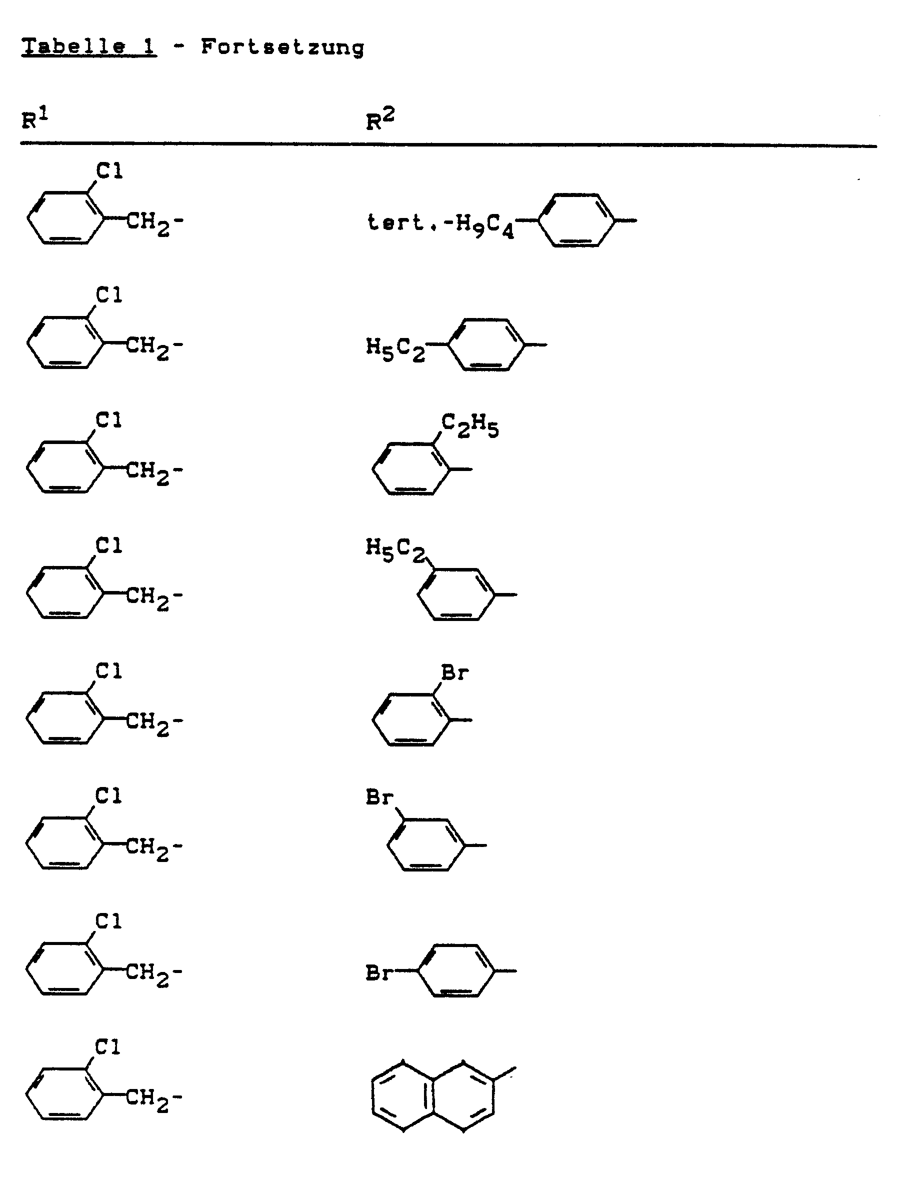

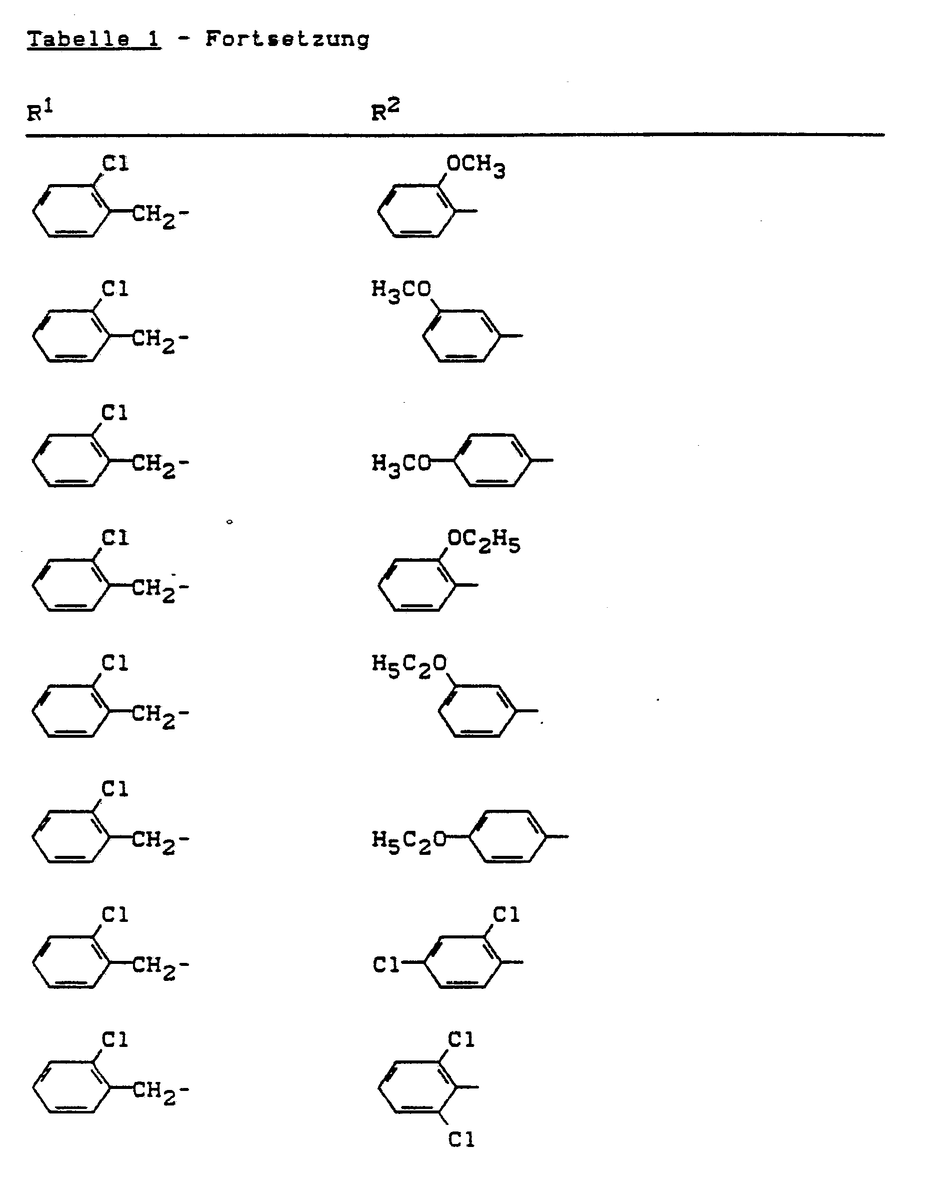

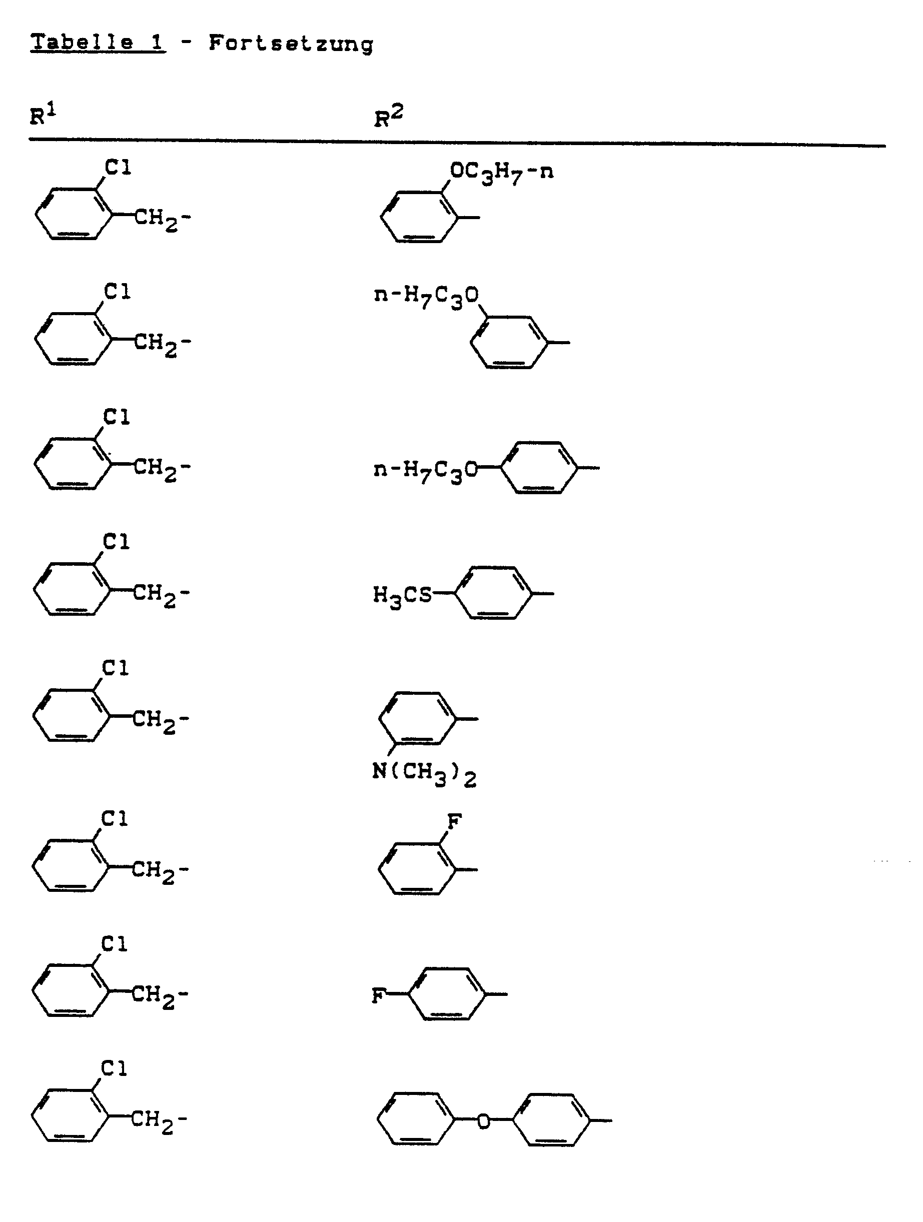

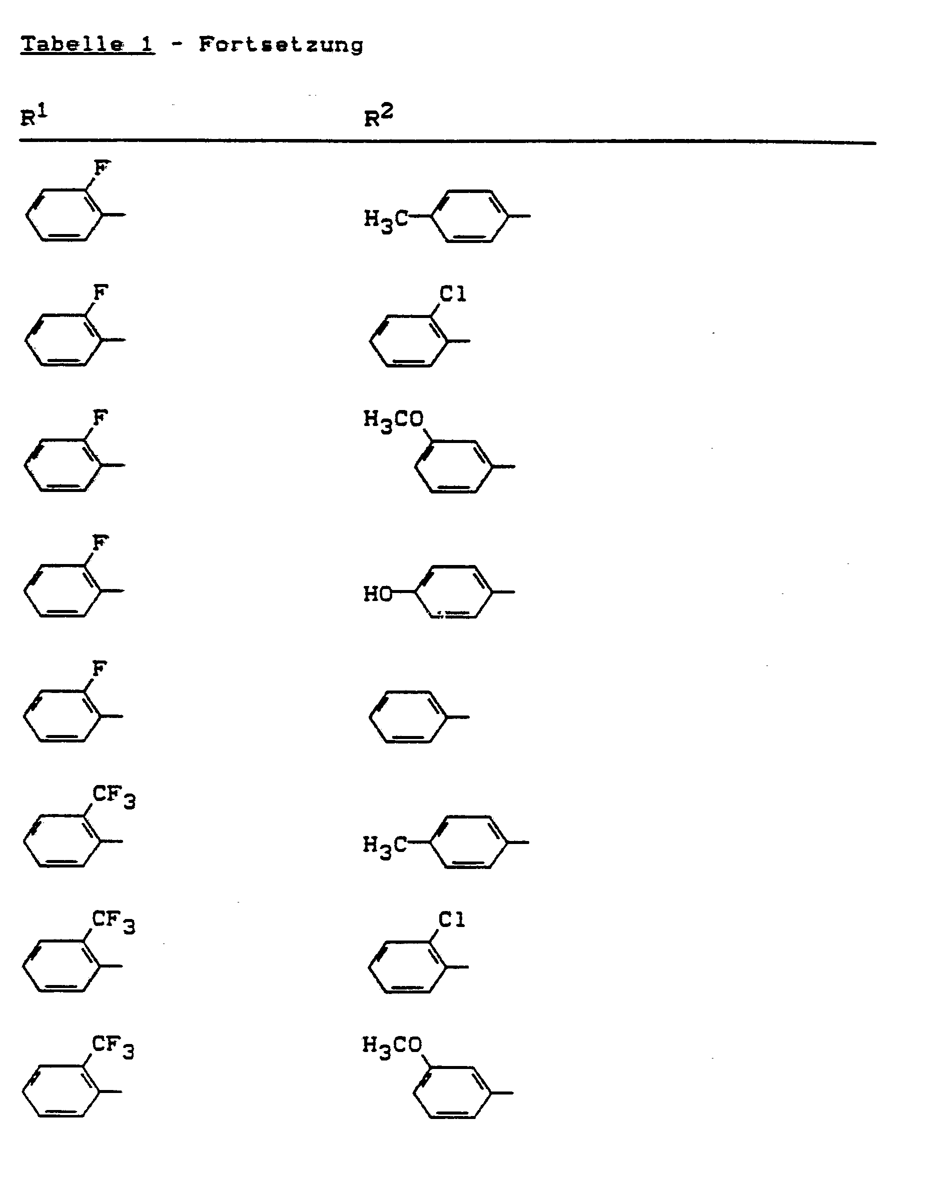

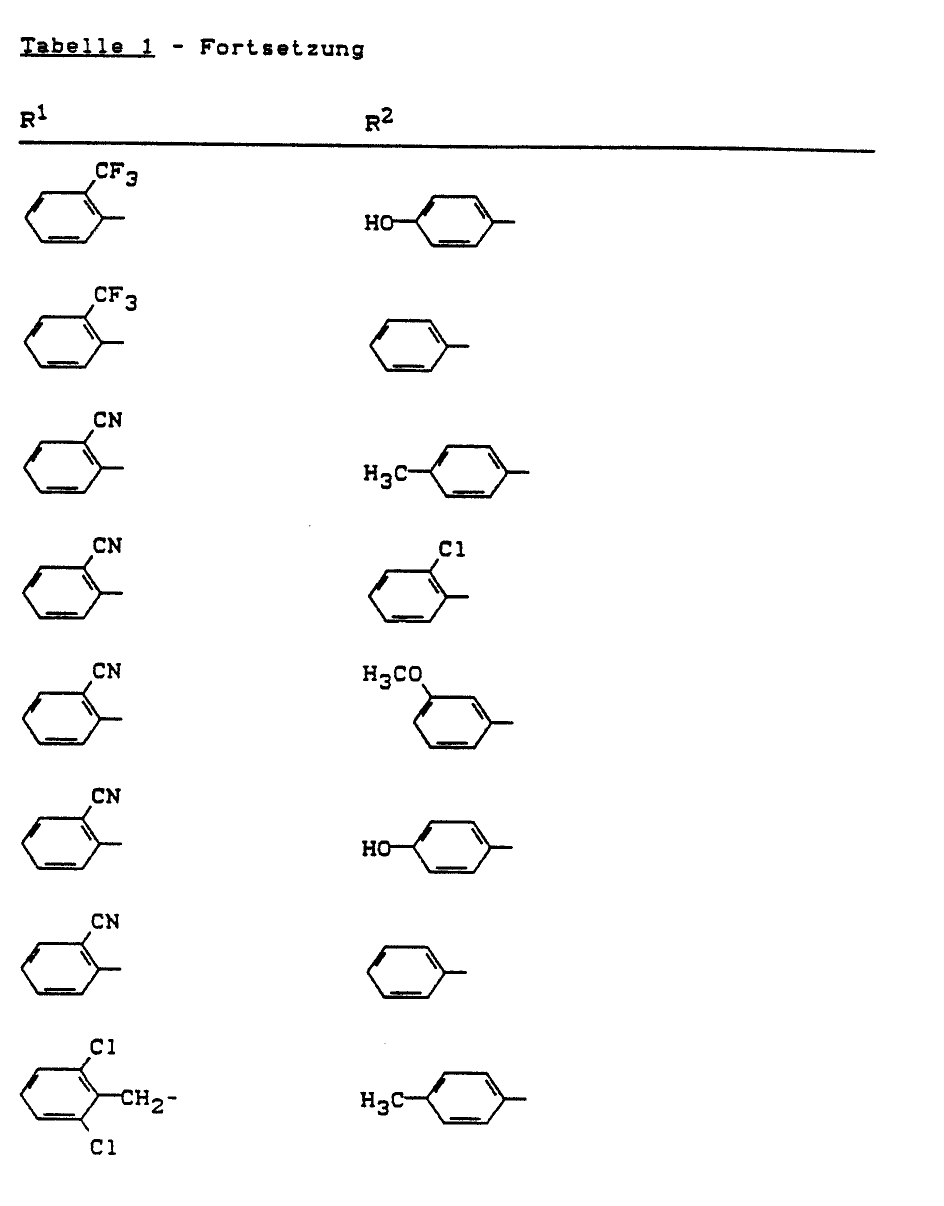

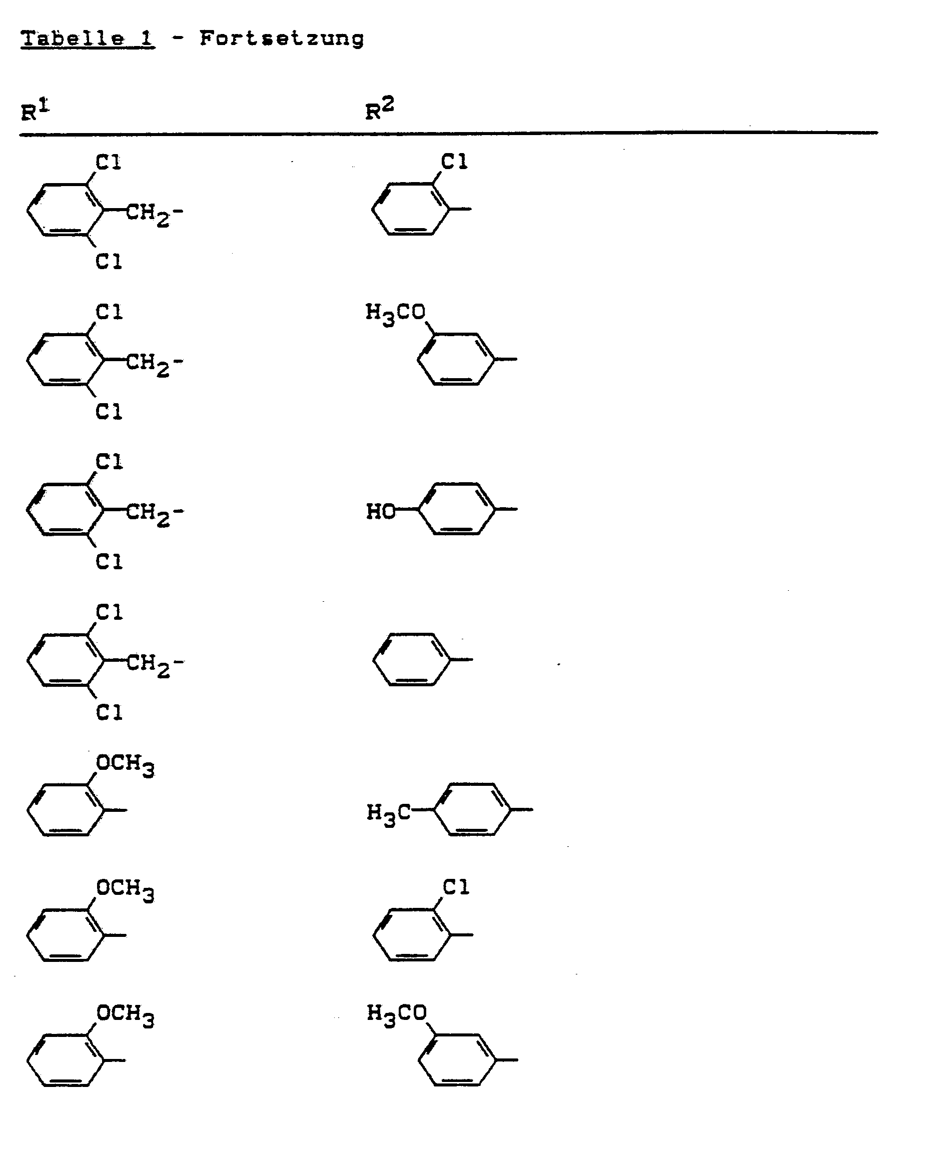

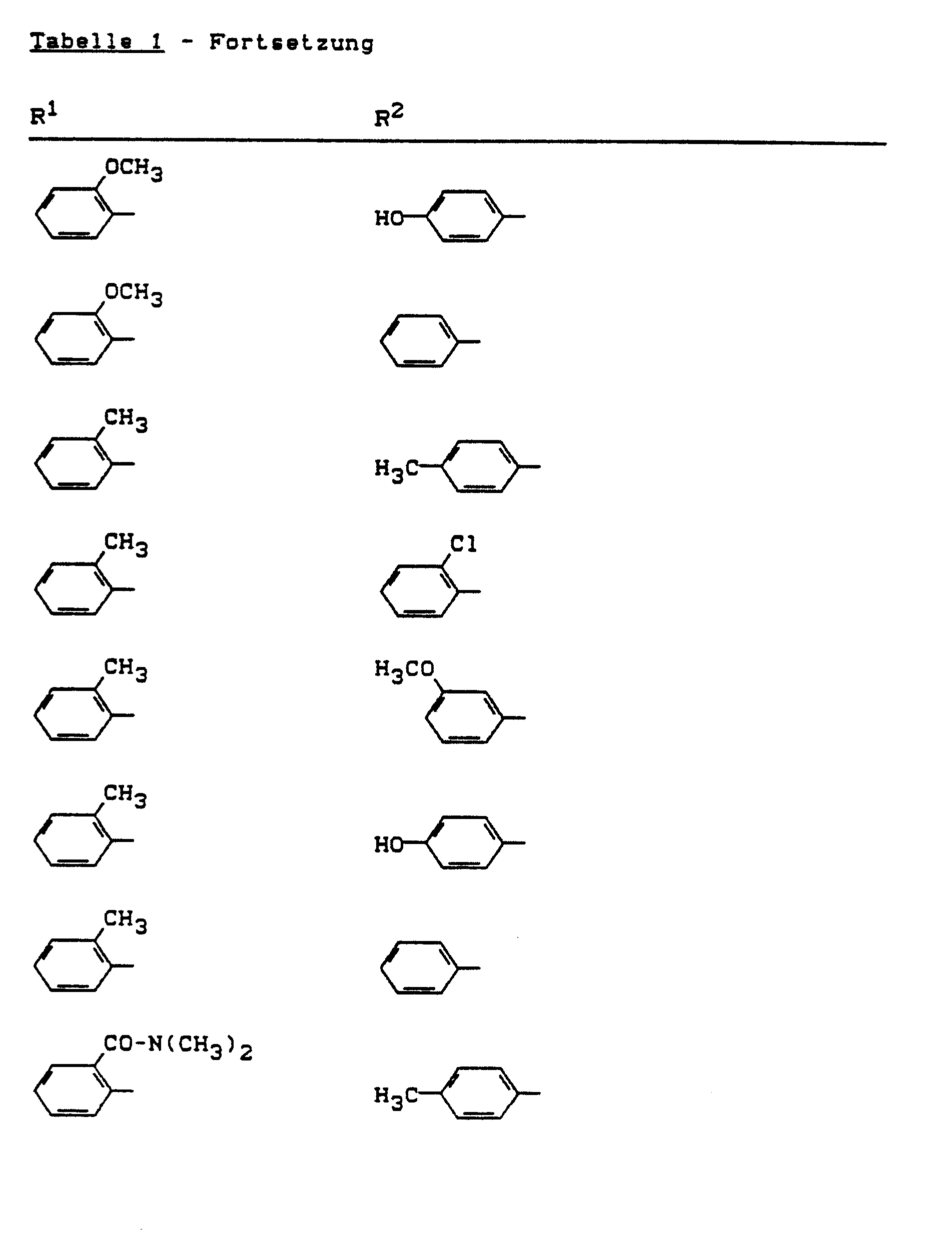

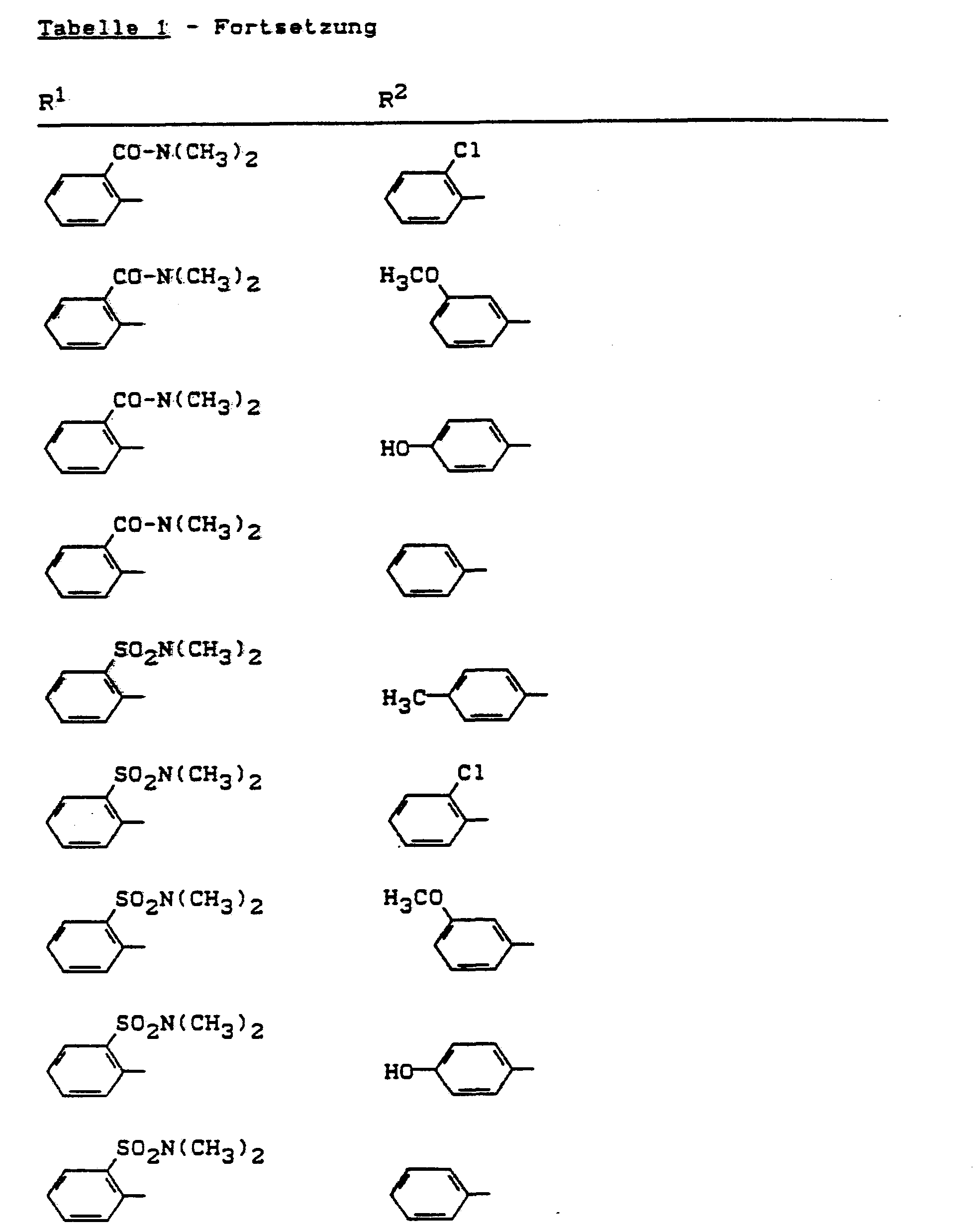

Man erhält die Verbindungen der Formel (II), wenn man Iminokohlensäuredichloride der Formel (IV) 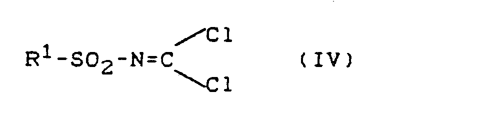 in welcher
R¹ die oben angegebenen Bedeutungen hat, mit Verbindungen der Formel (V)
R²-OM (V)
   in welcher
R² die oben angegebenen Bedeutungen hat und
M für Wasserstoff oder ein Alkalimetallatom steht, gegebenenfalls in Gegenwart von Säureakzeptoren, wie z. B. Natrium-oder Kaliumhydroxid, und gegebenenfalls in Gegenwart von inerten Verdünnungsmitteln, wie z. B. Aceton, Methylethylketon oder Acetronitril, bei Temperaturen zwischen 10 °C und 100°C umsetzt.

Die als Ausgangsstoffe für die Herstellung der Verbindungen der Formel (II) einzusetzenden Iminokohlensäuredichloride sind durch die Formel (IV) allgemein definiert. In dieser Formel steht R¹ vorzugsweise für diejenigen Reste, die bereits im Rahmen der Substituentendefinition der Formel (I) vorzugsweise genannt wurden.

Die Iminokohlensäuredichloride der Formel (IV) sind zum Teil bekannt und/oder lassen sich nach bekannten Verfahren herstellen (vergl. z. B. Chem. Ber. 99, 2900 (1966), CA 90:137826b; Angew. Chem. 77, 430 (1965)). Neu sind die Iminokohlensäuredichloride der Formel (IVa) 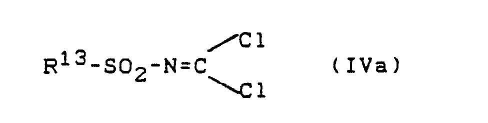 in welcher
R¹³ für den Rest 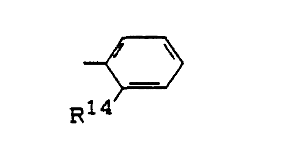 steht. worin
R¹⁴ für Halogen [wie insbesondere Fluor, Chlor, Brom und/oder lod], Cyano, C₂-C₆-Alkyl, C₁-C₆-Alkyl [welches durch Fluor, Chlor, Brom, Cyano, C₁-C₄-Alkoxy, C₃-C₆-Cycloalkyl oder Phenyl substituiert ist], für C₁-C₄-Alkoxy [welches gegebenenfalls durch Fluor, Chlor, Brom, Cyano oder C₁-C₄-Alkoxy substituiert ist], für Di-(Ci-C₄-alkyl)-aminosulfonyl, für Di-(C₁-C₄-alkyl)-amino-carbonyl, für Phenyl oder Phenoxy, für C₁-C₄-Alkylcarbonylamino, Ci-C₄-Alkylamino-carbonyl-amino, Di-(C₁-C₄-alkyl)-amino-carbonylamino, Di-(C₁-C₄-alkyl)-aminocarbonyl oder für Di-(C₁-C₄-alkyl)-aminosulfonylamino steht, worin weiter
R'3 für den Rest 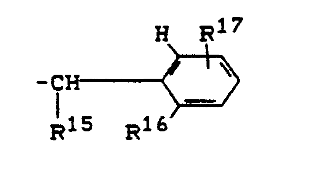 steht, worin
R¹⁵ für Wasserstoff oder C₁-C₄-Alkyl steht,
R¹⁶ und R¹⁷ gleich oder verschieden sind und für Wasserstoff, Fluor, Chlor, Brom, Nitro, Cyano, C₁-C₄-Alkyl [welches gegebenenfalls durch Fluor und/oder Chlor substituiert ist], C₁-C₄-Alkoxy [welches gegebenenfalls durch Fluor und/oder Chlor substituiert ist] oder Di-(C₁-C₄-alkyl)-aminosulfonyl stehen; worin weiter
R¹³ für den Rest 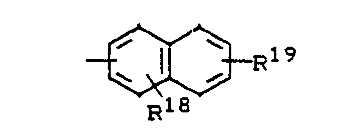 steht, worin
R¹⁸ und R¹⁹ gleich oder verschieden sind und für Wasserstoff, Fluor, Chlor, Brom. Nitro, Cyano. C₁-C₄-Alkyl [welches gegebenenfalls durch Fluor und/oder Chlor substituiert ist] oder C₁-C₄-Alkoxy [welches gegebenenfalls durch Fluor und/oder Chlor substituiert ist], stehen.

Man erhält die neuen Verbindungen der Formel (IVa), wenn man Iminodithiokohlensäuredimethylester der Formel (VI) 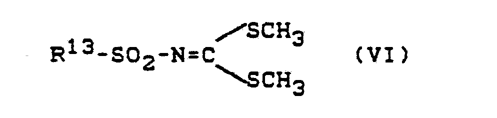 in welcher
R¹³ die oben bei Formel (IVa) angegebenen Bedeutungen hat,
   in Gegenwart von inerten Verdünnungsmitteln, wie z. B. Tetrachlorkohlenstoff, bei Temperaturen zwischen 0 °C und 25 °C mit einem Chlorierungsmittel, wie z.B. Sulfurylchlorid oder Chlorgas, umsetzt.

Die Iminodithiokohlensäuredimethylester der Formel (VI) sind bekannt und/oder lassen sich nach bekannten Methoden herstellen (vergl. z. B. EP-A 121 082, EP-A 151 554 und EP-A 173 957).

Als Beispiele für die neuen Iminokohlensäuredichloride der Formel (IVa) seien genannt:

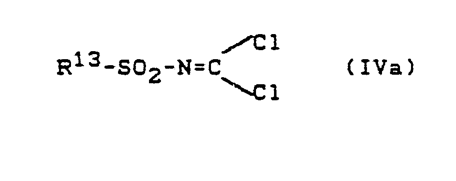

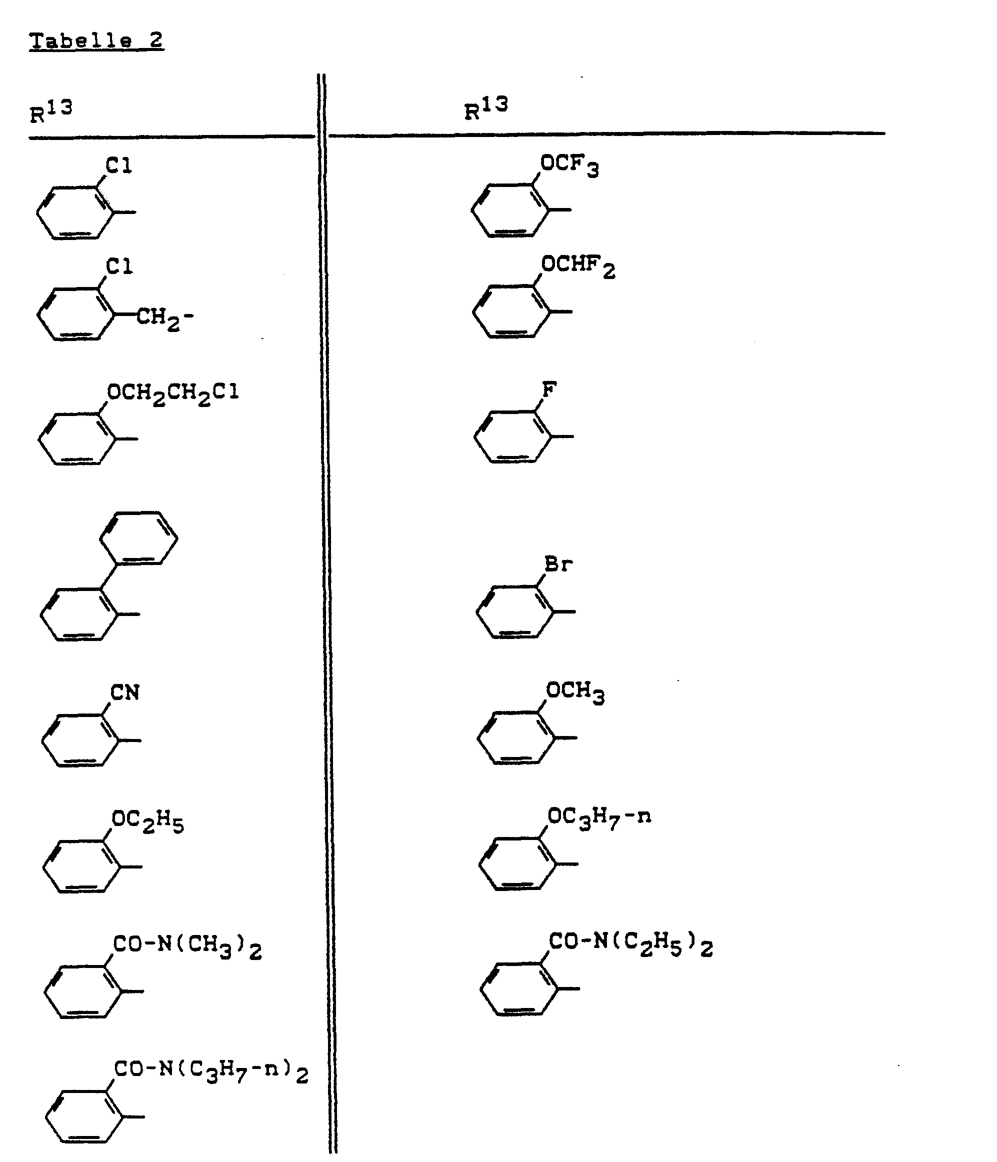

Die weiterhin als Ausgangsstoffe für die Herstellung der Verbindungen der Formel (11) einzusetzenden Verbindungen sind durch die Formel (V) allgemein definiert. In dieser Formel steht R² vorzugsweise für diejenigen Reste, die bereits im Rahmen der Substituentendefinition der Formel (I) vorzugsweise genannt wurden. M steht in dieser Formel für Wasserstoff oder ein Alkalimetallatom, wie vorzugsweise Natrium-oder Kalium. Für den Fall, daß M für ein Alkalimetallatom steht, wird das Verfahren zur Herstellung der Verbindungen der Formel (II) bevorzugt ohne Säureakzeptor durchgeführt.

Die Verbindungen der Formel (V) sind allgemein bekannte Verbindungen der organischen Chemie.

Die beim erfindungsgemäßen Verfahren außerdem als Ausgangsstoffe zu verwendenden Amine sind durch die Formel (111) allgemein definiert. In dieser Formel (III) stehen R³, R⁴ und R⁵ vorzugsweise für diejenigen Reste, die bereits im Rahmen der Substituentendefinition der Formel (I) vorzugsweise genannt wurden.

Als Beispiele für die Amine der Formel (III) seien genannt:

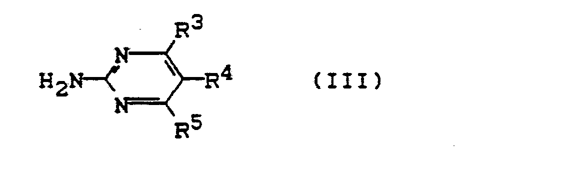

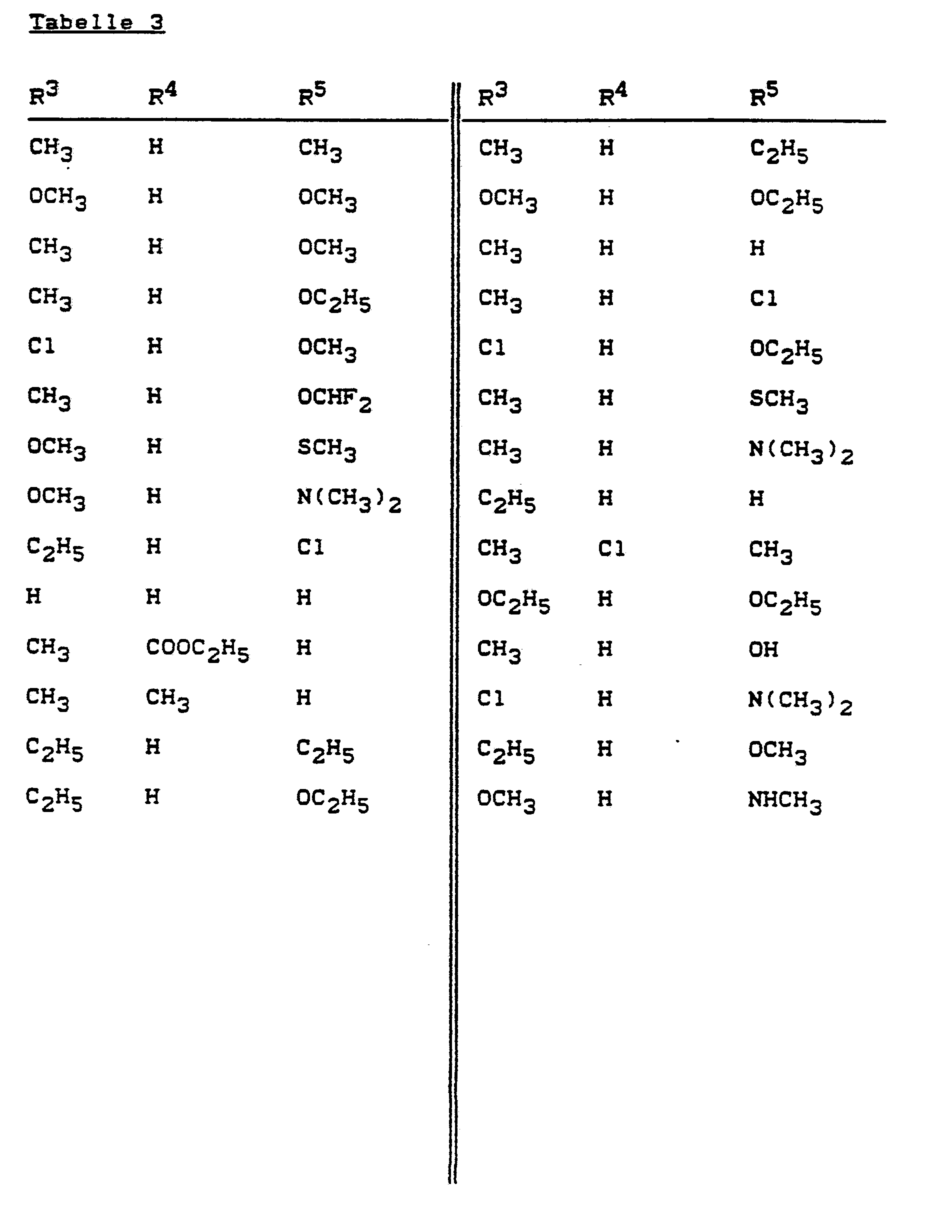

Die Amine der Formel (111) sind bekannt und/oder können nach an sich bekannten Verfahren hergestellt werden (vergl. z. B. Chem. Pharm. Bull. 11 (1963), 1382 - 1388; J. Chem. Soc. 1946, 81; US-PS 4 299 960 und EP-A 173 957).

Das erfindungsgemäße Verfahren wird vorzugsweise unter Verwendung von Verdünnungsmitteln durchgeführt. Als Verdünnungsmittel kommen dabei praktisch alle inerten organischen Lösungsmittel in Frage. Hierzu gehören vorzugsweise aliphatische und aromatische, gegebenenfalls halogenierte Kohlenwasserstoffe wie Pentan, Hexan, Heptan, Cyclohexan, Petrolether, Benzin, Ligroin, Benzol, Toluol, Xylol, Methylenchlorid, Ethylenchlorid, Chloroform, Tetrachlorkohlenstoff, Chlorbenzol und o-Dichlorbenzol, Ether wie Diethyl-und Dibutylether, Glykoldimethylether und Diglykoldimethylether, Tetrahydrofuran und Dioxan, Ketone wie Aceton, Methyl-ethyl-, Methyl-isopropyl-und Methyl-isobutyl-keton, Ester wie Essigsäuremethylester und -ethylester, Nitrile wie z. B. Acetonitril und Propionitril, Amide wie z. B. Dimethylformamid, Dimethylacetamid und N-Methyl-pyrrolidon sowie Dimethylsulfoxid, Tetramethylensulfon und Hexamethylphosphorsäuretriamid.

Als Säureakzeptoren können bei dem erfindungsgemäßen Verfahren zur Herstellung der Verbindungen der Formel (I) alle üblicherweise für derartige Umsetzungen verwendbaren Säurebindemittel eingesetzt werden. Vorzugsweise in Frage kommen Alkalimetallhydroxide wie z. B. Natrium-und Kaliumhydroxid, Erdalkalihydroxide wie z. B. Calciumhydroxid, Alkalicarbonate und -alkoholate wie Natrium-und Kaliumcar bonat, Natrium-und Kaliummethylat bzw. -ethylat oder Kalium-tert.-butylat.

Die Reaktionen werden im allgemeinen bei Temperaturen zwischen -20°C und 150°C, vorzugsweise bei Temperaturen zwischen 0°C und 80°C durchgeführt. Die Reaktionen werden im allgemeinen bei Normaldruck durchgeführt.

Zur Durchführung des erfindungsgemäßen Verfahrens werden die Ausgangsstoffe der Formeln (11) und (111) und gegebenenfalls der geeignete Säureakzeptor in äquimolaren Mengen eingesetzt.

Bevorzugt werden eine Verbindung der Formel (III), der Säureakzeptor und das Verdünnungsmittel wie z. B. Tetrahydrofuran vorgelegt und einige Stunden gerührt. Anschliessend wird die Verbindung der Formel (11) zugegeben. Nach der Umsetzung wird die Reaktionslösung eingeengt, mit Wasser versetzt, über Kieselgel abgesaugt und mit einer Mineralsäure wie z. B. Salzsäure schwach angesäuert. Die Lösung wird in einem organischen Verdünnungsmittel wie z. B. Methylenchlorid aufgenommen. Die organische Phase wird mit gesättigter Natriumcarbonatlösung und anschließend mit Wasser gewaschen, getrocknet und eingeengt. Die Reinigung der Rohprodukte geschieht nach üblichen Methoden.

Die nach diesem Verfahren leicht zugänglichen Verbindungen der Formel (I) sind bekannt und lassen sich als Pflanzenschutzmittel, insbesondere als Herbizide einsetzen (vergl. z. B. EP-A 173 957).

### Herstellungsbeispiele

### Beispiel 1

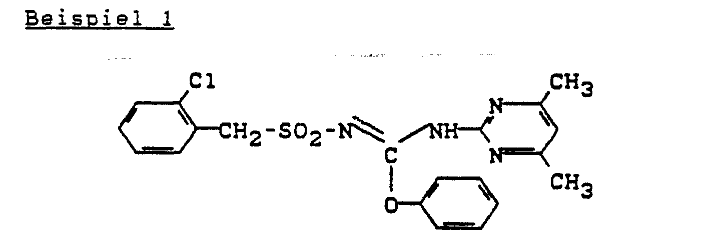

3,4 g (0,027 Mol) 2-Amino-4,6-dimethyl-pyrimidin in 100 ml Tetrahydrofuran werden bei 20 °C mit 3,2 g (0,027 Mol) Kalium-tert.-butylat versetzt und 3 Stunden bei 20 °C gerührt. Anschließend werden 11 g (0,027 Mol) N-(2-Chlorbenzylsulfonyl)-iminokohlensäurediphenylester zugegeben, wobei die Temperatur auf 35 °C ansteigt. Nach Abklingen der exothermen Reaktio wird das Reaktionsgemisch 15 Stunden bei 20 °C weitergerührt. Das Reaktionsgemisch wird abfiltriert und eingeengt. Der Rückstand wird in Wasser aufgenommen, über Kieselgur abgesaugt und das Filtrat mit verdünnter Salzsäure schwach angesäuert. Die wäßrige Lösung wird mit Methylenchlorid extrahiert. Die organische Phase wird mit gesättigter Natriumhydrogencarbonatlösung und mit Wasser gewaschen, getrocknet und im Vakuum eingeengt. Der Rückstand wird aus Isopropanol umkristallisiert.

Man erhält so 9,7 g (82 % der Theorie) N'-(4,6-Dimethyl-pyrimidin-2-yl)-N"-(2-chlor-benzylsulfonyl)-O-phenylisoharnstoff vom Schmelzpunkt 152 °C.

Analog zu Beispiel 1 können beispielsweise auch alle in dem oben genannten Dokument EP-A 173 957 beschriebenen O-Aryl-N'-pyrimidin-2-yl-isoharnstoffe hergestellt werden.

### Beispiel 2

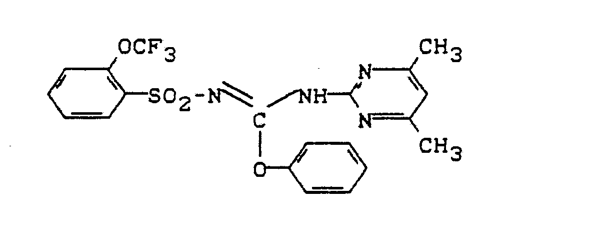

Fp: 100 °C
Ausbeute: 58,2 % der Theorie

### Ausgangsverbindungen der Formel (11)

### Beispiel (11-1)

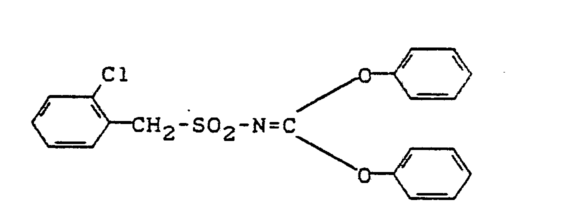

23,1 g (0,08 Mol) 2-Chlor-benzylsulfonylisocyaniddichlorid werden in 200 ml Aceton gelöst und bei 20 °C mit 18,8 g (0,16 Mol) Natriumphenolat versetzt. Die exotherme Reaktion wird gekühlt. Das Reaktionsgemisch wird über Nacht bei Raumtemperatur gerührt, anschließend abgesaugt. Das Filtrat wird im Vakuum eingeengt, der entstandene schmierige Rückstand kristallisiert langsam durch. Der Kristallbrei wird mit Diisopropylether verrieben, abgesaugt, gewaschen und getrocknet.

Man erhält 26,6 g (83 % der Theorie) 2-Chlor-benzylsulfonyliminokohlensäure-O,O-diphenylester vom Schmelzpunkt 130 °C.

Analog Beispiel (II-1) können die folgenden Verbindungen der Formel (11) hergestellt werden:

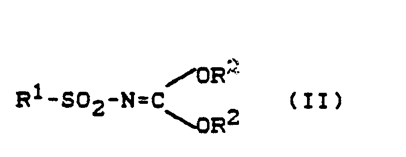

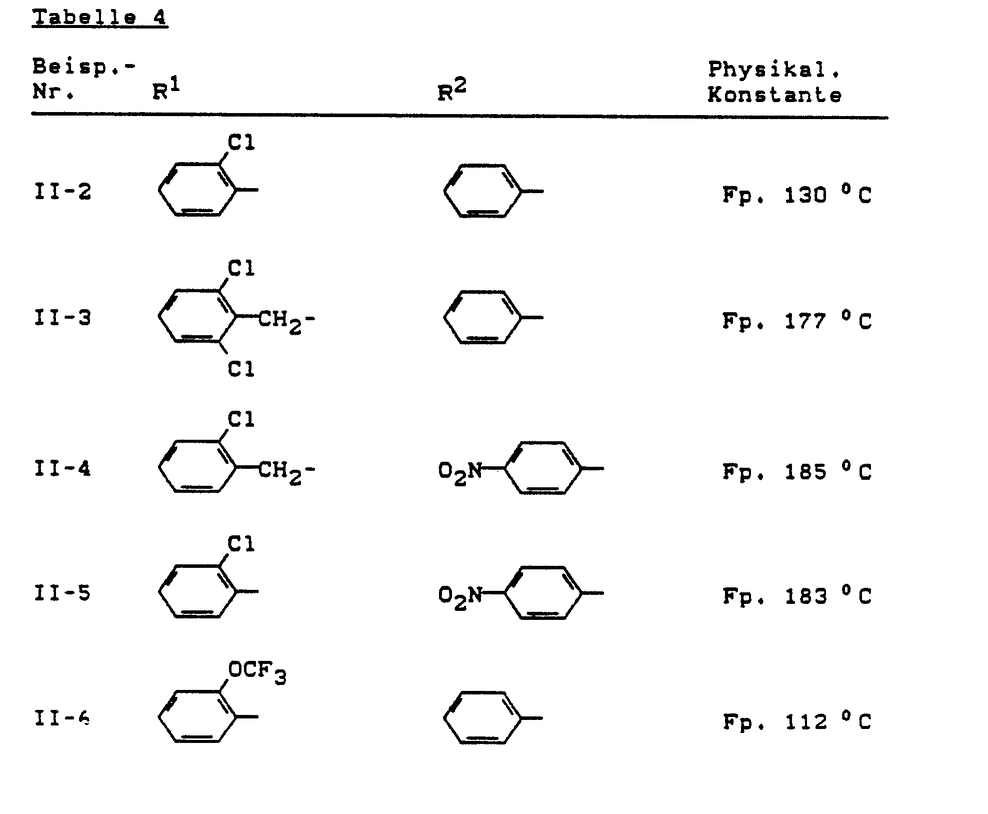

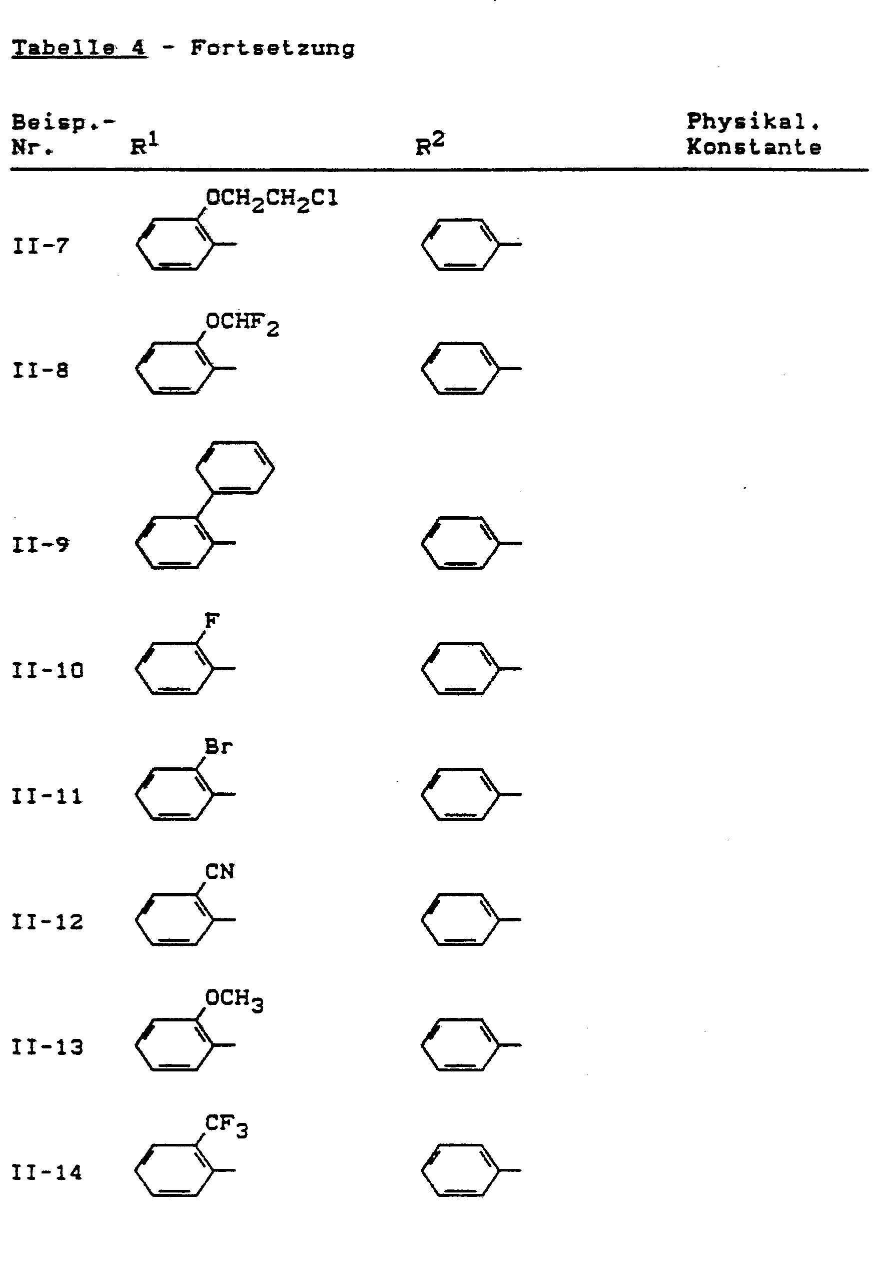

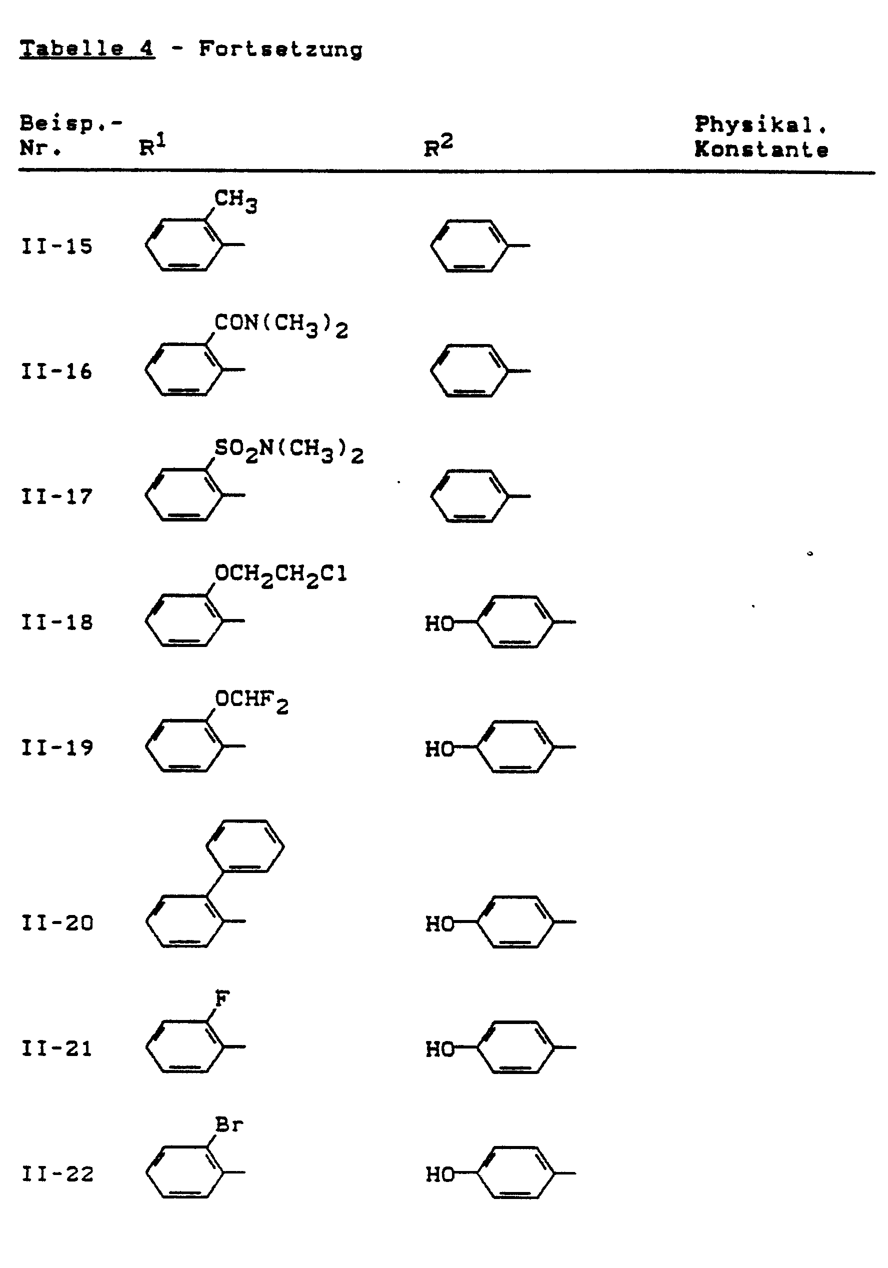

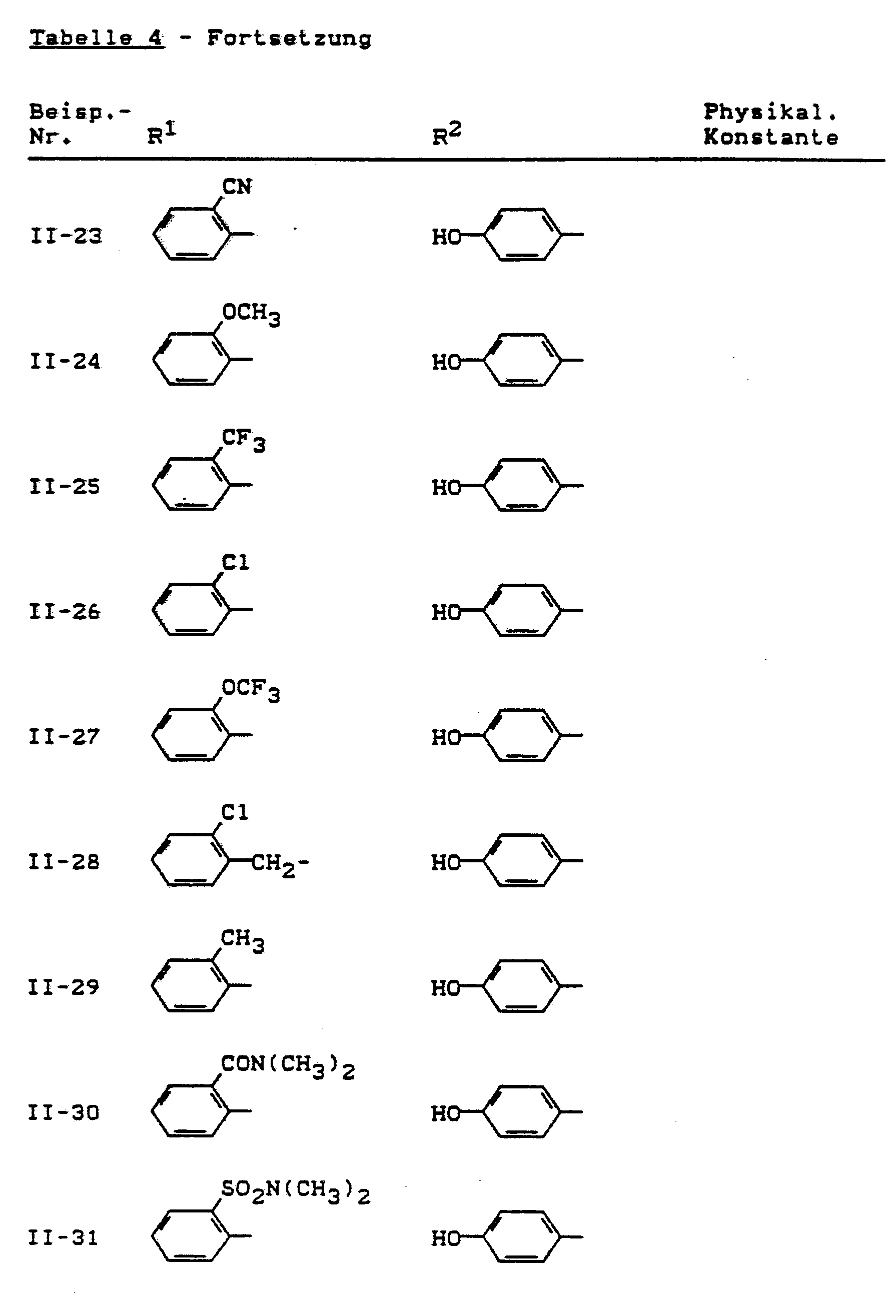

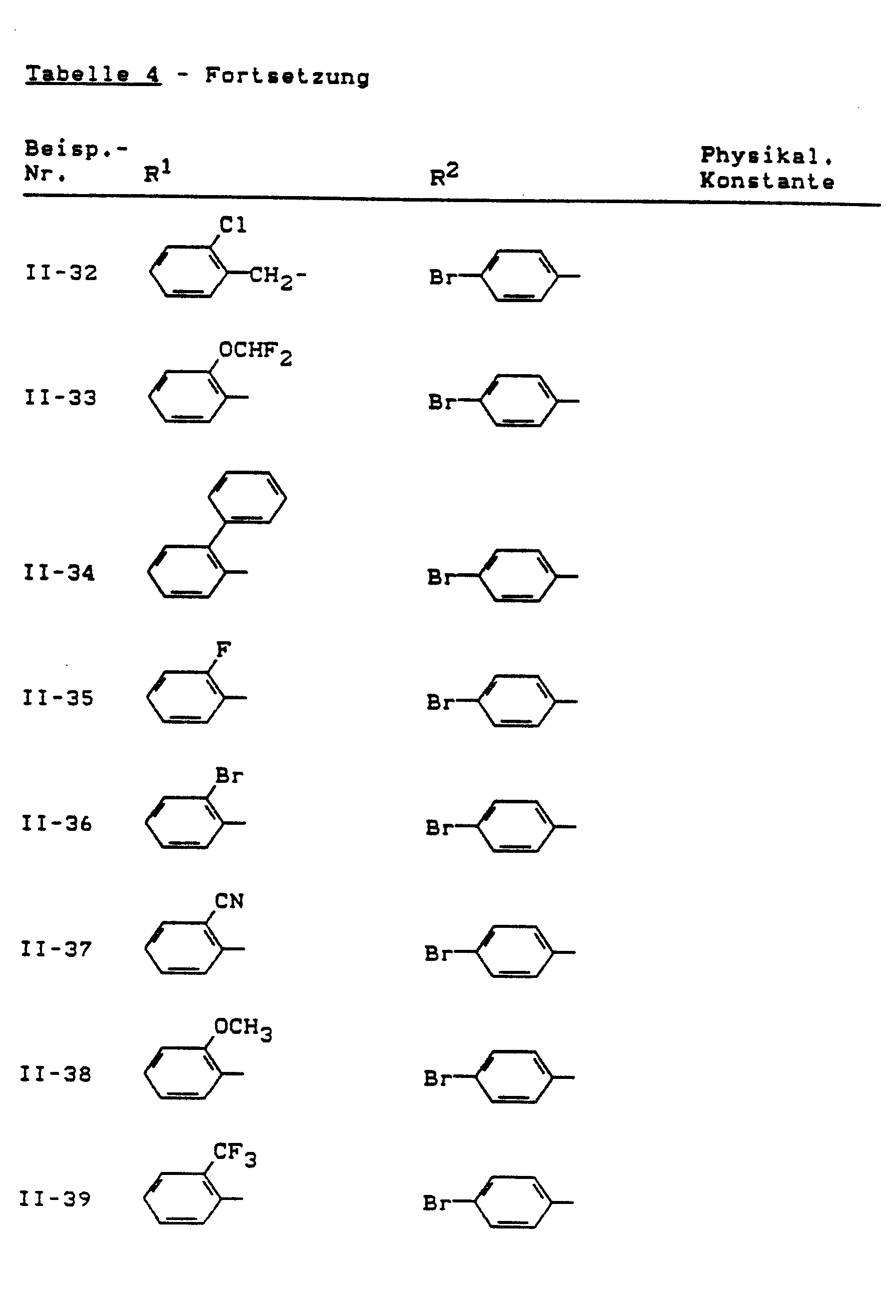

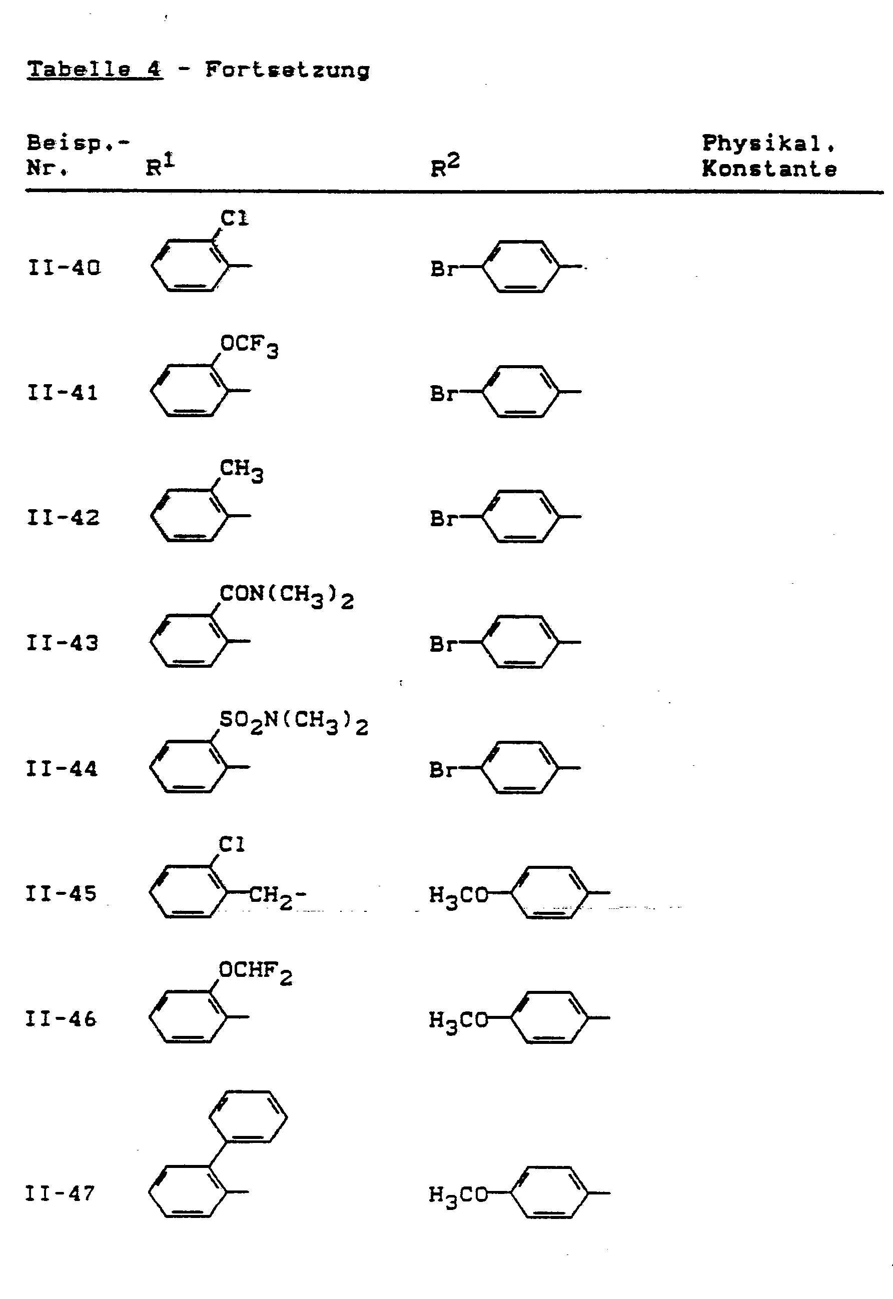

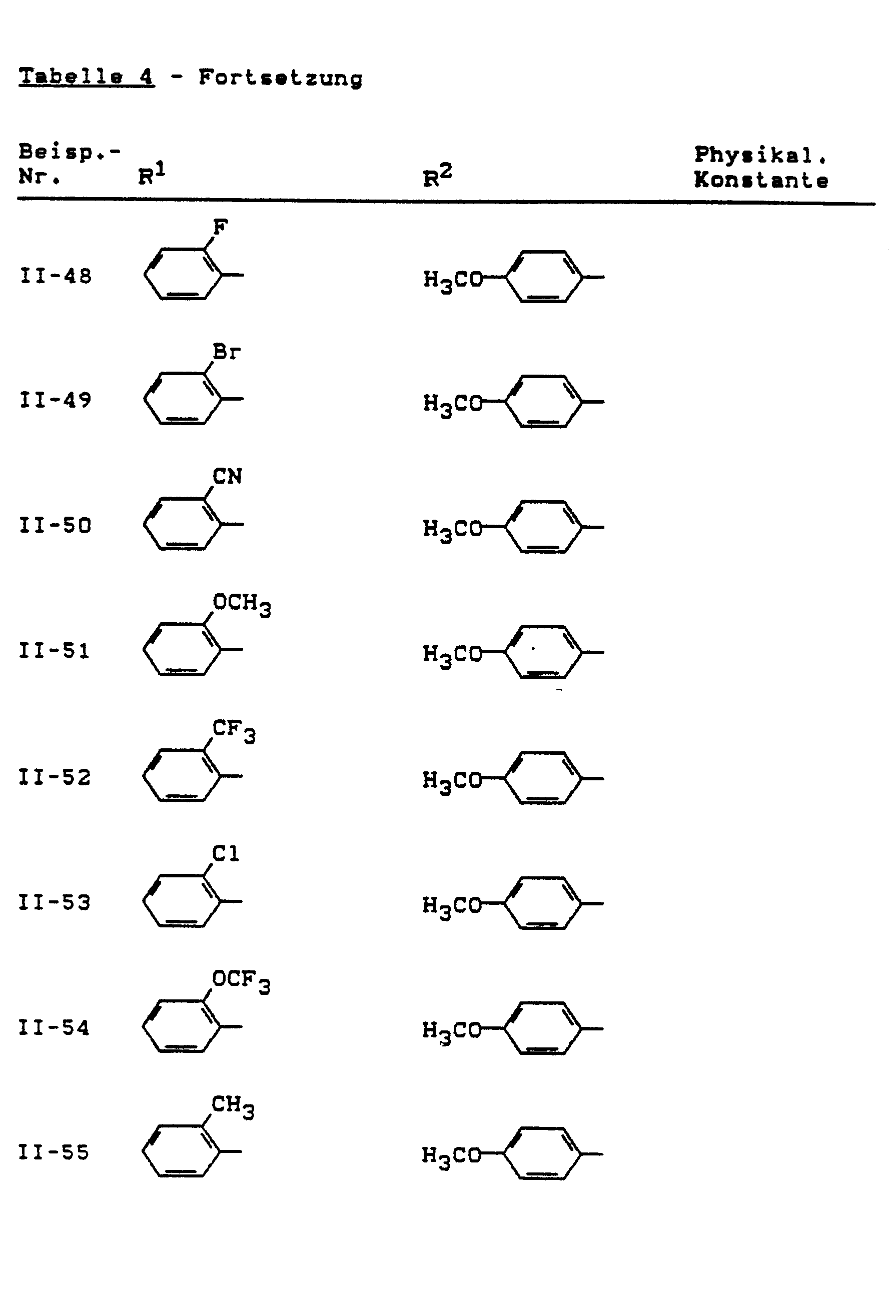

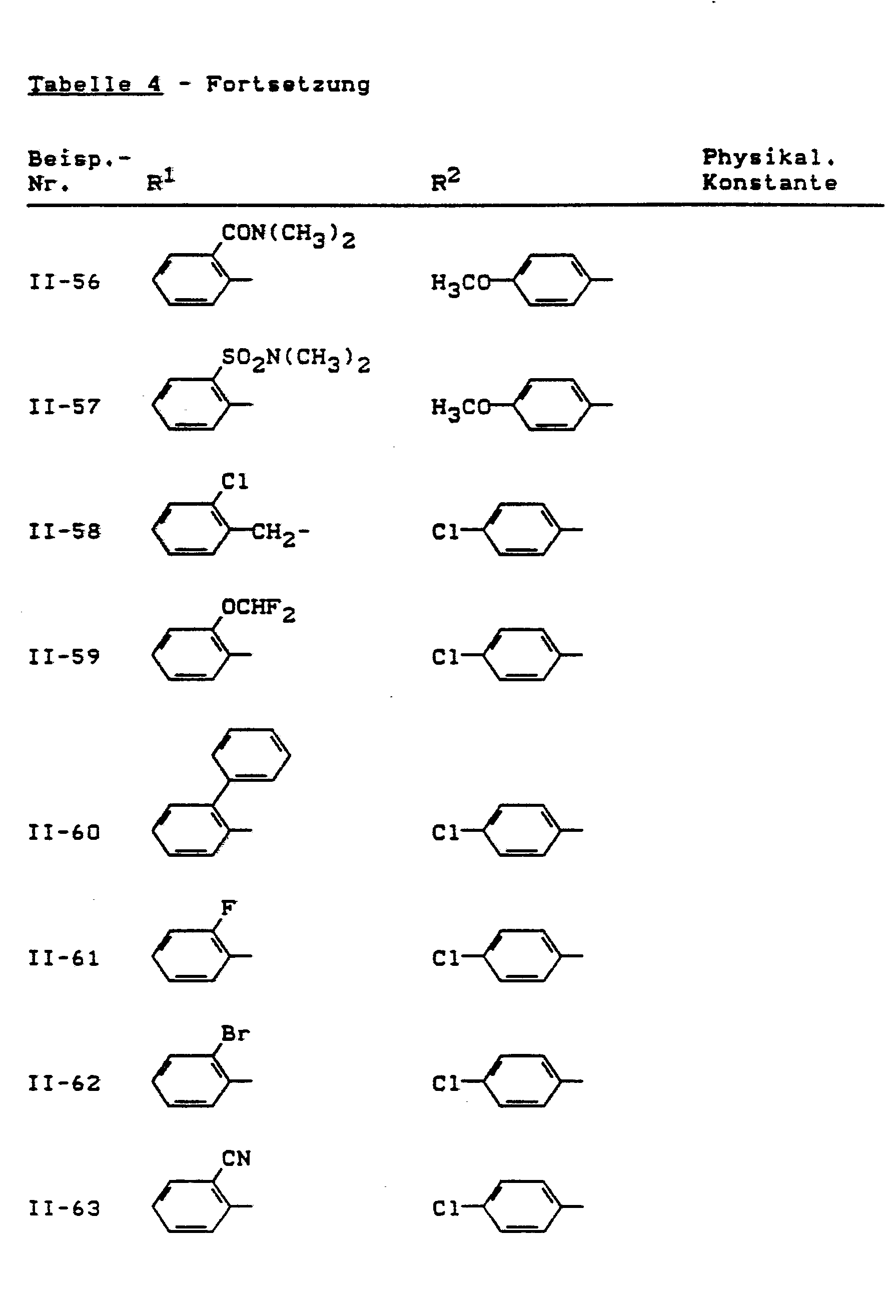

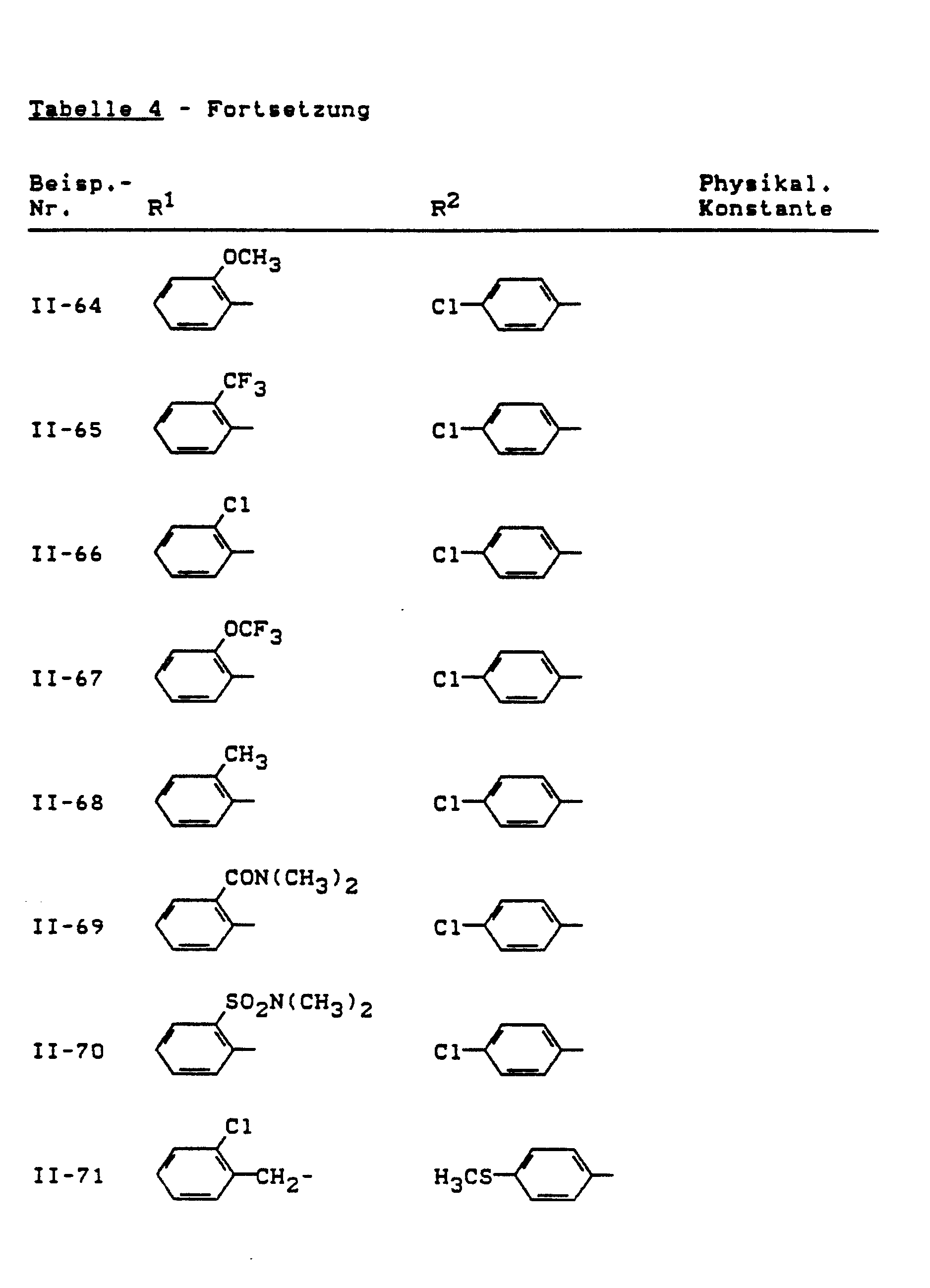

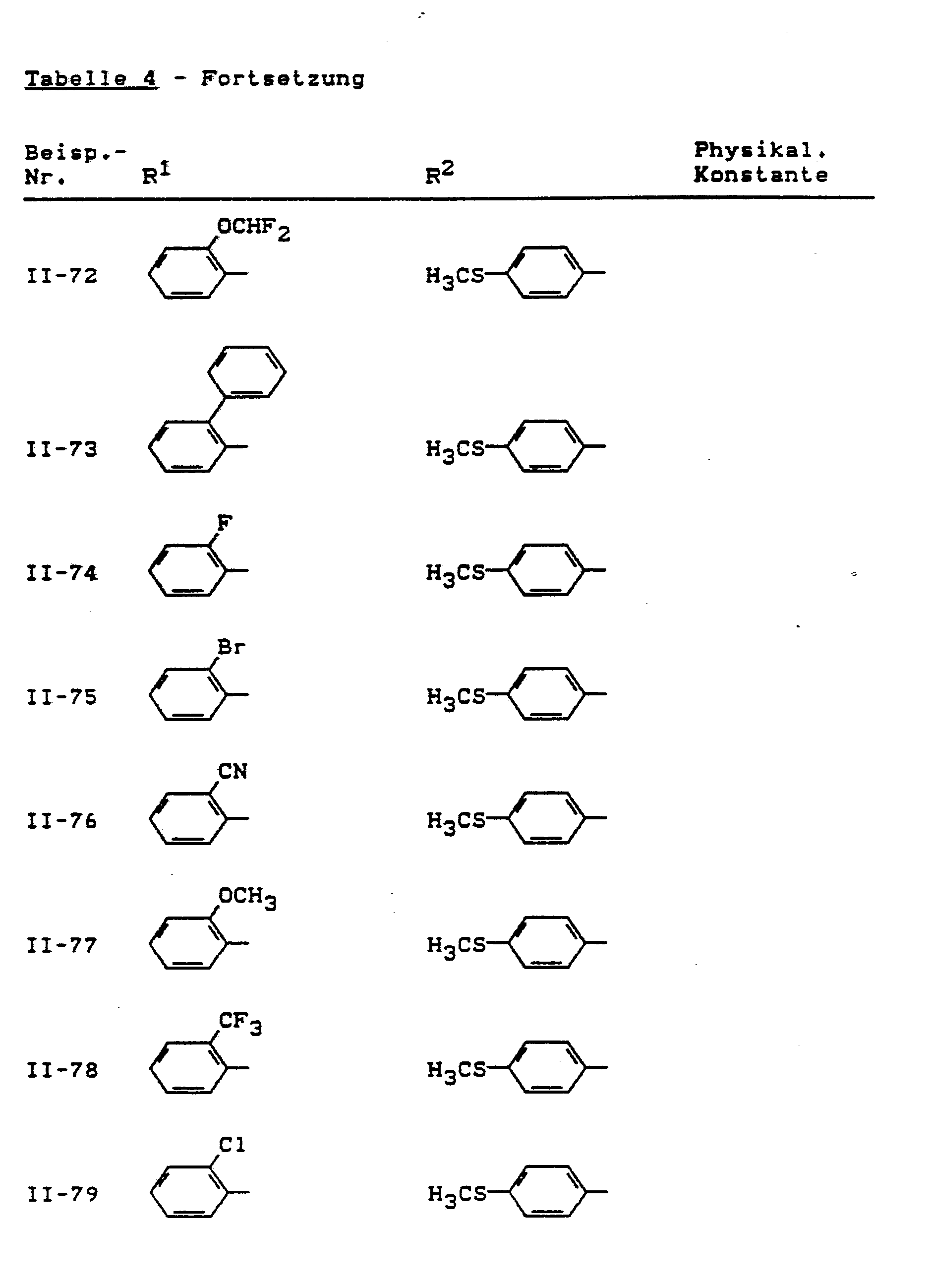

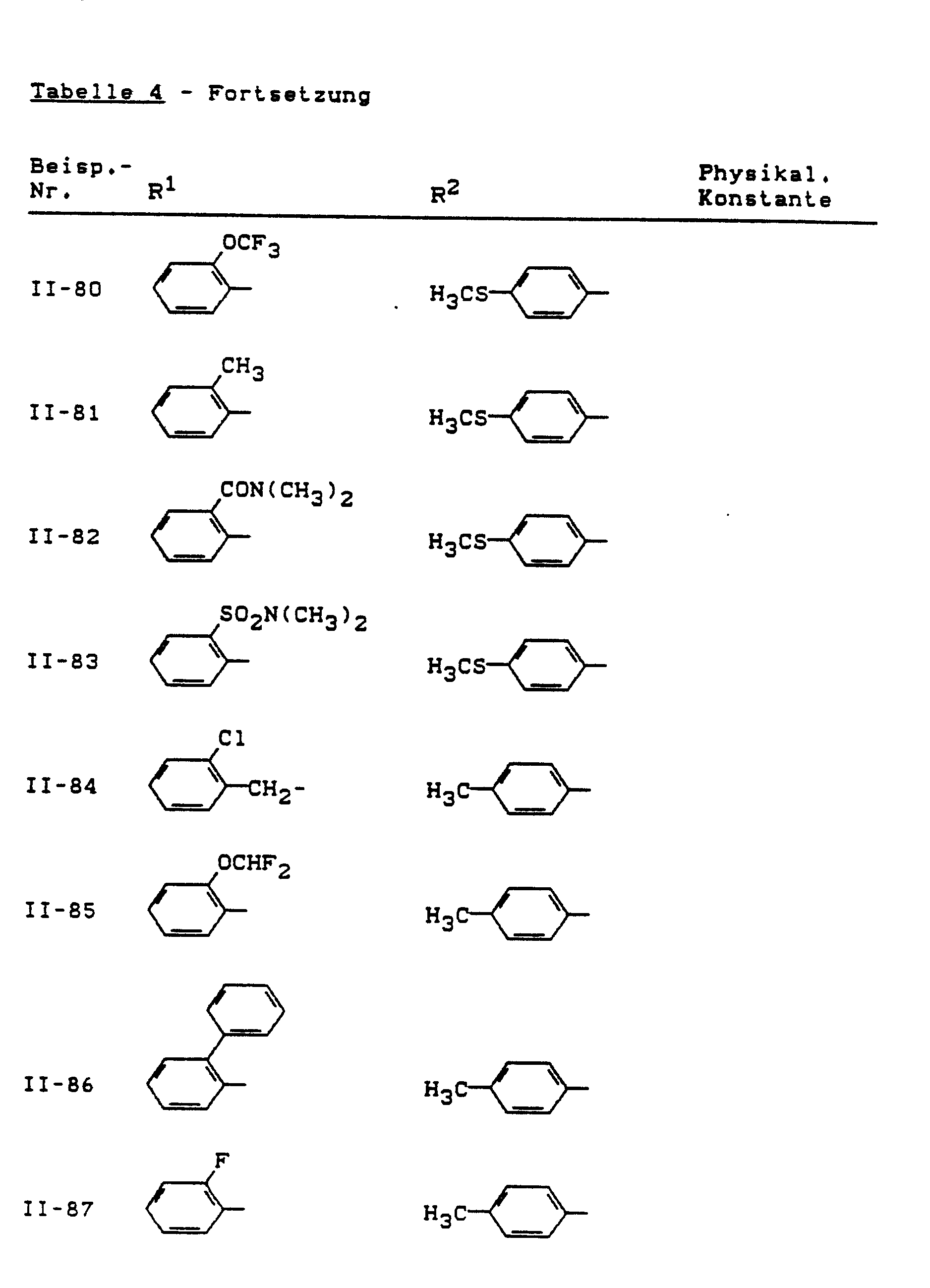

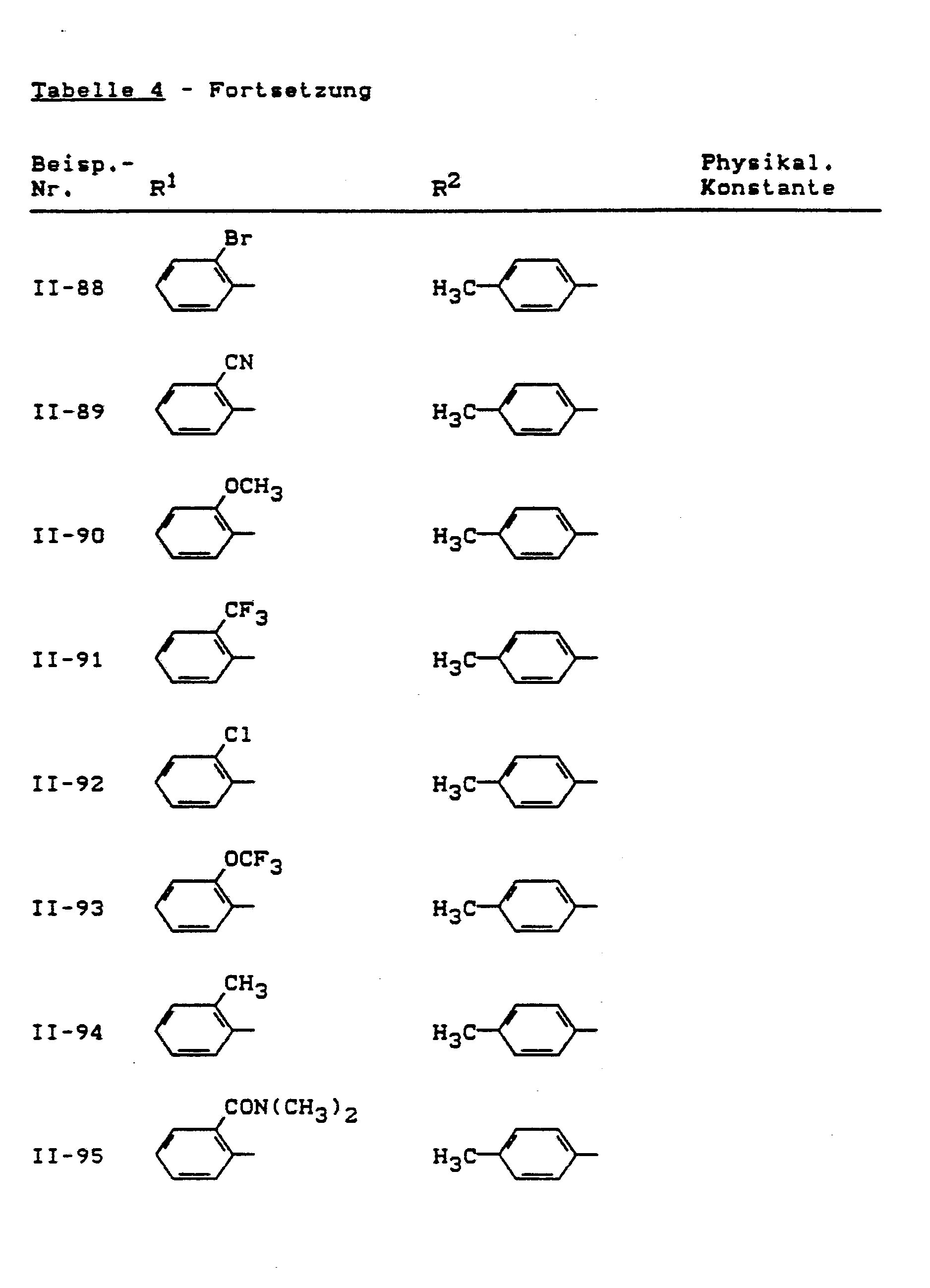

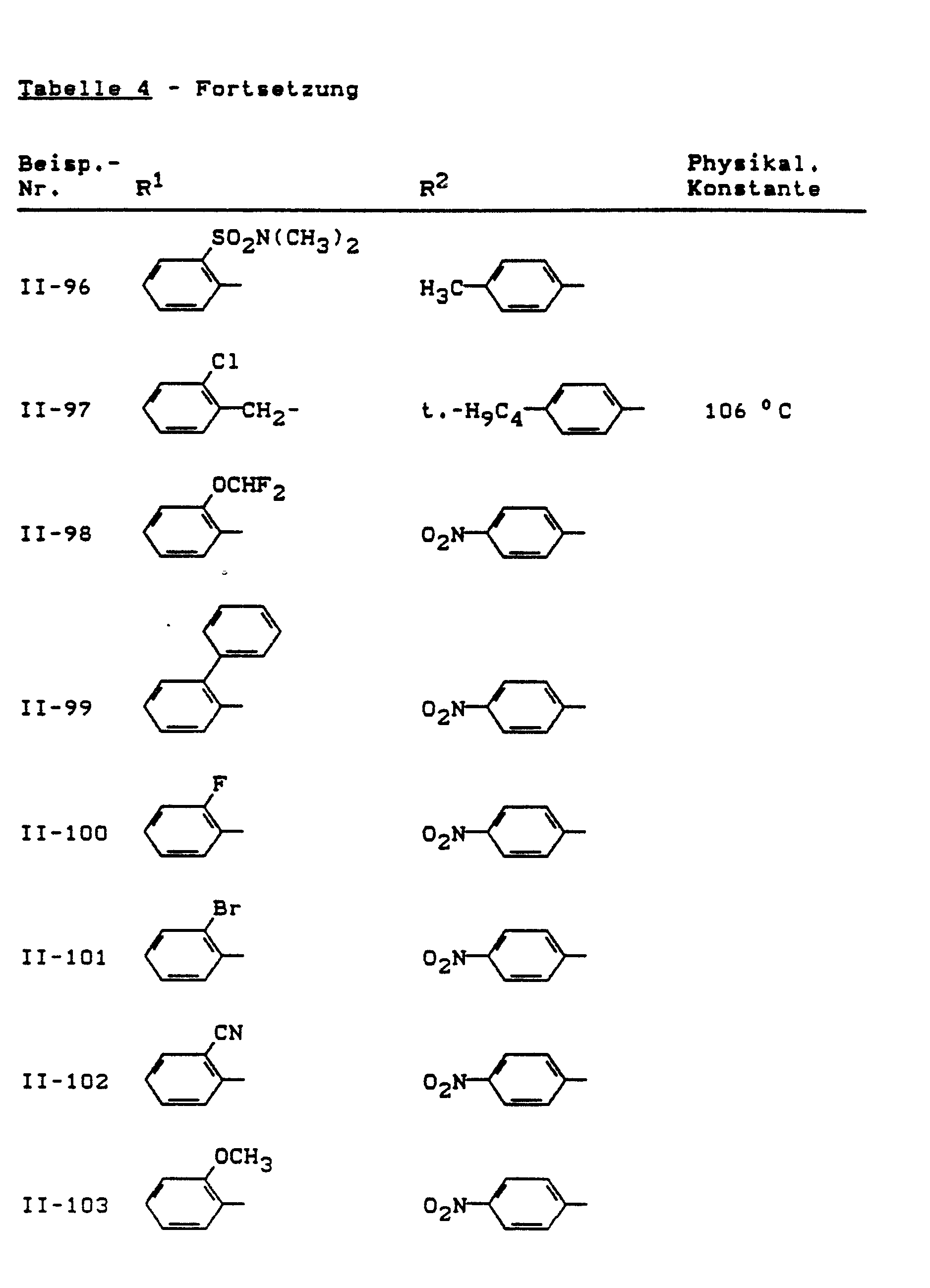

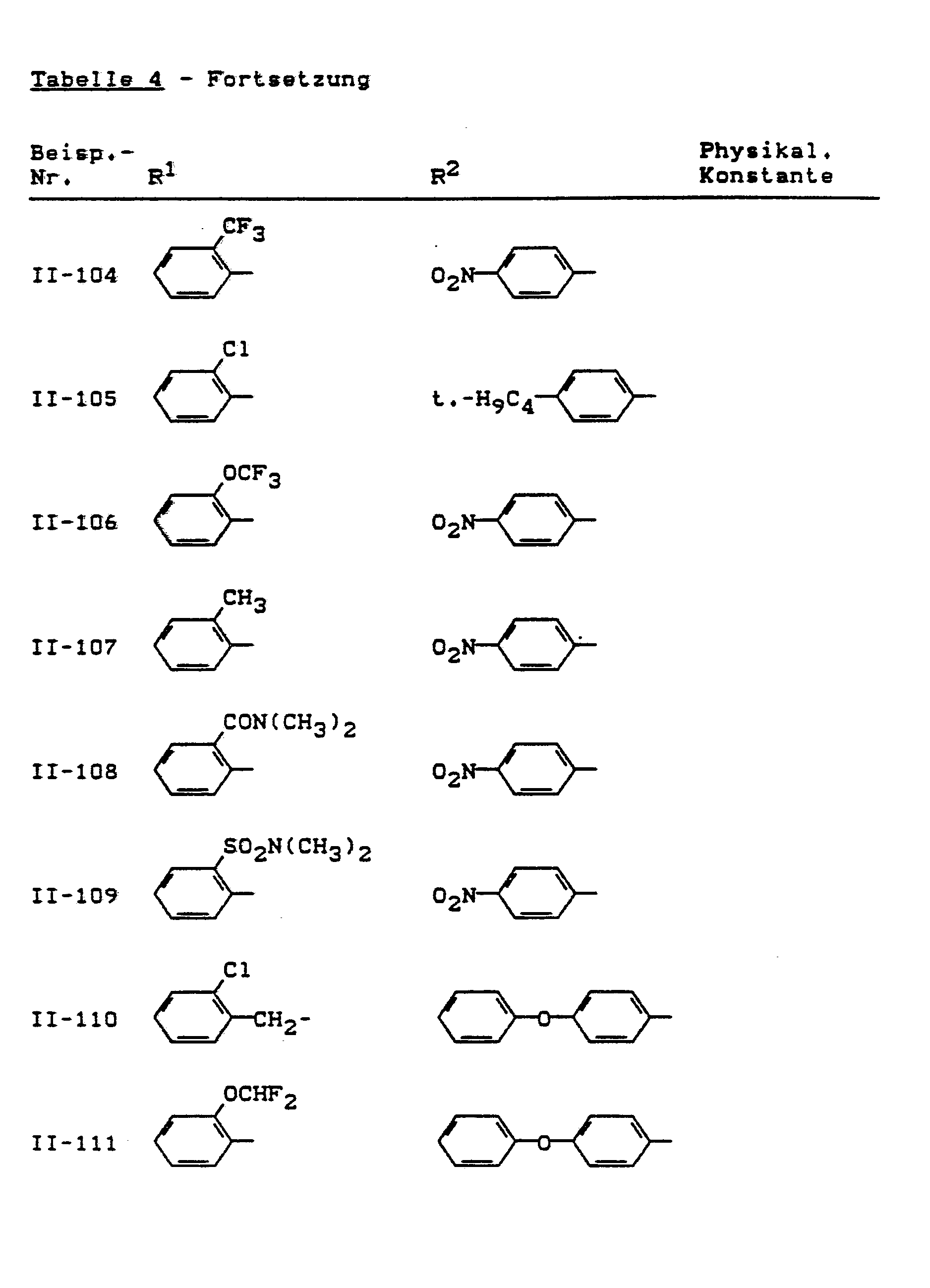

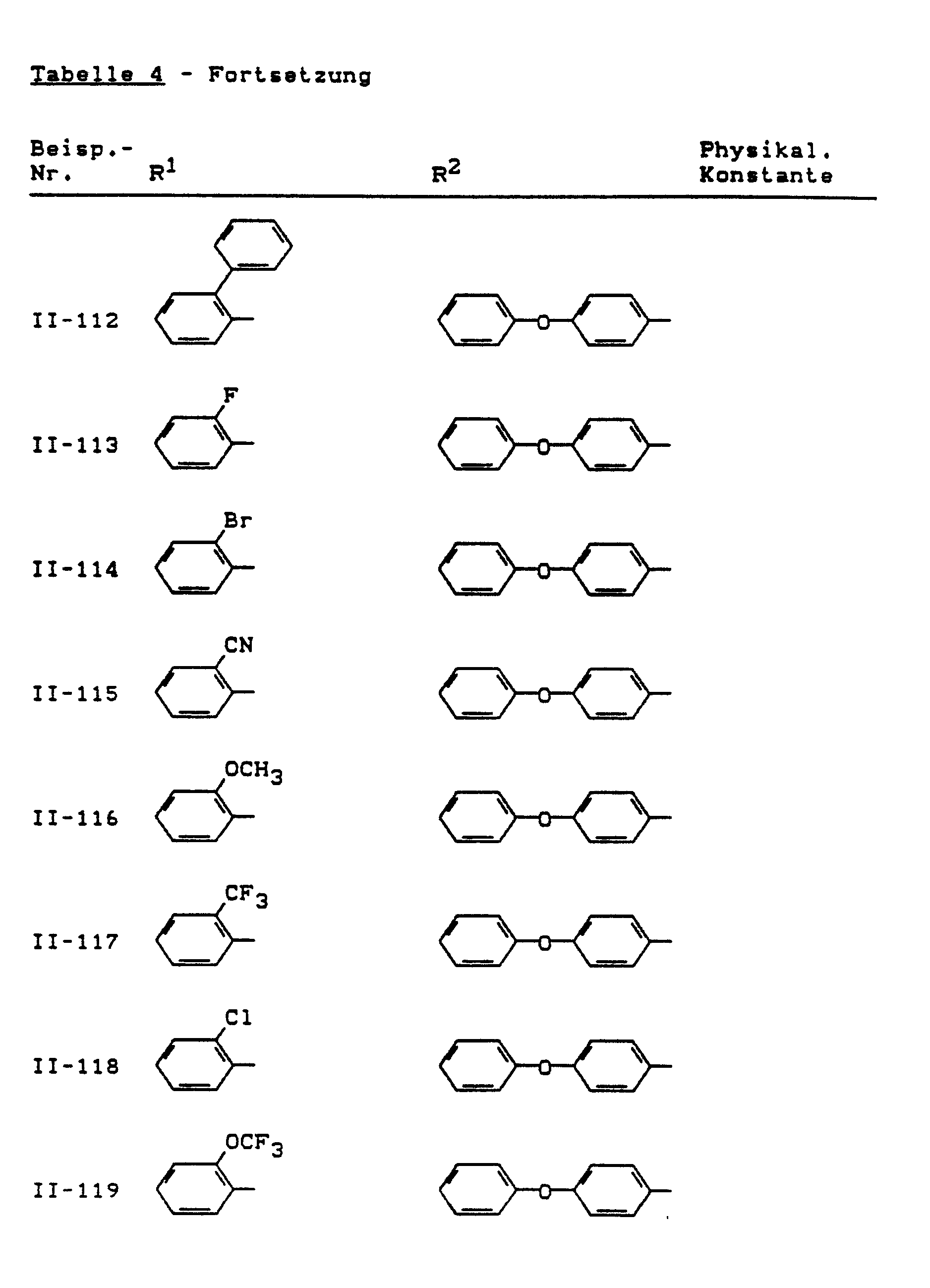

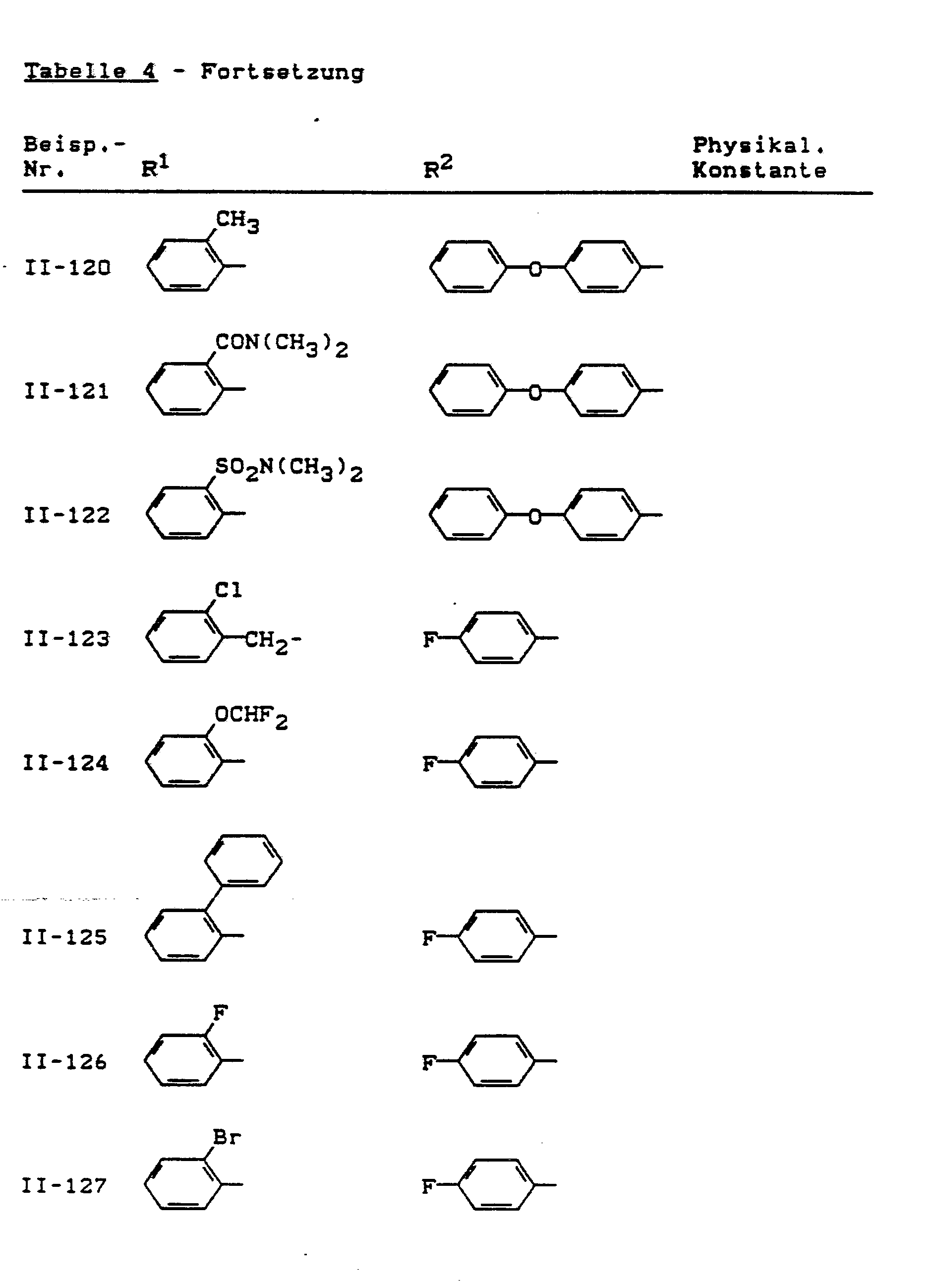

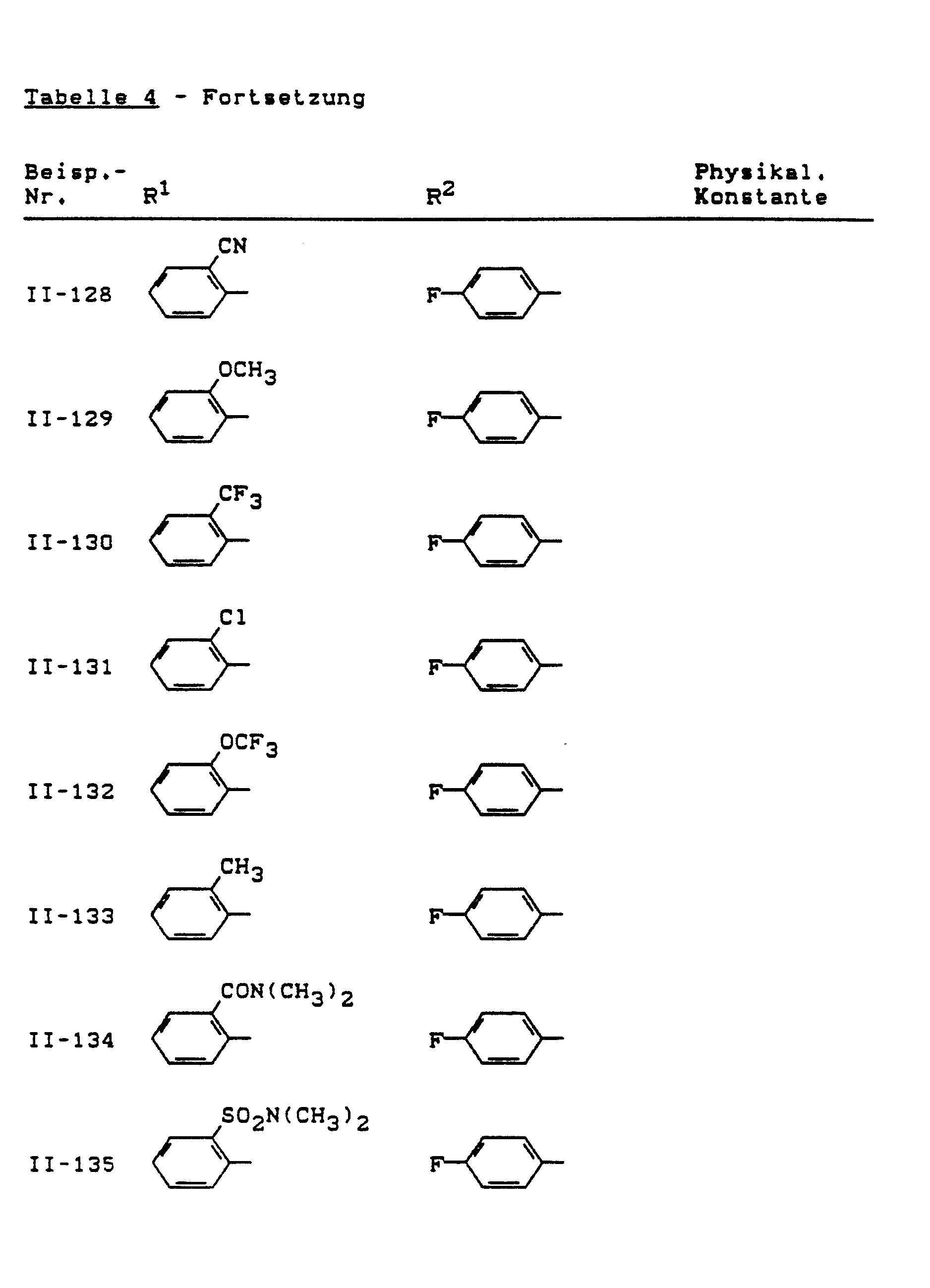

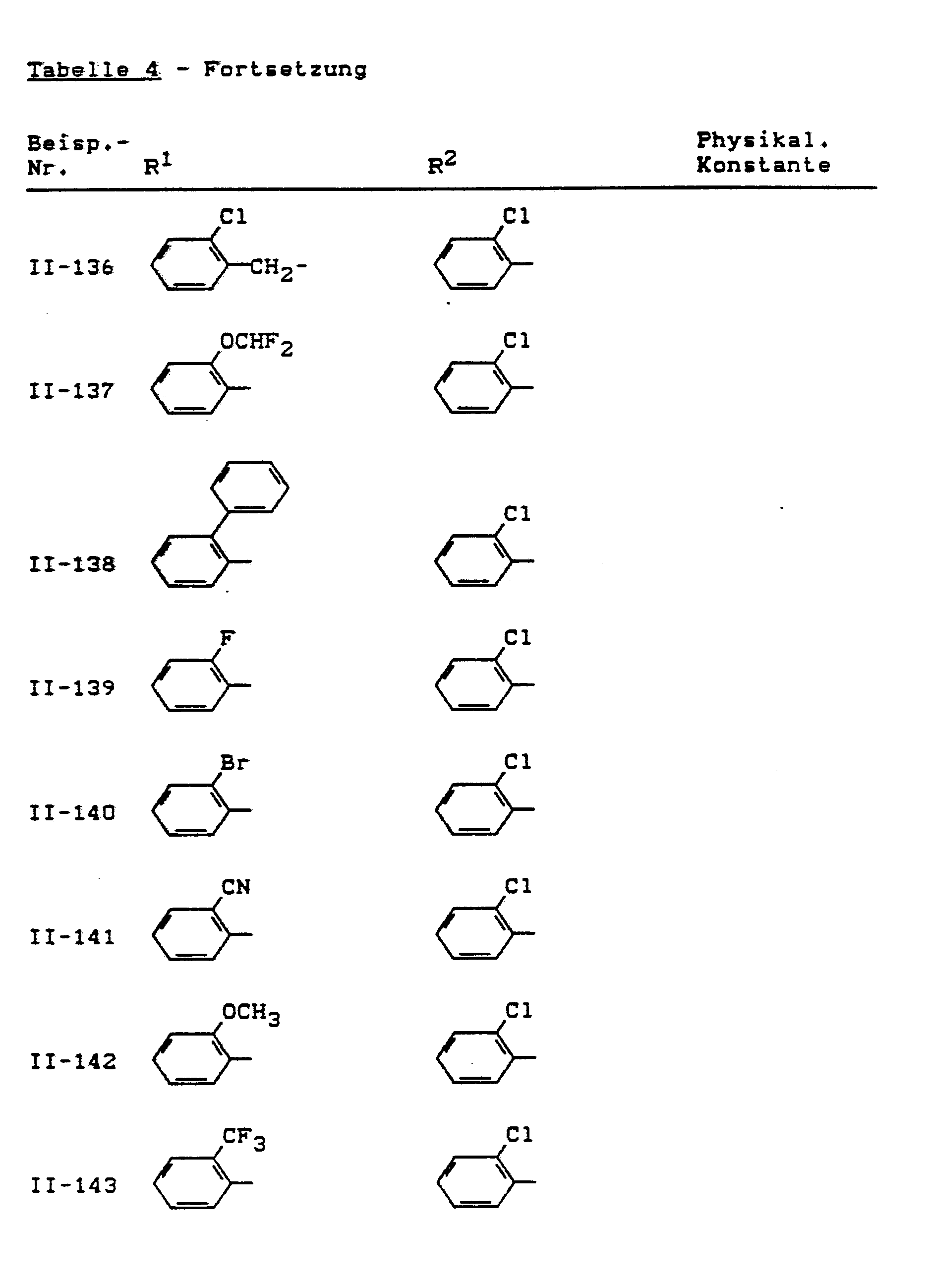

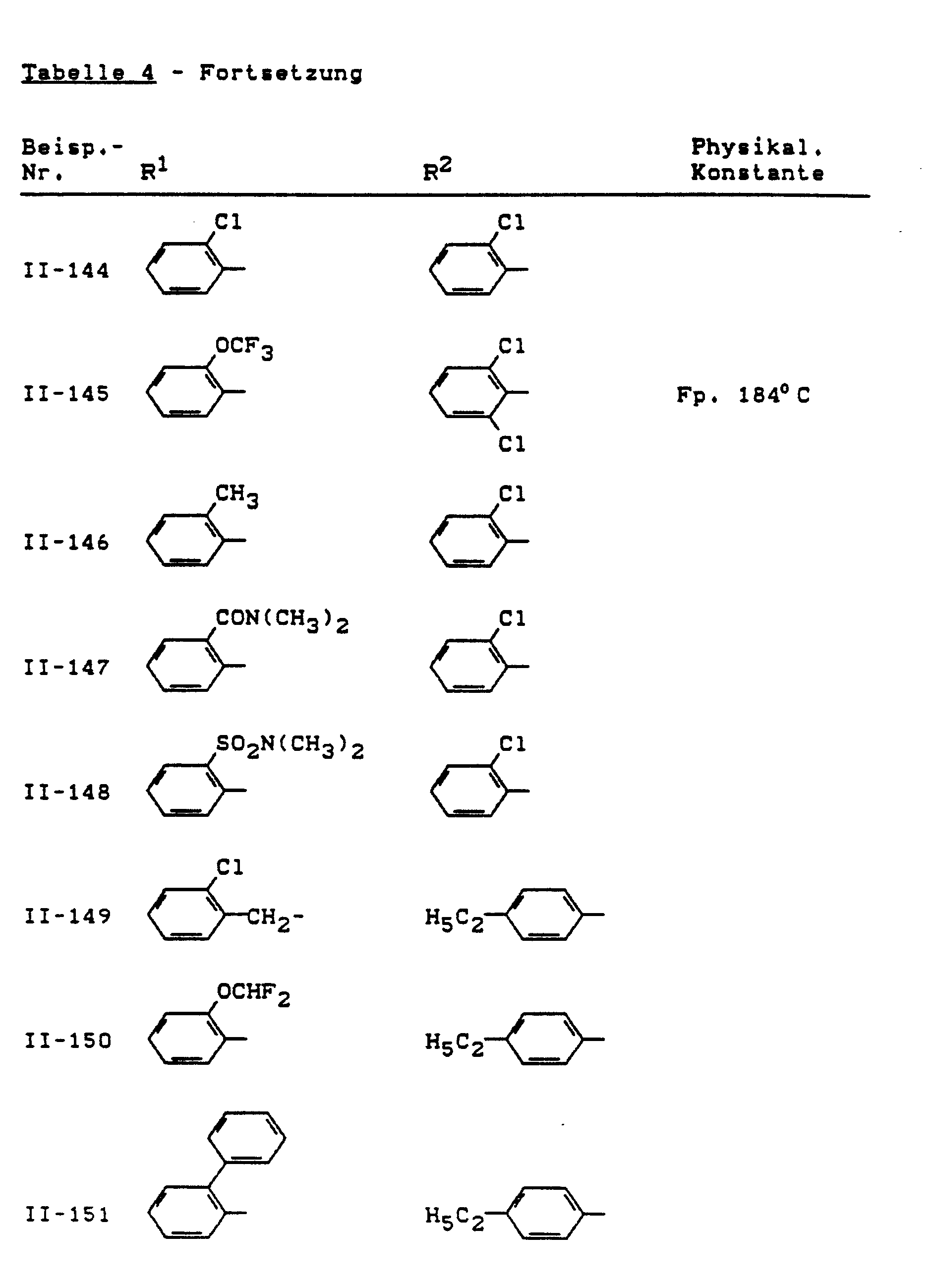

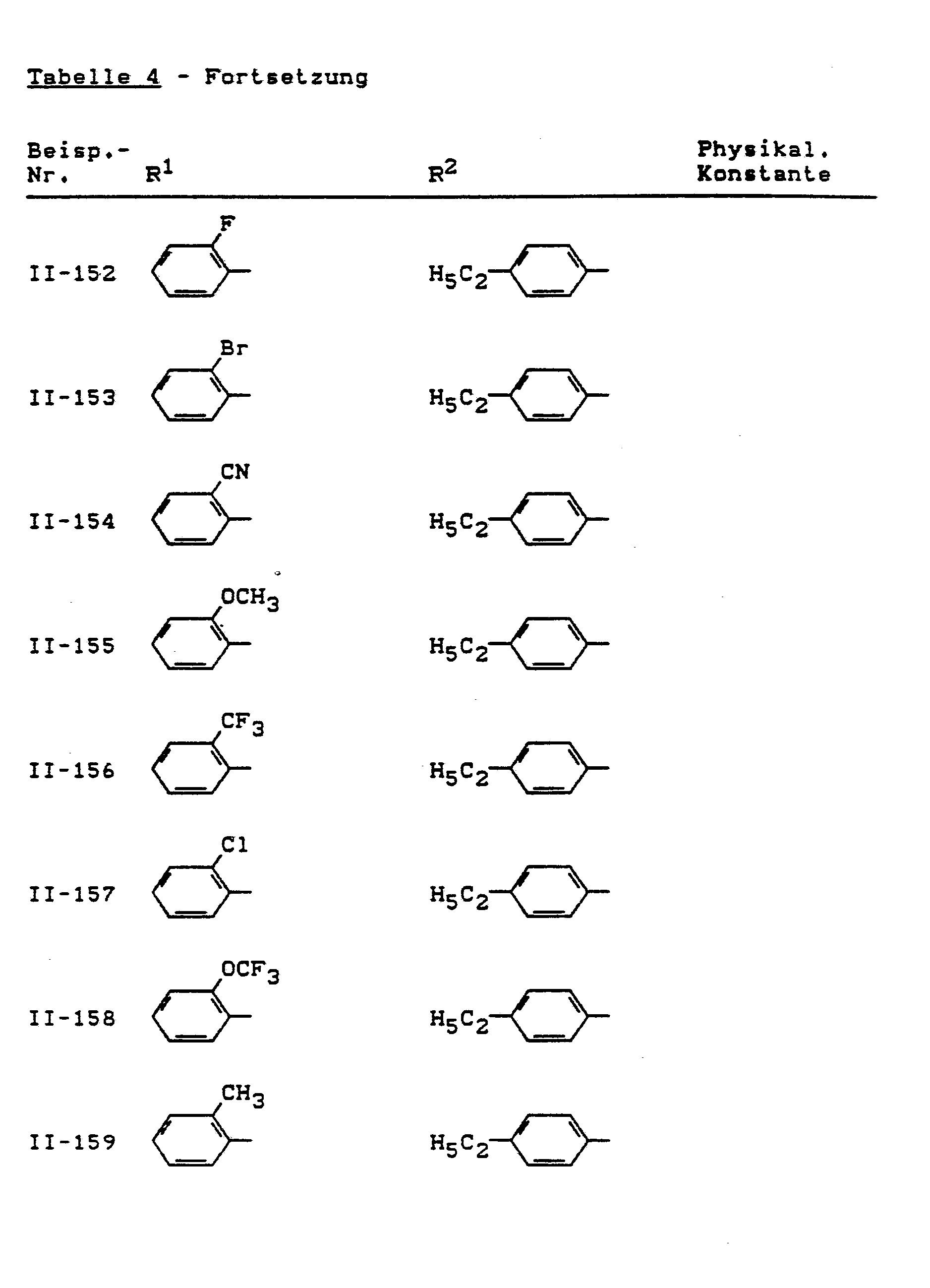

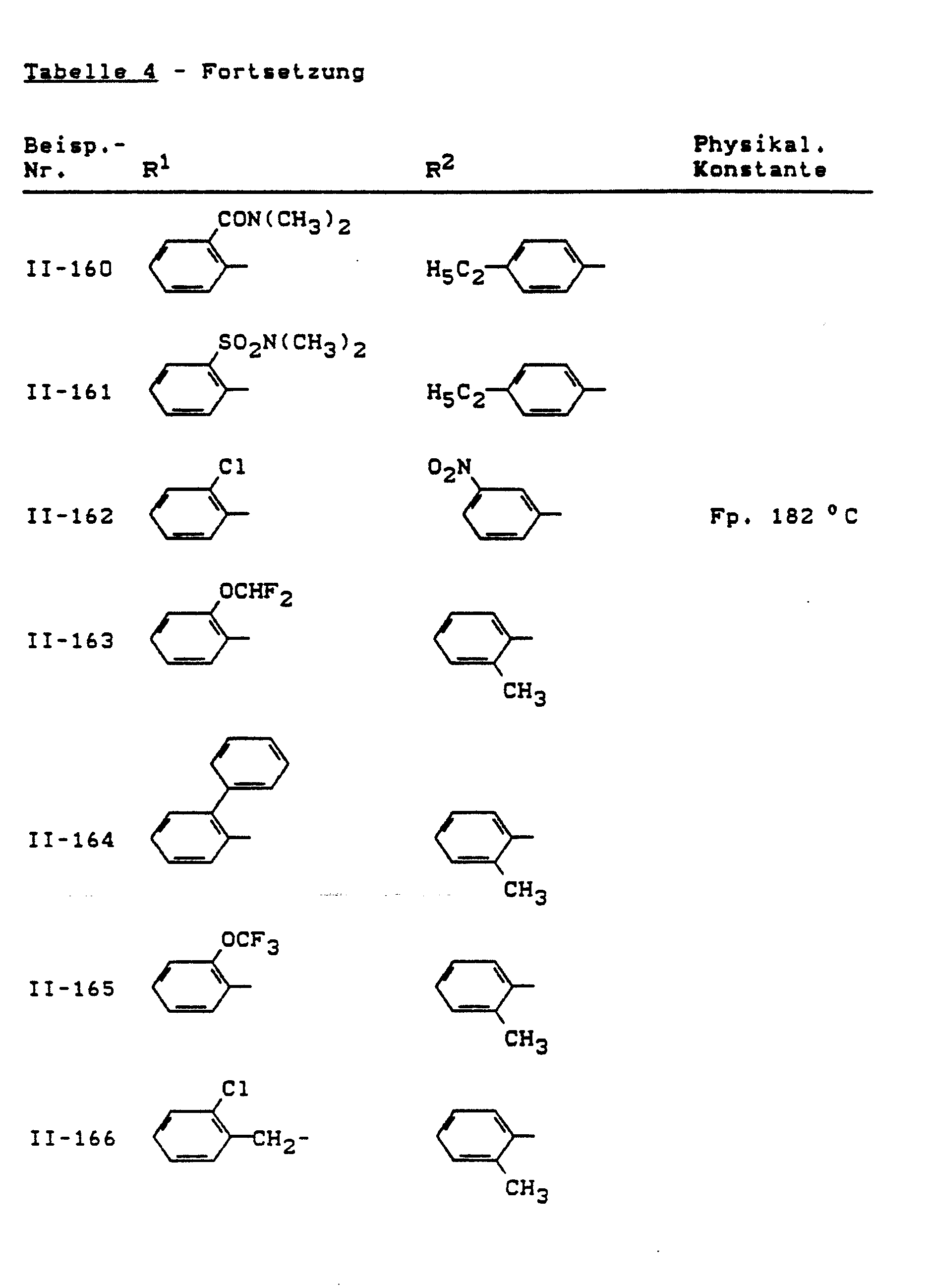

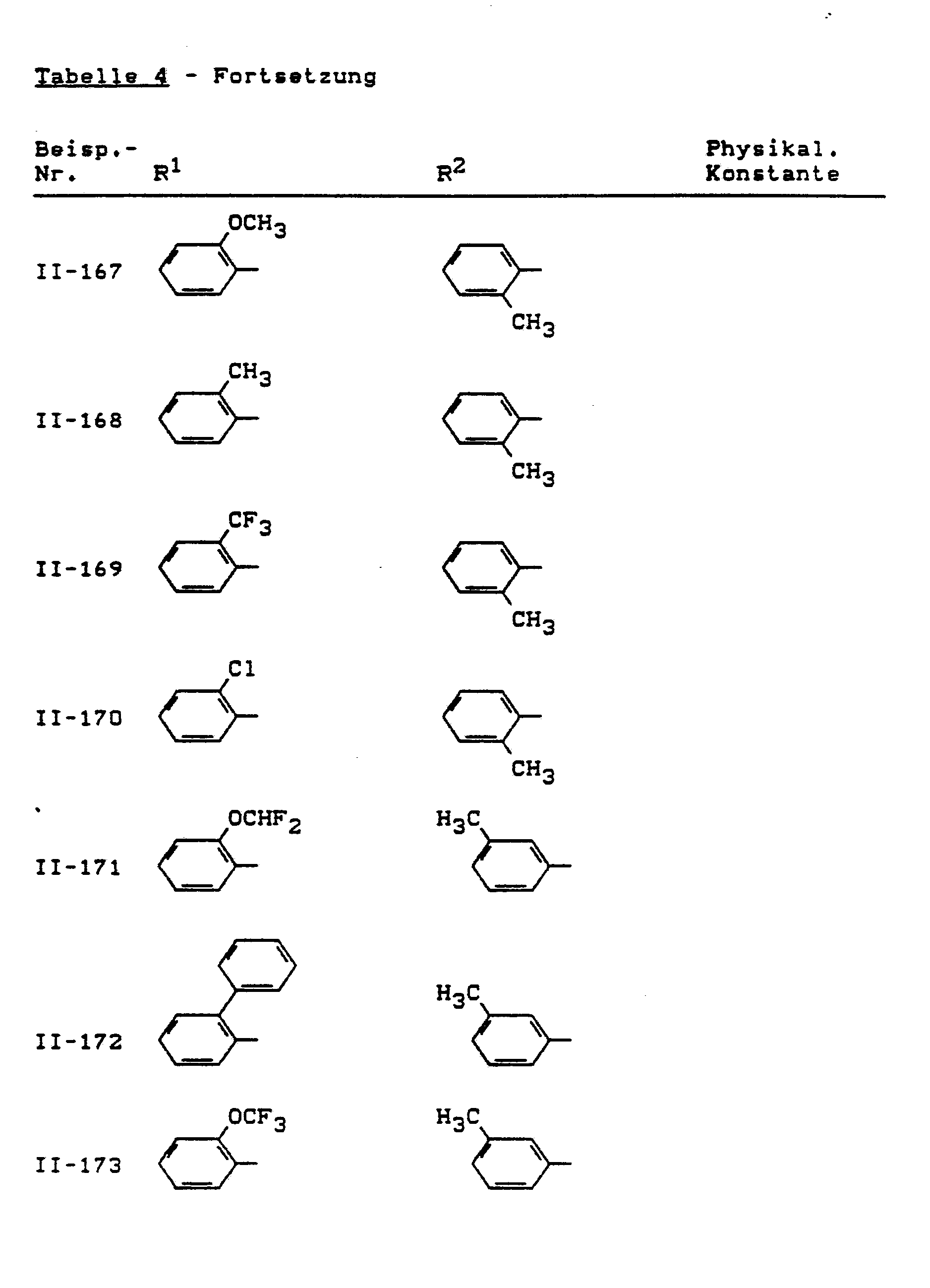

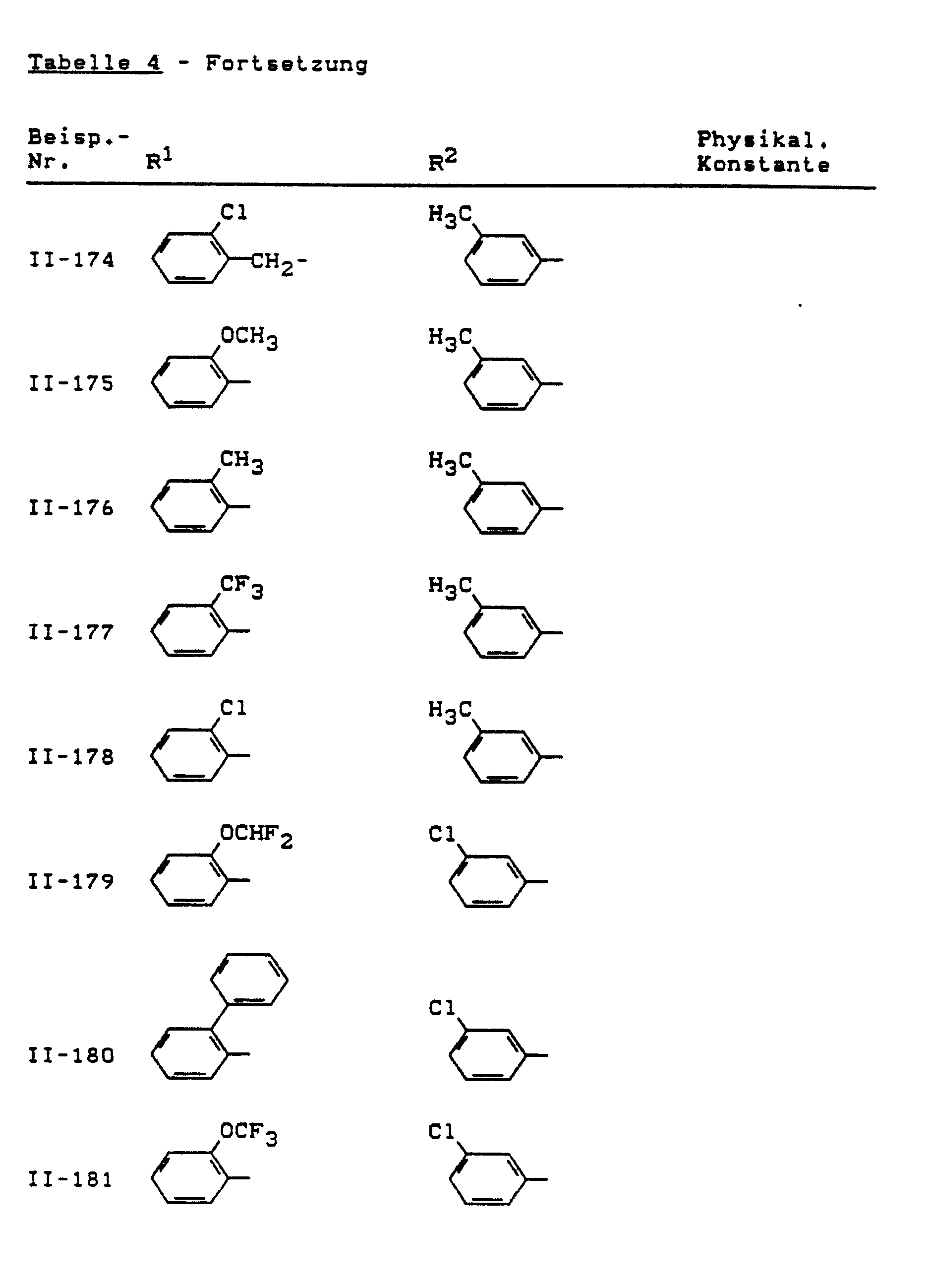

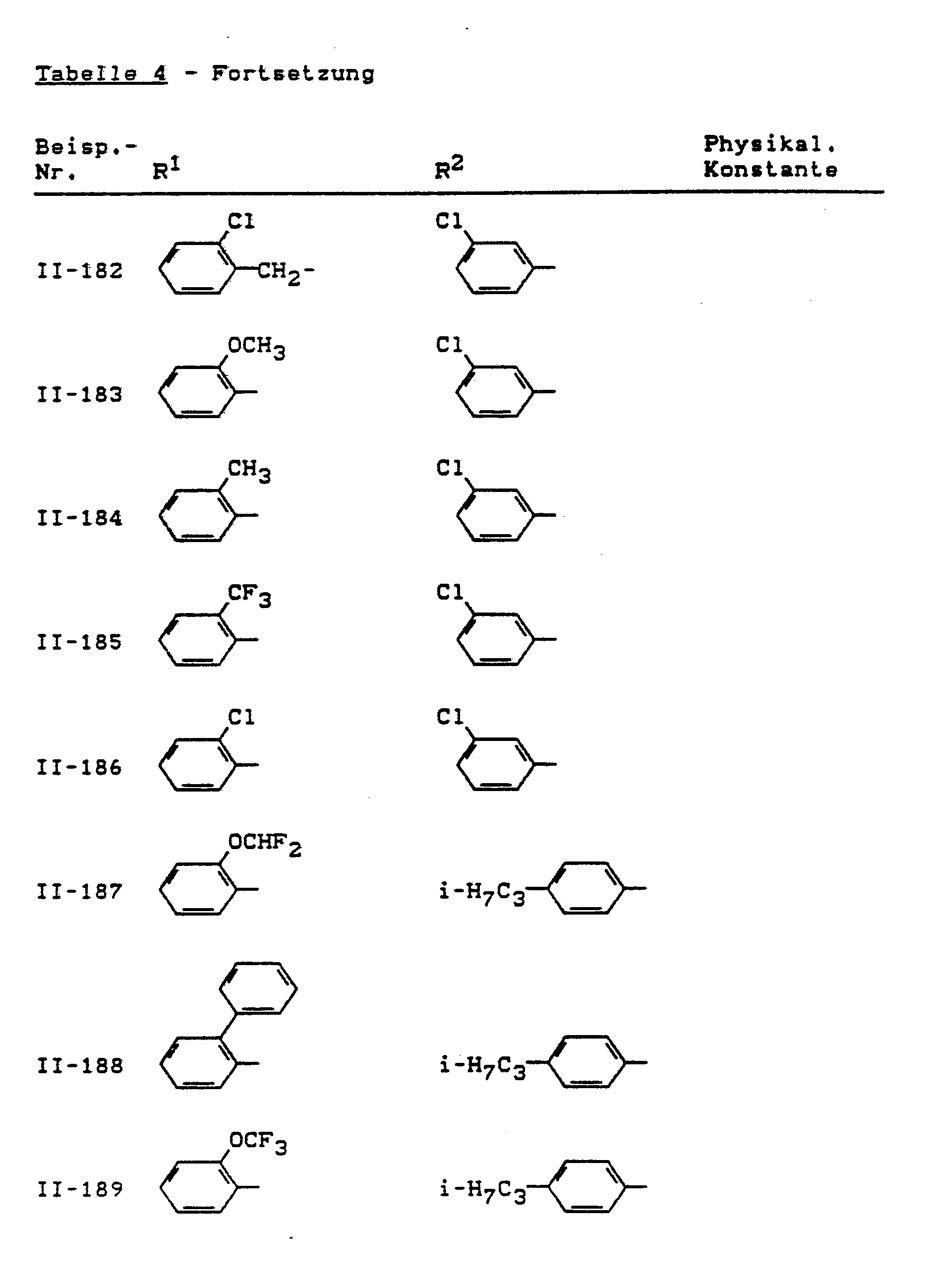

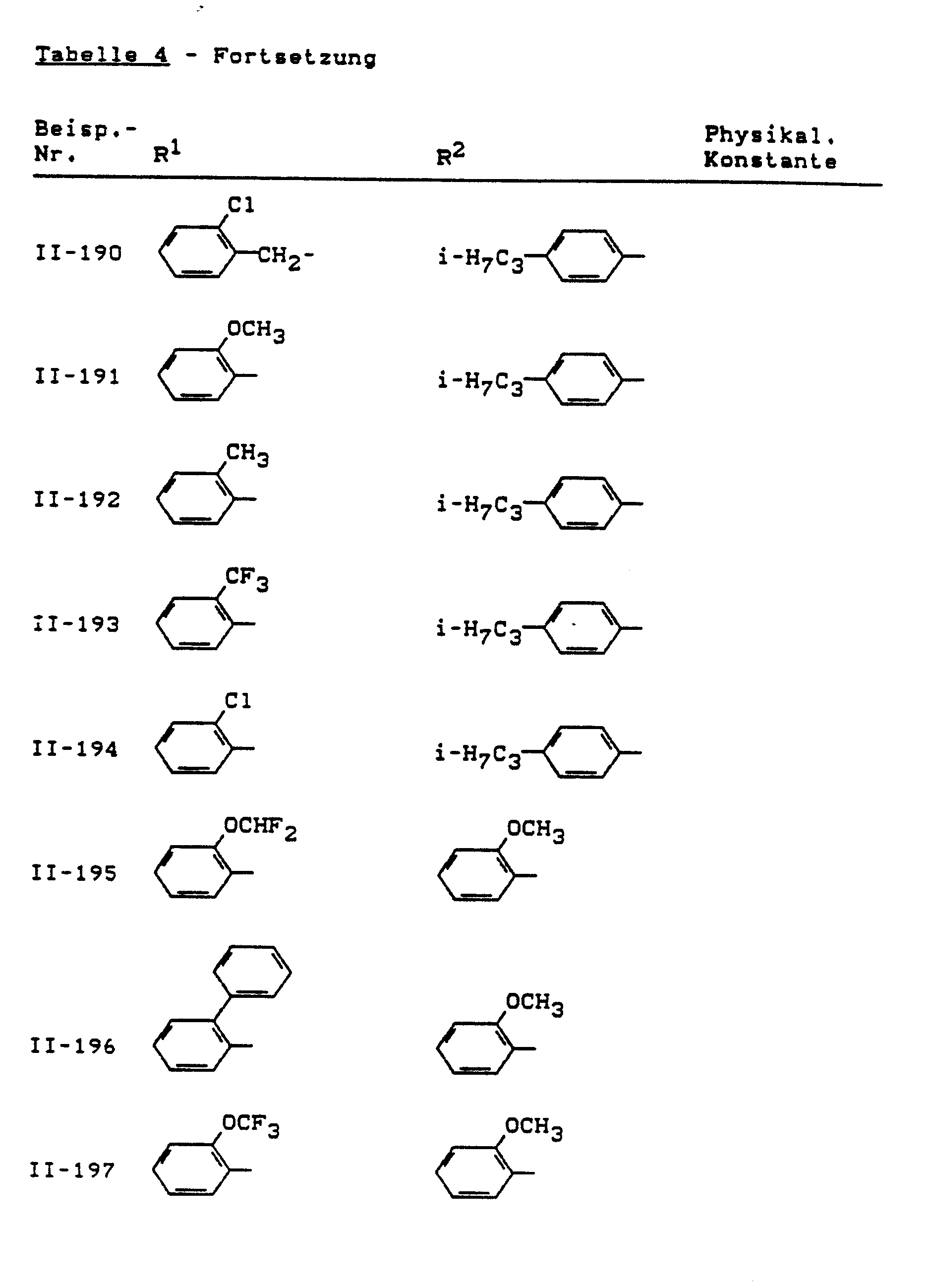

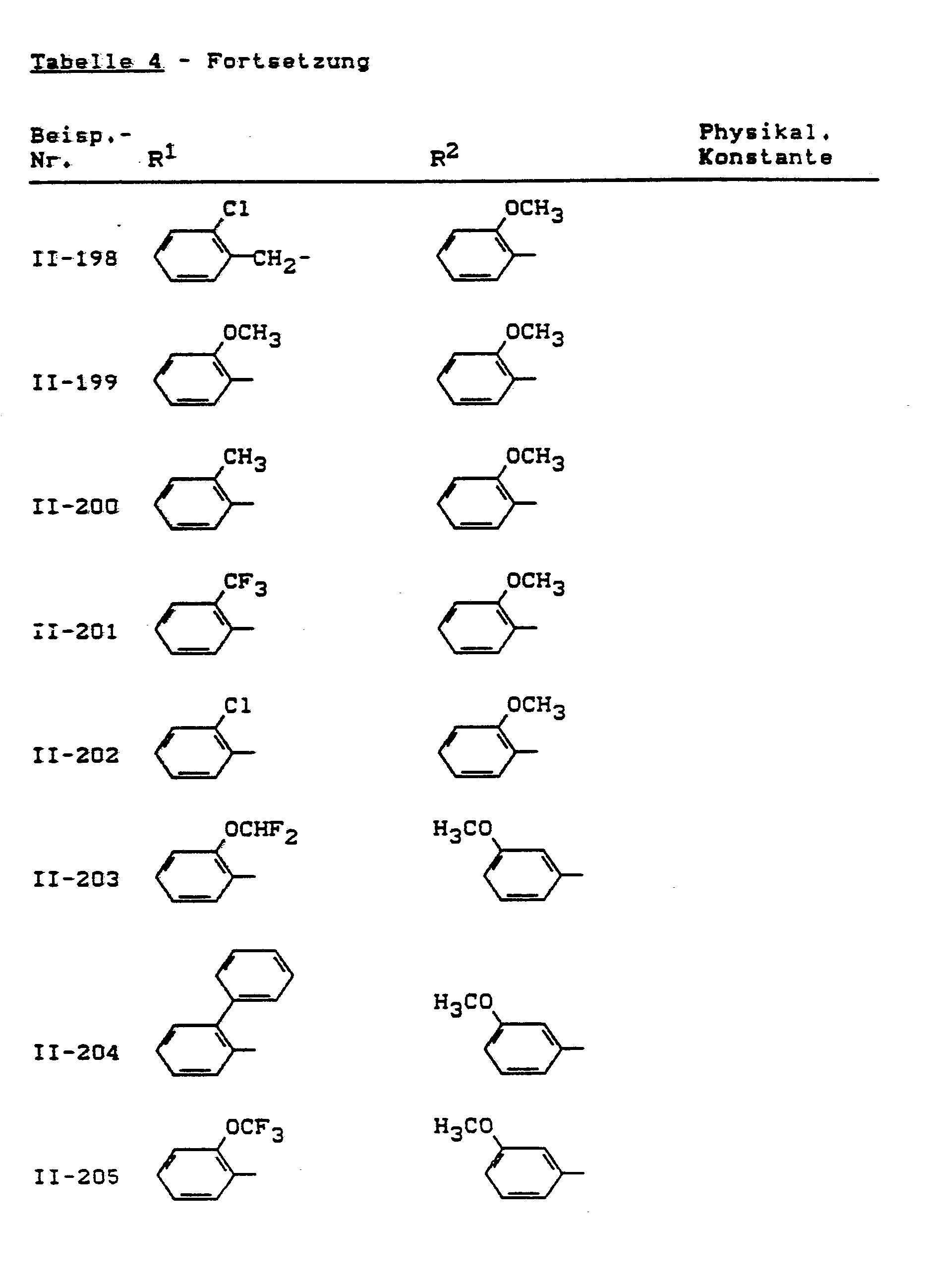

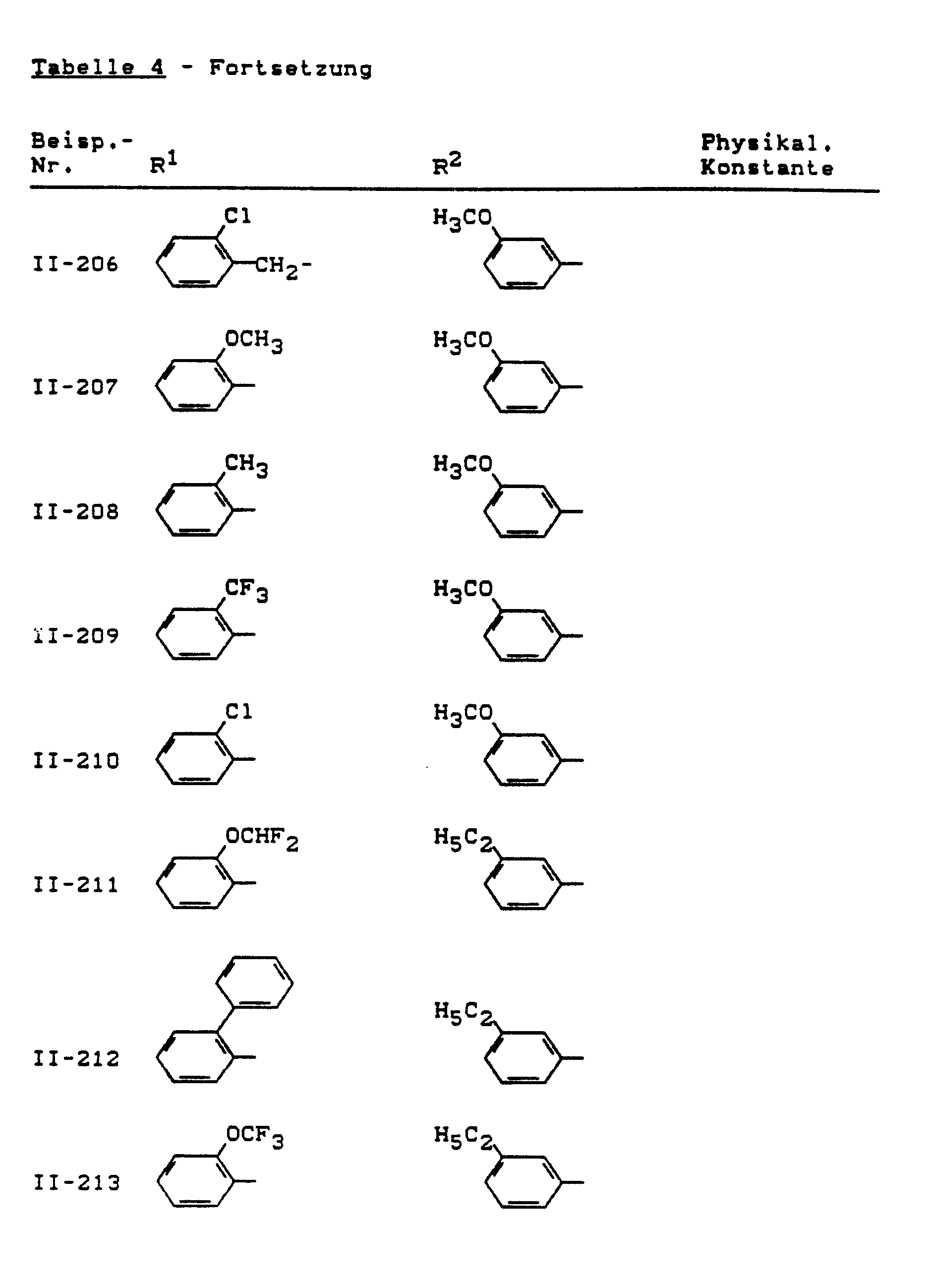

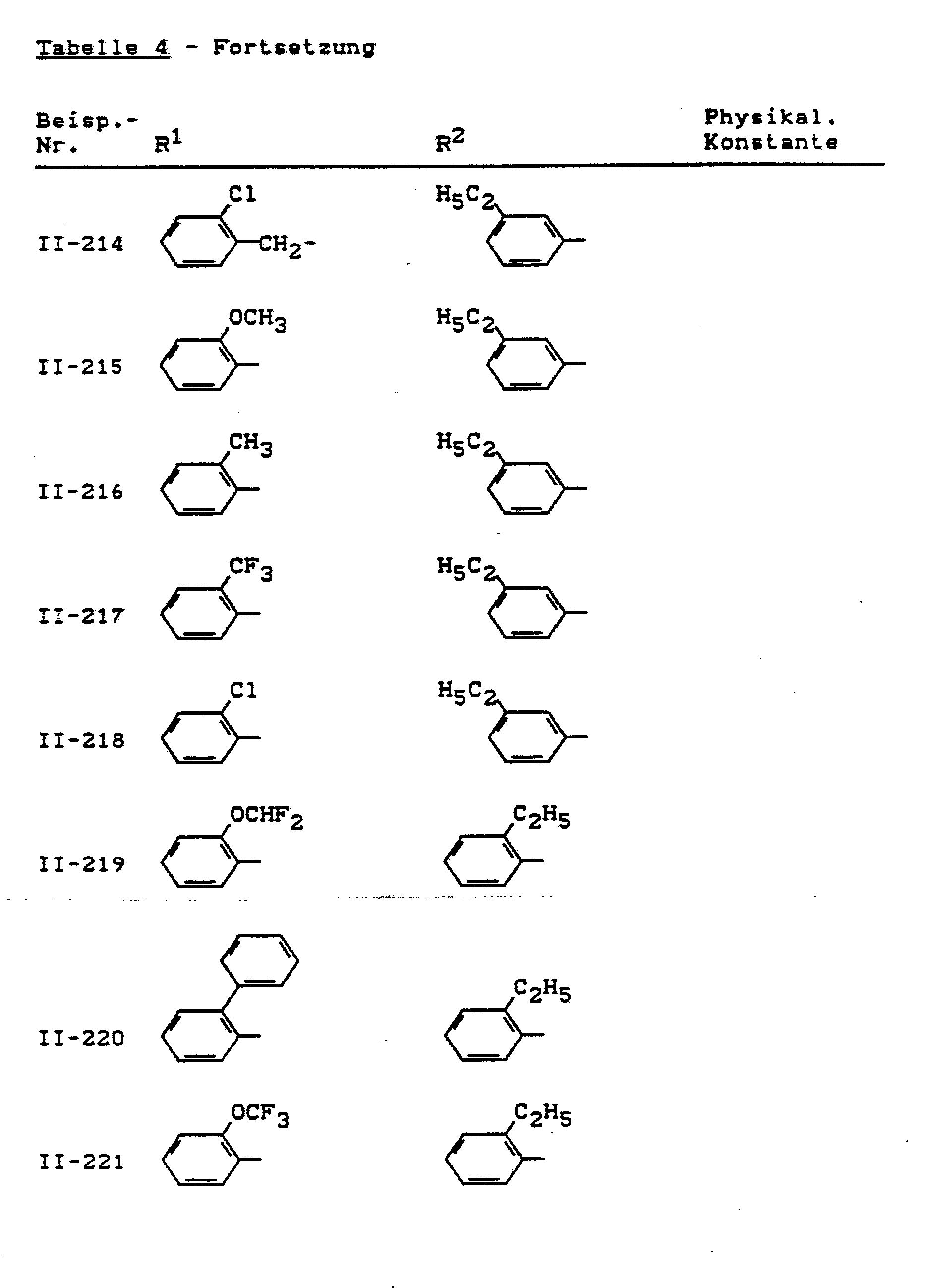

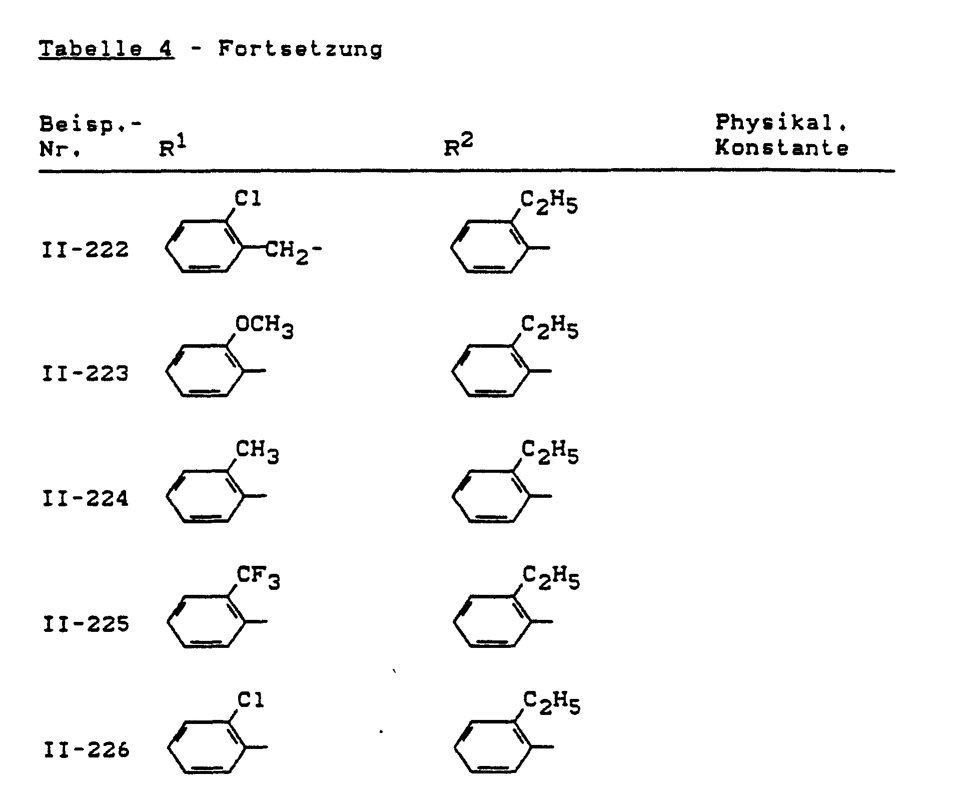

### Ausgangsverbindungen der Formel (IV) und (IVa)

### Beispiel (IVa-1)

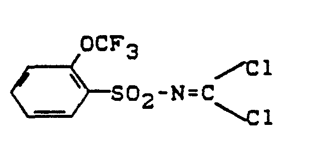

34,5 g (0,1 Mol) 2-Trifluormethoxy-benzosulfonyl-iminothiokohlensäure-S,S-dimethylester werden in 300 ml Tetrachlorkohlenstoff gelöst und bei 20 °C werden 568 g (8 Mol) trockenes Chlorgas eingeleitet. Das Reaktionsgemisch erwärmt sich dabei auf etwa 40 °C. Es wird 1 Stunde nachgerührt, abfiltriert, das Filtrat im Vakuum eingeengt und der Rückstand im Vakuum fraktioniert destilliert.

Man erhält so 23,2 g (72,4 % der Theorie) 2-Trifluormethoxy-benzolsulfonyl-isocyaniddichlorid vom Siedepunkt Kp: 126 °C/1 mbar.

Analog Beispiel (lVa-1) können die folgenden Verbindungen der Formeln (IVa) bzw. (IV) erhalten werden:

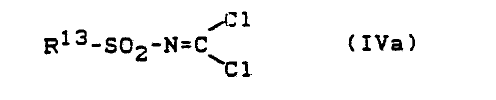

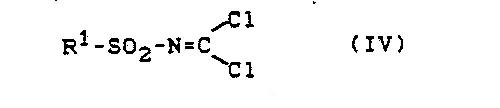

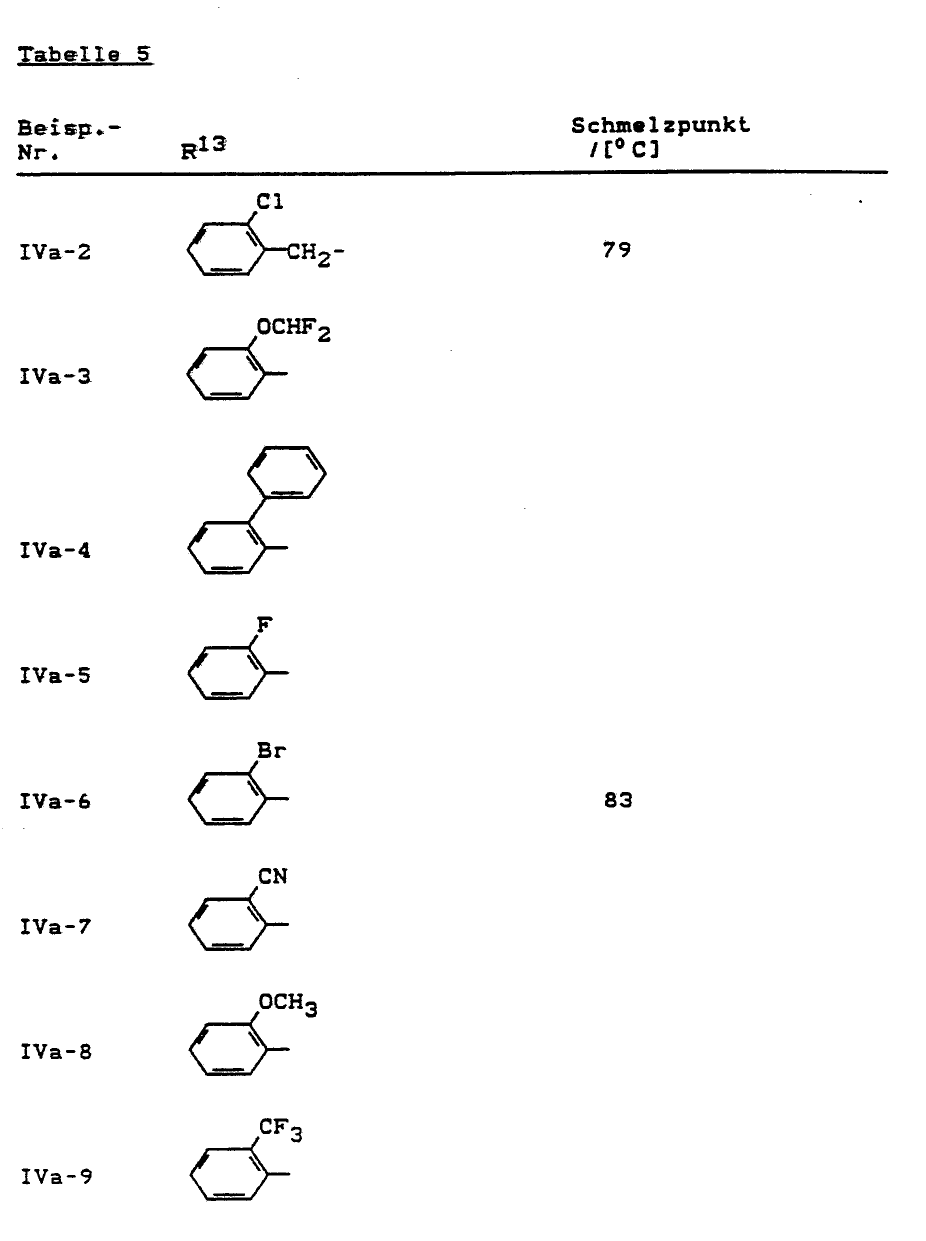

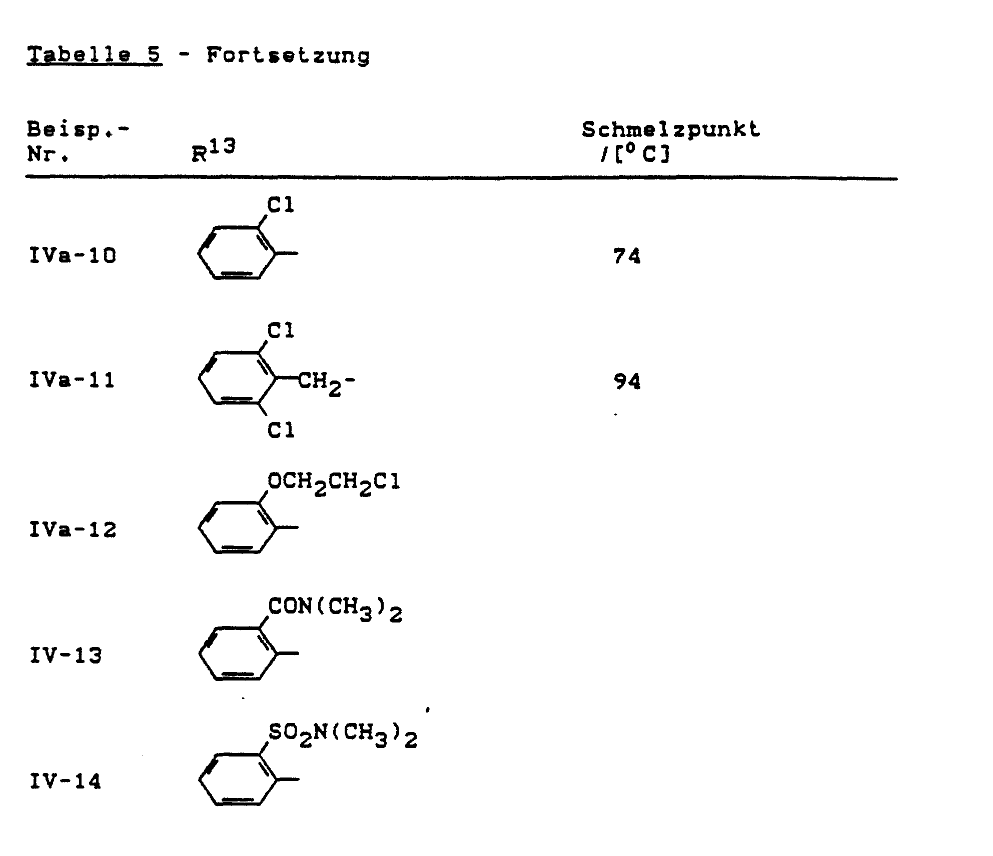

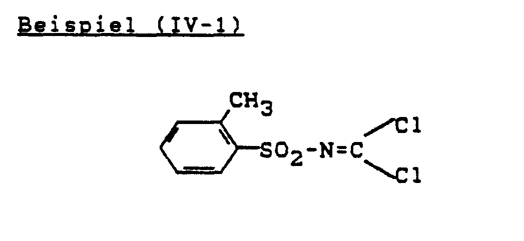

## Patentansprüche

1) Verfahren zur Herstellung von O-Aryl-N'-pyrimidin-2-yl-isoharnstoffen der allgemeinen Formel (I) 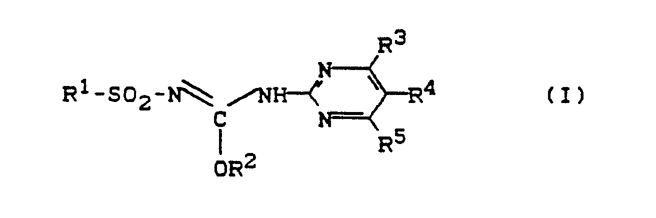 in welcher
R¹ für einen gegebenenfalls substituierten Rest aus der Reihe Aralkyl, Aryl und Heteroaryl steht,
R² für gegebenenfalls substituiertes Aryl steht,
R³ für Wasserstoff, Halogen, Hydroxy, C₁-C₆-Alkyl-amino, Di-(C₁-C₆-alkyl)-amino oder für gegebenenfalls substituiertes C₁-C₆-Alkyl, C₁-C₆-Alkoxy und C₁-C₆-Alkylthio steht,
R⁴ für Wasserstoff, Halogen, gegebenenfalls substituiertes C₁-C₆-Alkyl, Cyano, Formyl, C₁-C₆-Alkylcarbonyl oder C₁-C₆-Alkoxycarbonyl steht und
R⁵ für Wasserstoff, Halogen, Hydroxy, für gegebenenfalls substituiertes C₁-C₆-Alkyl, C₁-C₆-Alkoxy und C₁-C₆-Alkylthio, für C₁-C₆-Alkylamino oder Di-(C₁-C₆-alkyl)-amino steht, oder
R⁴ und R⁵ gemeinsam für C₃-C₆-Alkandiyl stehen, dadurch gekennzeichnet, daß man Iminokohlensäureester der Formel (II) 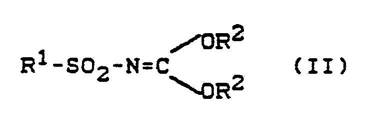 in welcher
R¹ und R² die oben angegebenen Bedeutungen haben,
mit Aminen der Formel (III) 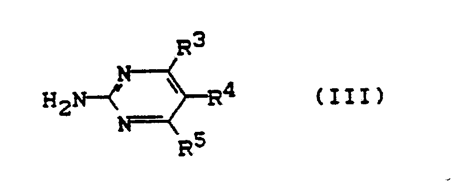 in welcher
R³, R⁴ und R⁵ die oben angegebenen Bedeutungen haben,
in Gegenwart von Säureakzeptoren und gegebenenfalls in Gegenwart von Verdünnungsmitteln bei Temperaturen zwischen 0 °C und 150 °C umsetzt.

2) Iminokohlensäureester der Formel (II) 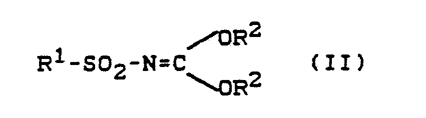 in welcher
R¹ für einen gegebenenfalls substituierten Rest aus der Reihe Aralkyl, Aryl und Heteroaryl steht und
R² für gegebenenfalls substituiertes Aryl steht.

3) Iminokohlensäureester der Formel (II), gemäß Anspruch 2,
in welcher
R¹ für den Rest 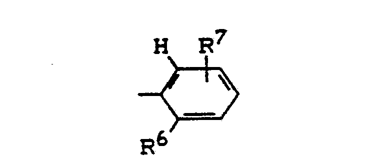 steht, worin
R⁶ und R⁷ gleich oder verschieden sind und für Wasserstoff, Halogen [wie insbesondere Fluor, Chlor, Brom und/oder lod], Cyano, C₁-C₆-Alkyl [welches gegebenenfalls durch Fluor, Chlor, Brom, Cyano, C₁-C₄-Alkoxy, C₃-C₆-Cycloalkyl oder Phenyl substituiert ist], für C₁-C₄-Alkoxy [weiches gegebenenfalls durch Fluor, Chlor, Brom, Cyano oder C₁-C₄-Alkoxy substituiert ist], für Di-(C₁-C₄-alkyl)-aminosulfonyl, für Phenyl oder Phenoxy, für C₁-C₄-Alkylcarbonyl-amino, C₁-C₄-Alkylamino-carbonyl-amino, Di-(C₁-C₄-alkyl)-amino-carbonyl-amino, Di-(C₁-C₄-alkyl)-aminocarbonyl oder für Di-(C₁-C₄-alkyl)-aminosulfonylamino stehen, worin weiter
R' für den Rest 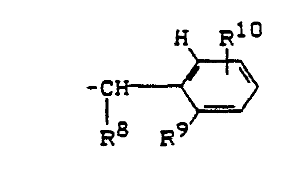 steht, worin
R⁸ für Wasserstoff oder C₁-C₄-Alkyl steht,
R⁹ und R¹⁰ gleich oder verschieden sind und für Wasserstoff, Fluor, Chlor, Brom, Nitro, Cyano, Ci-C_{d}-Alkyl [welches gegebenenfalls durch Fluor und/oder Chlor substituiert ist], C₁-C₄-Alkoxy [welches gegebenenfalls durch Fluor und/oder Chlor substituiert ist] oder Di-(C₁-C₄-alkyl)-aminosulfonyl stehen; worin weiter
R' für den Rest 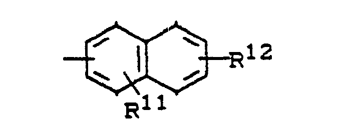 steht, worin
R¹¹ und R¹² gleich oder verschieden sind und für Wasserstoff, Fluor, Chlor, Brom, Nitro, Cyano, C₁-C₄-Alkyl [welches gegebenenfalls durch Fluor und/oder Chlor substituiert ist] oder C₁-C₄-Alkoxy [welches gegebenenfalls durch Fluor und/oder Chlor substituiert ist], stehen; und
R² für einen Phenylrest steht, welcher gegebenenfalls substituiert ist durch einen oder mehrere Reste aus der Reihe
Halogen [wie insbesondere Fluor, Chlor, Brom und lod], Cyano, Nitro, Hydroxy, Carboxy, C₁-C₆-Alkyl [welches gegebenenfalls durch Fluor, Chlor, Brom, Nitro, Cyano, Hydroxy, Carboxy, C₁-C₄-Alkoxy-carbonyl, C₁-C₄-Alkoxy, C₁-C₄-Alkylthio oder Phenyl substituiert ist], C₃-C₆-Cycloalkyl, C₁-C₄-Alkoxy [welches gegebenenfalls durch Fluor, Chlor, Brom, Cyano. Carboxy, C₁-C₄-Alkoxy, C₁-C₄-Alkylthio oder C₁-C₄-Alkoxy-carbonyl substituiert ist], C₁-C₄-Alkylthio [welches gegebenenfalls durch Fluor, Chlor, Brom, Cyano, Carboxy, C₁-C₄-Alkoxy-carbonyl substituiert ist], Amino, C₁-C₄-Alkyl-amino bzw. Di-(C₁-C₄-alkyl)-amino [weiche gegebenenfalls durch Fluor, Chlor, Brom, Cyano, Carboxy, C₁-C₄-Alkoxy oder C₁-C₄-Alkoxy-carbonyl substituiert sind], C₁-C₄-Alkyl-carbonyl-amino, C₁-C₄-Alkoxy-carbonyl-amino, (Di)-C,-C_{d}-Alkylamino-carbonyl-amino, Formyl, Ci-C₄-Alkyl-carbonyl, Benzoyl, C₁-C₄-Alkoxy-carbonyl, Phenoxy-carbonyl, Benzyloxycarbonyl, Phenyl [welches gegebenenfalls durch Fluor, Chlor, Brom, Cyano, Nitro, Hydroxy oder Methyl substituiert ist], Phenoxy, Phenylthio, Phenylsulfonyl, Phenylamino oder Phenylazo [wel che gegebenenfalls durch Fluor, Chlor, Brom, Cyano, Nitro, Methyl und/ oder Trifluormethyl substituiert sind], Pyridoxy oder Pyrimidoxy [welche gegebenenfalls durch Fluor, Chlor, Brom, Cyano, Nitro, Methyl und/oder Trifluormethyl substituiert sind], C₁-C₄-Alkyl-carbonyloxy, C₁-C₄-Alkoxy-carbonyloxy, Ci-C_{d}-Alkyl-amino-carbonyloxy und Di-(C₁-C₄-alkyl)-amino-car- bonyloxy, oder welcher gegebenenfalls durch eine Alkylenkette [welche gegebenenfalls verzweigt und/oder durch ein oder mehrere Sauerstoffatome unterbrochen ist] oder einen Benzorest [welcher gegebenenfalls durch Fluor, Chlor, Brom, Cyano, Nitro, Methyl und/oder Trifluormethyl substituiert ist] anelliert ist.

4) Verfahren zur Herstellung von Iminokohlensäureestern der allgemeinen Formel (II), gemäß Anspruch 2, dadurch gekennzeichnet, daß man Iminokohlensäuredichloride der allgemeinen Formel (IV) 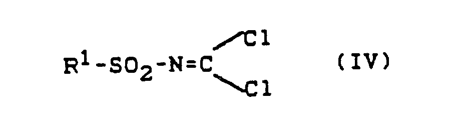 in welcher
R' die in Anspruch 2 angegebenen Bedeutungen hat,
mit Verbindungen der Formel (V)
R²⁻OM (V)
in welcher
R² die in Anspruch 2 angegebenen Bedeutungen hat und
M für Wasserstoff oder ein Alkalimetallatom steht.
gegebenenfalls in Gegenwart von Säureakzeptoren und gegebenenfalls in Gegenwart von inerten Verdünnungsmitteln bei Temperaturen zwischen 10° und 100°C umsetzt.

5) Iminokohlensäuredichloride der Formel (IVa) 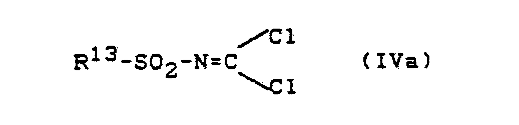 in welcher
R¹³ für den Rest 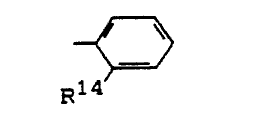 steht, worin
R¹⁴ für Halogen [wie insbesondere Fluor, Chlor, Brom und/oder lod], Cyano, C₂-C₆-Alkyl, C₁-C₆-Alkyl [welches durch Fluor, Chlor, Brom, Cyano, C₁-C₄-Alkoxy, C₃-C₆-Cycloalkyl oder Phenyl substituiert ist], für C₁-C₄-Alkoxy [welches gegebenenfalls durch Fluor, Chlor, Brom, Cyano oder C₁-C₄-Alkoxy substituiert ist], für Di-(C₁-C₄-alkyl)-aminosulfonyl,
für Di-(C₁-C₄-alkyl)-aminocarbonyl, für Phenyl oder Phenoxy, für C₁-C₄-Alkylcarbonylamino, C₁-C₄-Alkylamino-carbonyl-amino, Di-(C₁-C₄-alkyl)-amino-carbonylamino, Di-(C₁-C₄-alkyl)-aminocarbonyl oder für Di-(C₁-C₄-alkyl)-aminosulfonylamino steht, worin weiter
R¹³ für den Rest 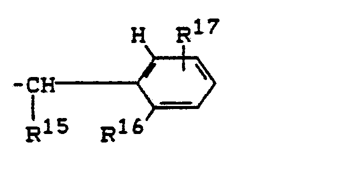 steht, worin
R¹⁵ für Wasserstoff oder C₁-C₄-Alkyl steht,
R¹⁶ und R¹⁷ gleich oder verschieden sind und für Wasserstoff, Fluor, Chlor, Brom, Nitro, Cyano, C₁-C₄-Alkyl [welches gegebenenfalls durch Fluor und/oder Chlor substituiert ist], C₁-C₄-Alkoxy [welches gegebenenfalls durch Fluor und/oder Chlor substituiert ist] oder Di-(C₁-C₄-alkyl)-aminosulfonyl stehen; worin weiter
R¹³ für den Rest 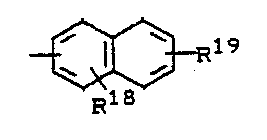 steht, worin
R¹⁸ und R¹⁹ gleich oder verschieden sind und für Wasserstoff, Fluor, Chlor, Brom, Nitro, Cyano, C₁-C₄-Alkyl [welches gegebenenfalls durch Fluor und/oder Chlor substituiert ist] oder C₁-C₄-Alkoxy [welches gegebenenfalls durch Fluor und/oder Chlor substituiert ist], stehen.

6) Verfahren zur Herstellung von Iminokohlensäuredichloriden der allgemeinen Formel (IVa), gemäß Anspruch 5, dadurch gekennzeichnet, daß man Iminodithiokohlensäuredimethylester der Formel (VI) 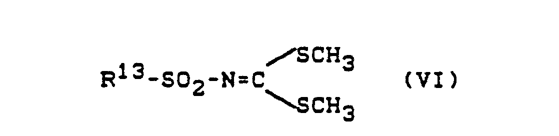 in welcher
R¹3 die in Anspruch 5 angegebenen Bedeutungen hat,
in Gegenwart von inerten Verdünnungsmitteln bei Temperaturen zwischen 0° und 25°C mit einem Chlorierungsmittel umsetzt.
